(19) Europäisches Patentamt
European Patent Office
Office européen des brevets

(11) EP 4 039 801 A1

(12) EUROPEAN PATENT APPLICATION
published in accordance with Art. 153(4) EPC

(43) Date of publication:
**10.08.2022 Bulletin 2022/32**

(21) Application number: **20855397.4**

(22) Date of filing: **17.08.2020**

(51) International Patent Classification (IPC):
***C12N 5/079*** *(2010.01)* ***C12N 15/864*** *(2006.01)*
***A61K 48/00*** *(2006.01)* ***A61P 25/00*** *(2006.01)*
***C07K 14/47*** *(2006.01)*

(52) Cooperative Patent Classification (CPC):
**C12N 15/86; A61P 1/18; A61P 25/00; A61P 25/28;**
A61K 38/00; A61K 48/005; C12N 2310/20;
C12N 2750/14143; C12N 2750/14171

(86) International application number:
**PCT/CN2020/109653**

(87) International publication number:
**WO 2021/032068 (25.02.2021 Gazette 2021/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.08.2019 CN 201910760367**
**30.10.2019 CN 201911046435**
**26.03.2020 PCT/CN2020/081489**
**28.07.2020 CN 202010740568**

(71) Applicant: **Center for Excellence in Brain Science and Intelligence Technology, Chinese Academy of Sciences**
**Xuhui District**
**Shanghai 200031 (CN)**

(72) Inventors:
- **YANG, Hui**
**Shanghai 200031 (CN)**
- **ZHOU, Haibo**
**Shanghai 200031 (CN)**

(74) Representative: **Lavoix**
**Bayerstraße 83**
**80335 München (DE)**

# (54) APPLICATION OF PTBP1 INHIBITOR IN PREVENTING AND/OR TREATING NERVOUS SYSTEM DISEASE RELATED TO FUNCTIONAL NEURONAL DEATH

(57) An application of a Ptbpl inhibitor in preventing and/or treating a nervous system disease related to functional neuronal death. Specifically, provided is a use of a Ptbpl gene or RNA or an encoded protein inhibitor thereof in preparing a composition or a preparation. The composition or the preparation is used for preventing and/or treating a nervous system disease related to functional neuronal death. By inhibiting the expression or activity of the Ptbpl gene or RNA or encoded protein thereof in astrocytes in the brain, the transdifferentiation of the astrocytes into dopamine neurons can be effectively induced. In addition, by inhibiting the expression or activity of the Ptbpl gene or RNA or encoded protein thereof in muller glial cells in the retinal, the transdifferentiation of the muller glial cells into retinal ganglion cells can be effectively induced, thereby preventing and/or treating a nervous system disease related to functional neuronal death.



Processed by Luminess, 75001 PARIS (FR)

## Description

### Technical field

The present invention relates to the field of biomedicine. More specifically, the present invention relates to the use of Ptbpl inhibitors in the prevention and/or treatment of neurological diseases related to functional neuronal death.

### Background

Parkinson's disease (PD) is a serious neurodegenerative disease characterized by the loss of dopamine neurons in the substantia nigra of the midbrain. Previous studies have achieved the direct reprogramming of astrocytes to dopamine neurons in vitro and in animal models by simultaneously overexpressing several transcription factors. However, so far, Ptbpl mediated neuronal reprogramming in vivo has not been reported yet.

At present, the main treatment for Parkinson's disease is drugs represented by levodopa preparations. At the same time, surgical treatment can also improve symptoms to a certain extent. It should be pointed out that all these methods can only partially alleviate the disease, and cannot achieve the effect of preventing the development of the disease.

Therefore, there is an urgent need in this field to develop targets that can effectively treat neurodegenerative diseases.

### Summary of the invention

The purpose of the present invention is to provide a target that can effectively treat neurodegenerative diseases.

Another purpose of the present invention is to provide a new target Ptbpl for the treatment of Parkinson's disease. By inhibiting the expression of Ptbpl, astrocytes in the striatum can be directly converted into dopamine neurons and the phenotype of Parkinson's disease can be restored.

Another purpose of the present invention is to provide a new target Ptbpl for the treatment of visual impairment. By inhibiting the expression of Ptbpl, the Müller glial cells in the retina can be directly converted into retinal ganglion cells and the phenotype of permanent visual impairment can be alleviated.

In the first aspect of the present invention, it provides a use of an inhibitor of Ptbpl gene or RNA or an encoded protein thereof for the preparation of a composition or preparation for the treatment of a nervous system disease related to functional neuronal death.

In another preferred embodiment, the composition or preparation is also used for one or more purposes selected from the group consisting of:

(a1) inducing the transdifferentiation of astrocytes into dopamine neurons;
(b1) treating movement disorders in mammalian Parkinson's disease;
(c1) inducing the conversion or differentiation of glial cells into functional neurons;
(d1) treating nervous system diseases related to retinal ganglion cell (RGC) degeneration;
(e1) preventing or treating retinal diseases;
(f1) preventing and/or treating neurodegenerative diseases;
(g1) transdifferentiation of Müller glial cells into retinal ganglion cells;
(h1) treating visual impairment caused by the death of the optic ganglia in mammals.

In another preferred embodiment, the nervous system disease is selected from the group consisting of glaucoma, age-related RGC loss, injury of optic nerve, retinal ischemia, Leber hereditary optic neuropathy, Alzheimer's disease, Huntington's disease, schizophrenia, depression, drug taking, movement disorders (such as chorea, hypercholesterolemia, and movement disorders), motor neuron injury diseases (such as amyotrophic lateral sclerosis, spinal cord injury), bipolar disorder, autism spectrum disorder (ASD), dysfunction, Parkinson's disease, and a combination thereof.

In another preferred embodiment, the glial cells are selected from the group consisting of astrocytes, MG cells, oligodendrocytes, ependymal cells, Schwan cells, NG2 cells, satellite cells, and combinations thereof.

In another preferred embodiment, the functional neuron is selected from the group consisting of: RGC neuron, dopamine neuron, and a combination thereof.

In another preferred embodiment, the functional neuron is derived from the striatum.

In another preferred embodiment, the functional neuron is derived from mature retina.

In another preferred embodiment, the retinal disease is a retinal disease caused by neurodegeneration.

In another preferred embodiment, the composition or preparation induces the transdifferentiation of a MG cell into a RGC cell to treat a retinal disease caused by neurodegeneration.

In another preferred embodiment, the MG cell is a Müller glial cell.

In another preferred embodiment, the MG cell is derived from the retina.

In another preferred embodiment, the RGC cell is a retinal ganglion cell.

In another preferred embodiment, the RGC cell is a functional RGC.

In another preferred embodiment, the RGC cell can be integrated into the visual pathway and improve visual performance.

In another preferred embodiment, the RGC cell can achieve functional projection to the central visual area and improve visual performance.

In another preferred embodiment, the improvement of visual performance is to improve the visual performance of mammals suffering from a retinal disease caused by neurodegeneration.

In another preferred embodiment, when the MG cell is transdifferentiated into a RGC cell, at the same time the MG cell is also differentiated into an amacrine cell.

In another preferred embodiment, RGC (1) expresses Brn3a, Rbpms, Foxp2, Brn3c and/or parvalbumin; (2) is F-RGC, type 3 RGC or PV-RGC; (3) is integrated into existing retinal pathways in the subject (for example, the central information can be projected to dLGN, and the vision can be partially restored by relaying the visual information to VI); and/or (4) the visual information can be received, it is characterized by the ability to establish action potential synaptic responses in light stimulation, synaptic connections (for example with existing functional dLGN neurons in the brain), biogenesis of presynaptic neurotransmitters, and/or subsequent actions.

In another preferred embodiment, the Müller glial cell in the mature retina is reprogrammed and converted into RGC.

In another preferred embodiment, dopamine neuron (1) expresses tyrosine hydroxylase (TH), dopamine transporter (DAT), vesicular monoamine transporter 2 (VMAT2), hybrid homeobox 1 (Enl) , FoxA2 and/or LIM homeobox transcription factor 1 alpha (Lmxla); (2) exhibits the biogenesis of presynaptic neurotransmitters; (3) is integrated into existing neuronal circuits in the subject's brain; and/or (4) is characterized by its ability to establish action potentials, synaptic connections, biogenesis of presynaptic neurotransmitters and/or postsynaptic responses.

In another preferred embodiment, a plurality of glial cells in the striatum are reprogrammed, and at least 10% or at least 30% of the glial cells are converted into dopamine neurons.

In another preferred embodiment, the mammal includes a mammal suffering from a neurodegenerative disease.

In another preferred embodiment, the mammal includes a human or a non-human mammal.

In another preferred embodiment, the non-human mammal includes a rodent (such as a mouse, rat, or rabbit), primate (such as a monkey).

In another preferred embodiment, the astrocyte is derived from the striatum, substantia nigra, spinal cord, dorsal midbrain or cerebral cortex, preferably, the astrocyte is derived from the striatum.

In another preferred embodiment, the astrocyte includes an astrocyte in the striatum.

In another preferred embodiment, the astrocyte is an astrocyte in brain tissue.

In another preferred embodiment, the inhibitor is selected from the group consisting of an antibody, small molecule compound, microRNA, siRNA, shRNA, antisense oligonucleotide, aptamer, gene editor, and a combination thereof.

In another preferred embodiment, the gene editor includes a DNA gene editor and an RNA gene editor.

In another preferred embodiment, the gene editor includes optional gRNA and gene editing protein.

In another preferred embodiment, the expression of the gene editor is driven by a glial cell-specific promoter (e.g., a GFAP promoter).

In another preferred embodiment, the gene editor includes two or more gNRAs and gene editing proteins.

In another preferred embodiment, the gRNA guides the gene editing protein to specifically bind to the RNA of the Ptbpl gene.

In another preferred embodiment, the gRNA guides gene editing protein to specifically bind to the mRNA of the Ptbpl gene.

In another preferred embodiment, the gene editing protein is selected from the group consisting of Cas13d, CasRx, Cas13e, CRISPR/Cas9, Cpfl, Cas9, Cas13a, Cas13b, Cas13c, Cas13f, RNA-targeted gene editing protein, and a combination thereof.

In another preferred embodiment, the gene editing protein is CasRx, and the nucleotide sequence of the gRNA is selected from the group consisting of: SEQ ID NO.: 1, 2, 3, 4, 5 and 6.

In another preferred embodiment, the source of the gene editing protein is selected from the group consisting of: *Streptococcus pyogenes, Staphylococcus aureus, Acidaminococcus sp*, *Lachnospiraceae bacterium, Ruminococcus Flavefaciens*, and a combination thereof.

In another preferred embodiment, the Ptbpl is derived from a mammal; preferably, from a human, mouse, rat, or rabbit; more preferably, from a human.

In another preferred embodiment, the Ptbpl gene includes a wild-type Ptbpl gene and a mutant Ptbpl gene.

In another preferred embodiment, the mutant includes a mutant form in which the function of the encoded protein is not changed after mutation (i.e., the function is the same or substantially the same as that of the wild-type encoded protein).

In another preferred embodiment, the polypeptide encoded by the mutant Ptbpl gene is the same or substantially the same as the polypeptide encoded by the wild-type Ptbpl gene.

In another preferred embodiment, the mutant Ptbpl gene comprises a polynucleotide with homology ≥ 80% (preferably ≥ 90%, more preferably ≥ 95%, more preferably, ≥98% or 99%) compared with the wild-type Ptbpl gene.

In another preferred embodiment, the mutant Ptbpl gene comprises a polynucleotide with 1-60 (preferably 1-30, more preferably 1-10) nucleotides truncated or added at the 5' end and/or 3' end of the wild-type Ptbp1 gene.

In another preferred embodiment, the Ptbpl gene includes a cDNA sequence, a genomic sequence, and a combination thereof.

In another preferred embodiment, the Ptbpl protein includes an active fragment of Ptbp1 or a derivative thereof.

In another preferred embodiment, the active fragment or derivative thereof has at least 90% homology with Ptbpl, preferably 95%, more preferably 98%, 99%.

In another preferred embodiment, the active fragment or derivative thereof has at least 80%, 85%, 90%, 95%, 100% of the Ptbpl activity.

In another preferred embodiment, the amino acid sequence of the Ptbpl protein is selected from the group consisting of:

(i) a polypeptide having the amino acid sequence as shown in SEQ ID NO.: 11;
(ii) a polypeptide with the protein function derived from (i) formed by substitution, deletion or addition of one or several (e.g. 1-10) amino acid residues to the amino acid sequence as shown in SEQ ID NO.: 11; or
(iii) a polypeptide with the protein function whose amino acid sequence has ≥90% (preferably ≥95%, more preferably ≥98% or 99%) homology with the amino acid sequence as shown in SEQ ID NO.: 11.

In another preferred embodiment, the nucleotide sequence of the Ptbpl gene is selected from the group consisting of:

(a) a polynucleotide encoding a polypeptide as shown in SEQ ID NO.: 11;
(b) a polynucleotide whose sequence is shown in SEQ ID NO.: 12;
(c) a polynucleotide whose nucleotide sequence has a homology of ≥95% (preferably ≥98%, more preferably ≥99%) with the sequence as shown in SEQ ID NO.: 12;
(d) a polynucleotide having 1-60 (preferably 1-30, more preferably 1-10) nucleotides truncated or added at the 5' end and/or 3' end of the polynucleotide as shown in SEQ ID NO.: 12;
(e) a polynucleotide complementary to any of the polynucleotides of (a)-(d).

In another preferred embodiment, the ptbpl protein is shown in SEQ ID NO.: 11.

In another preferred embodiment, the nucleic acid encoding the ptbpl protein is shown in SEQ ID NO.: 12.

In another preferred embodiment, the region targeted by the inhibitor (e.g., gene editing protein) of the ptbpl gene or the encoded protein thereof is positions 4758-4787 and/or 5381-5410 of the ptbpl gene sequence.

In another preferred embodiment, the inhibitor of the ptbpl gene or the encoded protein thereof inhibits the activity and/or expression level of ptbpl.

In another preferred embodiment, the concentration (titer of virus) of the inhibitor of the ptbpl gene or the encoded protein thereof is $>1\times10^{13}$, preferably, $1\times10^{13}$-$1\times10^{14}$.

In another preferred embodiment, the inhibitory rate of the inhibitor of the ptbpl gene or the encoded protein thereof on the activity and/or expression level of ptbpl is greater than 90%, preferably, 90%-95%.

In another preferred embodiment, the inhibitor targets astrocytes in brain tissue.

In another preferred embodiment, the inhibitor targets MG cells of the retina.

In another preferred embodiment, the neurodegenerative disease includes Parkinson's disease.

In a second aspect of the present invention, it provides a composition comprising:

(a) a gene editing protein, or an expression vector thereof, selected from the group consisting of CasRx, CRISPR/Cas9, Cpfl, Cas9, Cas13a, Cas13b, Cas13c, RNA-targeted gene editing protein, and a combination thereof; and
(b) a gRNA or an expression vector thereof, the gRNA guides the gene editing protein to specifically bind to the DNA or RNA of the Ptbpl gene.

In another preferred embodiment, the gRNA guides the gene editing protein to specifically bind to the mRNA of the Ptbpl gene.

In another preferred embodiment, the nucleotide sequence of the gRNA is selected from the group consisting of: SEQ ID NO.: 1, 2, 3, 4, 5 and 6.

In another preferred embodiment, the composition includes a pharmaceutical composition.

In another preferred embodiment, the composition further comprises:

(c) other drugs for the prevention and/or treatment of neurodegenerative diseases.

In another preferred embodiment, the composition further comprises:

(d) other drugs for the treatment of a nervous system disease associated with functional neuronal death.

In another preferred embodiment, the composition further comprises:

(e) other drugs for the prevention and/or treatment of retinal diseases.

In another preferred embodiment, the expression vector of the gene editing protein includes a vector targeting glial cells.

In another preferred embodiment, the expression vector of the gene editing protein includes a vector targeting astrocytes in brain tissue.

In another preferred embodiment, the expression vector of the gene editing protein includes a vector targeting retinal MG cells.

In another preferred embodiment, the expression vector includes a viral vector.

In another preferred embodiment, the viral vector is selected from the group consisting of adeno-associated virus (AAV), adenovirus, lentivirus, retrovirus, herpes virus, SV40, poxvirus, and a combination thereof.

In another preferred embodiment, the vector is selected from the group consisting of lentivirus, adenovirus, adeno-associated virus (AAV), and a combination thereof, preferably, the vector is adeno-associated virus (AAV).

In another preferred embodiment, the vector includes AAV2 or AAV9.

In another preferred embodiment, the gene encoding the gene editing protein and the gRNA are located in the same expression vector (such as an AAV vector).

In another preferred embodiment, the expression vector of the gene editing protein and the expression vector of the gRNA are the same expression vector (such as an AAV vector).

In another preferred embodiment, the expression vector further includes a glial cell-specific promoter (e.g., a GFAP promoter) for driving the expression of the gene editing protein.

In another preferred embodiment, the dosage form of the composition is selected from the group consisting of: freezed-drying preparation, liquid preparation, and a combination thereof.

In another preferred embodiment, the dosage form of the composition is a liquid preparation.

In another preferred embodiment, the dosage form of the composition is an injection dosage form.

In another preferred embodiment, other drugs for the prevention and/or treatment of the neurodegenerative diseases are selected from the group consisting of dopamine precursor, non-ergot dopamine agonist, monoamine oxidase B inhibitors, and combinations thereof.

In another preferred embodiment, the composition is a cell preparation.

In another preferred embodiment, the gene editing protein expression vector and the gRNA expression vector are the same vector or different vectors.

In another preferred embodiment, the weight ratio of the component (a) to the component (b) is 100:1-0.01:1, preferably 10:1-0.1:1, more preferably 2: 1-0.5:1.

In another preferred embodiment, in the composition, the content of the component (a) is 0.001%-99%, preferably 0.1%-90%, more preferably 1%-70%.

In another preferred embodiment, in the composition, the content of the component (b) is 0.001%-99%, preferably 0.1%-90%, more preferably 1%-70%.

In another preferred embodiment, in the composition, the content of the component (c) is 1%-99%, preferably 10%-90%, more preferably 30%-70%.

In another preferred embodiment, in the composition, the component (a), the component (b) and the optional component (c) account for 0.01-99.99 wt %, preferably 0.1-90wt%, more preferably 1-80wt% of the total weight of the composition.

In a third aspect of the present invention, it provides a kit, comprising:

(a1) a first container, and a gene editing protein or an expression vector thereof located in the first container, or a drug containing a gene editing protein or an expression vector thereof, the gene editing protein is selected from the group consisting of Cas13d, CasRx, Cas13e, CRISPR/Cas9, Cpfl, Cas9, Cas13a, Cas13b, Cas13c, Cas13f, RNA-targeted gene editing protein, and a combination thereof;

(b1) a second container, and a gRNA or an expression vector thereof located in the second container, or a drug containing a gRNA or an expression vector thereof, the gRNA guides the gene editing protein to specifically bind to the DNA or RNA of the Ptbpl gene.

In another preferred embodiment, the gRNA guides the gene editing protein to specifically bind to the mRNA of the Ptbpl gene.

In another preferred embodiment, the nucleotide sequence of the gRNA is selected from the following group: SEQ ID NO.: 1, 2, 3, 4, 5 and 6.

In another preferred embodiment, the region targeted by the gRNA is positions 4758-4787 and/or 5381-5410 of the *Ptbpl* gene sequence.

In another preferred embodiment, the kit further includes:

(c1) a third container, and other drugs for the prevention and/or treatment of the neurodegenerative diseases, and/or containing other drugs for the prevention and/or treatment of retinal diseases, and/or containing other drugs for the treatment of a nervous system disease associated with functional neuronal death located in the third container.

In another preferred embodiment, the first container, the second container, and the third container are the same or different containers.

In another preferred embodiment, the drug in the first container is a single preparation containing the gene editing protein or the expression vector thereof.

In another preferred embodiment, the drug in the second container is a single preparation containing gRNA or the expression vector thereof.

In another preferred embodiment, the drug in the third container is a single preparation containing other drugs for the treatment of a nervous system disease associated with functional neuronal death.

In another preferred embodiment, the dosage form of the drug is selected from the group consisting of freezed-drying preparation, liquid preparation, and a combination thereof.

In another preferred embodiment, the dosage form of the drug is an oral dosage form or an injection dosage form.

In another preferred embodiment, the kit also contains instructions.

In a fourth aspect of the present invention, it provides use of the composition according to the second aspect of the present invention or the kit according to the third aspect of the present invention for the preparation of a medicament for the treatment of a nervous system disease associated with functional neuronal death.

In another preferred embodiment, in the composition, the action concentration (virus titer) of the gene editing protein or the expression vector thereof is >$1\times10^{13}$, preferably, $1\times10^{13}$-$1\times10^{14}$.

In another preferred embodiment, in the composition, the action concentration (virus titer) of the gRNA or the expression vector thereof is >$1\times10^{13}$, preferably, $1\times10^{13}$-$1\times10^{14}$.

In another preferred embodiment, in the pharmaceutical composition, the action concentration (virus titer) of the other drugs for the treatment of a nervous system disease associated with functional neuronal death is >$1\times10^{13}$, preferably, $1\times10^{13}$-$1\times10^{14}$.

In another preferred embodiment, in the pharmaceutical composition, the action concentration (virus titer) of the other drugs for the treatment of a nervous system disease associated with functional neuronal death is >$1\times10^{13}$, preferably, $1\times10^{13}$-$1\times10^{14}$.

In another preferred embodiment, the composition or kit comprises (a) a gene editing protein or an expression vector thereof; and (b) a gRNA or an expression vector thereof; and (c) optional other drugs for the treatment of a nervous system disease associated with functional neuronal death; and (d) a pharmaceutically acceptable carrier.

In another preferred embodiment, in the composition or kit, (a) a gene editing protein or an expression vector thereof; and (b) a gRNA or an expression vector thereof; and (c) optional other drugs for the treatment of a nervous system disease associated with functional neuronal death account for 0.01-99.99wt%, preferably 0.1-90wt%, more preferably 1-80wt% of the total weight of the composition or kit.

In a fifth aspect of the present invention, it provides a method for promoting the differentiation of a glial cell into a functional neuron, comprising the steps of:

In the presence of the inhibitor of Ptbpl gene or RNA or an encoded protein thereof or the composition according to the second aspect of the present invention, culturing the glial cell, thereby promoting the differentiation of the glial cell into the functional neuron.

In another preferred embodiment, the glial cell is selected from the group consisting of astrocyte, MG cell, oligodendrocyte, ependymal cell, Schwan cell, NG2 cell, satellite cell, and a combination thereof.

In another preferred embodiment, the functional neuron is selected from the group consisting of a RGC neuron, dopamine neuron, and a combination thereof.

In another preferred embodiment, the glial cell is a in vitro cell.

In another preferred embodiment, the action concentration (virus titer) of the inhibitor of the Ptbpl gene or the encoded protein thereof is greater than $1\times10^{13}$, preferably, $1\times10^{13}$-$1\times10^{14}$.

In another preferred embodiment, the action concentration (virus titer) of the composition of claim 2 is greater than $1\times10^{13}$, preferably, $1\times10^{13}$-$1\times10^{14}$.

In a sixth aspect of the present invention, it provides a method for preventing and/or treating a nervous system disease associated with functional neuronal death, comprising:

Administering an inhibitor of the Ptbpl gene or RNA or the encoded protein thereof, or the composition according to the second aspect of the present invention, or the kit according to the third aspect of the present invention to a subject in need.

In another preferred embodiment, the subject includes a human or non-human mammal suffering from a nervous system disease associated with functional neuron death.

In another preferred embodiment, the non-human mammal includes a rodent and primate, preferably a mouse, rat, rabbit, and monkey.

In a seventh aspect of the present invention, it provides a method for screening a candidate compound for preventing and/or treating a nervous system disease associated with functional neuron death, comprising the steps of:

(a) in the test group, adding a test compound to the cell culture system, and observing the expression level (E1) and/or activity (A1) of Ptbpl in the cell of the test group; in the control group, the test compound is not added to the culture system of the same cell and the expression level (E0) and/or activity (A0) of Ptbpl in the cell of the control group is observed;

wherein, if the expression level (E1) and/or activity (A1) of Ptbpl in the cell in the test group is significantly lower than that in the control group, indicating that the test compound is a candidate compound for preventing and/or treating a nervous system disease associated with functional neuron death with an inhibitory effect on the expression and/or activity of Ptbpl.

In another preferred embodiment, the expression level of Ptbpl is obtained by qPCR.

In another preferred embodiment, the method further comprises the steps of:

(b) For the candidate compound obtained in step (a), further testing its promoting effect on the differentiation of a glial cell into a functional neuron; and/or further testing whether it has a down-regulation effect on the Ptbpl gene.

In another preferred embodiment, the method includes step (c): administering the candidate compound determined in step (a) to a mammalian model to determine its effect on a mammal.

In another preferred embodiment, the mammal is a mammal suffering from a nervous system disease associated with functional neuron death.

In another preferred embodiment, the "significantly lower than" refers to E1/E0≤1/2, preferably, ≤ 1/3, more preferably≤1/4.

In another preferred embodiment, the "significantly lower than" refers to A1/A0≤1/2, preferably, ≤1/3, more preferably≤1/4.

In another preferred embodiment, the cell includes a glial cell.

In another preferred embodiment, the cell is a cell cultured in vitro.

In another preferred embodiment, the method is non-diagnostic and non-therapeutic.

In an eighth aspect of the present invention, it provides a method for promoting the differentiation of an astrocyte into a dopamine neuron, comprising the steps of:

In the presence of the inhibitor of Ptbpl gene or RNA or an encoded protein thereof or the composition according to the second aspect of the present invention, culturing an astrocyte, thereby promoting the differentiation of the astrocyte into the dopamine neuron.

In another preferred embodiment, the astrocyte includes an astrocyte in striatum.

In another preferred embodiment, the astrocyte is astrocyte in brain tissue.

In another preferred embodiment, the astrocyte is a cell in vitro.

In another preferred embodiment, the action concentration (virus titer) of the inhibitor of the Ptbpl gene or the encoded protein thereof is greater than $1\times10^{13}$, preferably, $1\times10^{13}$-$1\times10^{14}$.

In another preferred embodiment, the action concentration (virus titer) of the composition is $>1\times10^{13}$, preferably, $1\times10^{13}$-$1\times10^{14}$.

In another preferred embodiment, the method is a non-diagnostic and non-therapeutic method.

In a ninth aspect of the present invention, it provides a method for promoting the differentiation of a Müller glial cell into a retinal ganglion cell, comprising the steps of: in the presence of an inhibitor of the Ptbpl gene or RNA or the encoded protein thereof or the composition according to the second aspect of the present invention, thereby promoting the differentiation of the retinal Müller glial cell into the retinal ganglion cell.

In another preferred embodiment, the action concentration (virus titer) of the inhibitor of the Ptbpl gene or RNA or the encoded protein thereof is $>1\times10^{13}$, preferably, $1\times10^{13}$-$1\times10^{14}$.

In another preferred embodiment, the action concentration (virus titer) of the composition is $>1\times10^{13}$, preferably, $1\times10^{13}$-$1\times10^{14}$.

In another preferred embodiment, the method is a non-diagnostic and non-therapeutic method.

In a tenth aspect of the present invention, it provides an AAV vector, comprising:

(a) a coding sequence for a gene editing protein selected from the group consisting of CasRx, CRISPR/Cas9, Cpfl, Cas9, Cas13a, Cas13b, Cas13c, an RNA-targeted gene editing protein, and a combination thereof; and
(b) a gRNA that guides the gene editing protein to specifically bind to the DNA or RNA of the Ptbpl gene.

In another preferred embodiment, the AAV vector further includes a glial cell-specific promoter (e.g., a GFAP promoter) for driving the expression of the gene editing protein.

It should be understood that, within the scope of the present invention, each technical feature of the present invention described above and in the following (as examples) may be combined with each other to form a new or preferred technical solution, which is not listed here due to space limitations.

**Description of Figure**

Figure 1 shows that CasRx can specifically knock down Ptbpl mRNA1 in vitro. (A) schematic diagram of CasRx-mediated knockdown of Ptbpl mRNA. (B) schematic diagram showing the target sites of six gRNAs in the Ptbpl gene. (C and D) Knockdown efficiencies of different combinations of gRNAs. gRNAs 5 and 6 show the highest knockout efficiency in both N2a cells (C) and astrocytes (D). The numbers above represent the number of repetitions for each group. All values are presented as mean ± SEM. (E and F) CasRx-Ptbpl can specifically knock down Ptbpl. N2a cells (E), n = 4 independent repeats; astrocytes (F), n = 2 independent repeats.

Figure 2 shows AAV specificity and expression, GFP expression turns off over time as well as isoforms of transformed RGCs, in relation to Figure 1. (A) Expression levels of $log_2$(FPKM+1) values of all detected genes in the RNA-seq library of CasRx-Ptbpl (y-axis) compared to CasRx control (x-axis). N2a cells, n=4 independent repeats; astrocytes, n=2 independent repeats. Typical neuron-derived genes are marked with black dots. (B) Specificity of AAV-GFAP-GFP-Cre. AAV-GFAP-GFP-Cre drives GFP expression and unlocks tdTomato expression in the MG of Ai9 mice. Sox9 is an MG specific marker. Scale bar, 50 μm. (C) Determination of AAV expression. Percentage of GFP+ cells expressing tdTomato and percentage of tdTomato+ cells expressing Sox9. All values are presented as mean ± SEM. (D) qPCR analysis has confirmed that infected retinas express AAV-GFAP-CasRx and AAV-GFAP-CasRx-Ptbpl. The numbers above represent the number of repetitions for each group. All values are presented as mean ± SEM. (E) MG-induced RGCs eventually turn off GFP expression over time. scale. 20 μm. experiments are repeated 6 times independently for each group with similar results. (F-H) Staining of Foxp2, Brn3c and PV. Note that Foxp2, Brn3c and PV are markers of RGC subtype F-RGC, type 3 RGC and PV-RGC, respectively. Yellow arrows indicate tdTomato+ cells are co-localized with different markers, white arrows indicate tdTomato+ cells are co-localized with different markers. Scale bar, 20 μm. The experiment is repeated 3 times for each group with similar results.

Figure 3 shows that Ptbpl knockdown converts MGs to RGCs in the intact mature retina. (A) schematic diagram of MG to RGC conversion. Vector I (AAV-GFAP-GFP-Cre) encodes Cre recombinase and GFP driven by the MG-specific promoter GFAP, and vector II (AAV-GFAP-CasRx-Ptbp1) encodes CasRx and gRNA. To induce RGCs, retinas (5-week-old Ai9 mice) are injected with AAV-GFAP-CasRx-Ptbpl or the control vector AAV-GFAP-CasRx along with AAV-GFAP-GFP-Cre. The occurrence of conversion is checked approximately one month after injection. ONL, outer nuclear layer; OPL, outer plexiform layer; INL, inner nuclear layer; IPL, inner plexiform layer; GCL, ganglion cell layer. (B) Representative images showing co-localization of tdTomato and Brn3a in GCLs (dotted line). White arrows indicate ends of MGs that do not colocalize with Brn3a, yellow arrows indicate colocalization of tdTomato and Brn3a in AAV-GFAP-GFP-Cre and AAV-GFAP-CasRx-Ptbp1-injected retinas. Brn3a is a specific marker for RGCs. Scale bar, 20 μm. (C) Number of $tdTomato^+$ or $tdTomato^+Brn3a^+$ cells in GCL at 1 month after AAV injection. AAV-GFAP-GFP-Cre plus AAV-GFAP-CasRx, n=6 retinas; AAV-GFAP-GFP-Cre plus AAV-GFAP-CasRx-Ptbpl, n=7 retinas. Data are presented as mean ± SEM, *p<0.05, **p<0.01, **p<0.001, unpaired t-test. (D) Representative images showing the co-localization of tdTomato with Rbpms, another specific marker for RGCs in GCLs. Yellow arrows indicate co-localization of tdTomato and Rbpms. Scale bar, 20 μm. (E) Number of tdTomato+ or tdTomato+Rbpms+ cells in GCL at 1 month after AAV injection. AAV-GFAP-GFP-Cre plus AAV-GFAP-CasRx, n=6 retinas; AAV-GFAP-GFP-Cre plus AAV-GFAP-CasRx-Ptbpl, n=8 retinas. Data are presented as mean ± SEM, *p<0.05, **p<0.01, ***p<0.001, unpaired t-test.

Figure 4 shows that knockdown of Ptbpl converts MGs to RGCs in the intact retina of C57BL mice, related to Figure 3.

(A) schematic diagram of RGC induction from MG. Vector 1 (GFAP-mCherry) expresses mCherry driven by the MG-specific promoter GFAP, and vector 2 (AAV-EFS-CasRx-Ptbp1) expresses gRNA and CasRx under the promoter of Spectrum. To induce RGCs, AAV-GFAP-mCherry is injected into the retina along with AAV-EFS-CasRx-Ptbp1 or the control virus AAV-GFAP-mCherry. The occurrence of conversion is checked 2-3 weeks after injection. (B) Representative images showing the co-localization of mCherry and the MG marker Sox9, indicating that AAV-GFAP-mCherry is specifically expressed in MG. Scale bar, 50 μm. (C, D) Representative images show mCherry+Brn3a+ and mCherry+Rbpms+ cells in GCL. n=3 retinas per group. Scale bar, 50 μm. (E) RGC-like mCherry+ cells generate electrophysiological responses to LED light.

Figure 5 shows that knockdown of Ptbpl converts MGs into amacrine cells, related to Figure 3. (A) tdToma-

to+Pax6+ cells are observed in the intact retina of Ai9 mice injected with AAV-GFAP-CasRx-Ptbpl. Green arrows indicate that tdTomato+ cells do not co-localize with Pax6, yellow arrows indicate that Pax6 and tdTomato co-localize. Note that Pax6 is a marker for amacrine cells. Scale bar, 20 μm. (B) No tdTomato+Proxl+ cells are observed. Arrows indicate that tdTomato cells do not colocalize with Prox1, a marker for bipolar cells. Scale bar, 20 μm. (C) No tdTomato+ cells are observed in the photoreceptor cell layer (ONL). White arrows indicate tdTomato-positive RGC-like cells in GCL, yellow arrows indicate tdTomato+ protamine-like cells in INL, and green arrows indicate tdTomato+ projections of MG. Scale bar, 20 μm. The experiment is repeated at least 3 times independently for each group with similar results.

Figure 6 shows induction of MG to RGC conversion in a mouse model of NMDA-induced retinal injury. (A) Experimental design. Retinal damage is induced in 4-8 week old Ai9 mice by intravitreal injection of NMDA (200 mM, 1.5 mL). Two to three weeks after NMDA injection, AAV is injected subretinal. Immunostaining and behavioral tests are performed 1 month after AAV injection. (B) NMDA injection has essentially killed most of the RGCs in the GCL. Scale bar, 50 μm. (C) Co-localization of tdTomato and Brn3a. Yellow arrows indicate co-localization of Brn3a and tdTomato in GCLs. n=6 retinas, scale bar, 20 μm. (D) Number of Brn3a+ or tdTomato+ or tdTomato+ Brn3a+ cells in GCL. Uninjured retina, n=6 retinas; GFAP-CasRx plus GFAP-GFP-Cre, n=6 retinas; GFAP-CasRx-Ptbpl plus GFAP-GFP-Cre, n=7 retinas. Data are presented as mean ± SEM, *p<0.05, **p<0.01, ***p<0.001, unpaired t-test. (E) Co-localization of tdTomato and Rbpms. Yellow arrows indicate co-localization of Rbpms and tdTomato in GCL injected with GFAP-CasRx-Ptbpl plus GFAP-GFP-Cre. Scale bar, 20 μm. (F) Number of Rbpms+ or tdTomato+ or tdTomato+Rbpms+ cells in GCL. Uninjured retinas, n=6 retinas; GFAP-CasRx plus GFAP-GFP-Cre, n=7 retinas; GFAP-CasRx-Ptbpl plus GFAP-GFP-Cre, n=7 retinas. Data are presented as mean ± SEM, *p<0.05, **p<0.01, ***p<0.001, unpaired t-test. (G) Image shows representative RGC-like tdTomato+ cells recorded under two-photon microscopy. Scale bar, 20 μm. (H) Induced retinal ganglion cell response to light. A total of 8 cells are recorded, 6 of which show responses to LED light. Among these cells, 5 of which are ON cells and 1 of which is OFF cell.

Figure 7 shows the progress of the MG to RGC conversion, which is related to Figure 6. (A) Representative images showing the conversion of MGs to RGCs in intact retina at five different time points (without NMDA injection). Arrows indicate induced RGCs. Scale bar, 20 μ m. Experiments are independently repeated ≥3 times with similar results. Note that representative pictures from "2 months" are also shown in Figures 2B and 2D. (B) Absolute numbers of tdTomato+Brn3a+ and tdTomato+Rbpms+ cells in GCL. Note that the values in "1 month" are also shown in Figures 1C and 3E. All values are presented as mean ± SEM. (C) Representative images showing intermediate stages of induced cells (white arrows) at 1.5 weeks after AAV injection. n>=3 mice per group. Note that intermediate units often lose their projections in ONL. Scale bar, 10 μ m. Experiments are independently repeated ≥ 3 times with similar results. (D) Representative images showing the conversion of MGs to RGCs in NMDA-injured retinas at four different time points. Arrows indicate induced RGCs. Scale bar, 20 μ m. Note that representative images in "3 months" are also shown in Figures 3C and 3E. Experiments are independently repeated ≥2 times with similar results.

Figure 8 shows that converted RGCs can project to the brain and partial restoration of visual function. (A) Schematic diagram of the visual pathway. The RGCs send axons through the optic nerve that transmits optic nerve signals into the dorsal geniculate nucleus and superior colliculus outside the retina. (B) Retinal tiling figure. Yellow arrows indicate MG-derived tdTomato-positive RGC axons. Scale bar, 100 μm. The experiment is repeated 3 times for each group with similar results. (C) Representative images showing tdTomato+ axons of RGCs induced in the optic nerve. Scale bar, 200 μm. Each group is repeated 5 times independently with similar results. (D and E) Representative images showing strong signals are observed in the contralateral dorsal geniculate nucleus (D) and superior colliculus (E) (target areas of RGC projections in the brain). Each group is repeated 4 times independently with similar results. Note that the ipsilateral dorsal geniculate nucleus and SC show weak tdTomato+ signal. Scale bars, 500 μm (left), 50 μm (right). (F) schematic diagram of VEP recordings. (G) VEP sending in primary visual cortex. Responses from the same group of mice are shown, with each line representing a retina. The top of the histogram represents the number of retinas in each group. (H) Wild type (WT, n=8 retinas), NMDA and AAV-GFAP-mCherry (n=12 retinas), NMDA, AAV-GFAP-mCherry and AAV-GFAP-CasRx (n=11 retinas), and NMDA, AAV-GFAP-mCherry and AAV-GFAP-CasRx-Ptbpl (n = 8 retinas). Each point represents a mouse. Data are presented as mean ± SEM, *p<0.05, **p<0.01, ***p<0.001, unpaired t-test. (I) Design of dark/light preference test. Note that both eyes are treated with NMDA and injected with the same AAV 2 weeks after NMDA injection. (J) Percentage of time spent in the dark room. WT, n=13 mice; NMDA and GFAP-mCherry, n=14 mice; NMDA, AAV-GFAP-mCherry and AAV-GFAP-CasRx, n=12 mice; NMDA, AAV-GFAP-mCherry and AAV-GFAP-CasRx-Ptbpl, n = 12 mice. All values are presented as mean ± SEM; unpaired t-test; *p<0.05, **p<0.01, ***p<0.001.

Figure 9 shows the projection of induced RGCs to the optic nerve and brain. (A) Representative images are shown at five different time points (1 week, 1.5 weeks, 2 weeks, 3 weeks and 1 month). tdTomato+ axons (yellow arrows) are first seen 1.5 weeks after AAV injection. Scale bar, 50 μm. The experiment is repeated 3 times independently with similar results. (B) A gradual increase in the density of tdTomato+ axons (yellow arrows) in the dorsal geniculate nucleus. Note that tdTomato+ axons are first observed in the contralateral dorsal geniculate nucleus 1.5 weeks after AAV injection. Scale bars, 500 μm (upper), 50 μm (lower). The experiment is repeated 3 times independently with similar results. (C) Projection of tdTomato+ axons (yellow arrows) to SC. Note that tdTomato+ axons are first observed in the contralateral SC 2 weeks after AAV injection. Scale bars, 500 μm (upper), 50 μm (lower). The experiment is repeated 3 times independently with similar results. (D) schematic diagram showing the stepwise projection of induced RGCs.

Figure 10 shows the progression of RGC projections induced in the damaged retina, related to Figure 9. (A) Representative images showing a gradual increase in tdTomato+ axons in NMDA-induced optic nerves at three different time points (1 week, 2 weeks, 3 weeks). Scale bar, 50 μm. The experiment is repeated 2 times independently with similar results. (B) The density of tdTomato+ axons (yellow arrows) gradually increases in the dorsal geniculate nucleus. Scale bars, 500 μm (upper), 50 μm (lower). The experiment is repeated 2 times independently with similar results. (C) Projection progression of tdTomato+ axons (yellow arrows) in the superior colliculus. Scale bars, 500 μm (upper), 50 μm (lower). The experiment is repeated 2 times independently with similar results.

Figure 11 shows CasRx-mediated conversion of glial cells to neurons. (A, B) schematic diagram of the injection strategy. The efficiency of conversion was assessed approximately one month after injection. ST, striatum. (C) Colocalization of mCherry and GFAP. Percentage of mCherry+ cells expressing GFAP, n=3 mice. Scale bar, 20 μm. (D) Flag (fused to CasRx) and GFAP co-localized one week after AAV injection. Scale bar, 20 μm. (E) Ptbpl expression (detected by Ptbpl antibody) in the striatum is downregulated one week after AAV injection. The expression of CasRx (fused to Flag) is detected by anti-Flag-tagged antibody. n=4 mice per group. Scale bar, 10 μm. (F) Quantification of Ptbpl fluorescence intensity using ImageJ. a.u. stands for any units. (G) Representative images of the striatum. NeuN is a specific marker for mature neurons. White arrows indicate that NeuN expression does not co-localize with mCherry, and yellow arrows indicate that NeuN and mCherry co-localize. Scale bar, 50 μm. (H) Percentage of mCherry+NeuN+ cells in mCherry+ cells (n=6 mice per group; t=-4.7, p<0.001). (I) Image showing mCherry+NeuN+glutaminase-positive cells (pink arrows) adjacent to mCherry+NeuN+glutaminase-negative cells (yellow arrows). Scale bar, 10 μm. (J) Percentage of mCherry+glutaminase+NeuN+ cells in mCherry+NeuN+ cells. N=5 mice. (K) Representative image showing that mCherry+NeuN+ cells rarely co-localize with somatostatin. Yellow arrows indicate mCherry+NeuN+Somatostatin- cells and pink arrows indicate mCherry+NeuN+Somatostatin+ cells. Control AAV shows the absence of mCherry+SST+ cells, indicating that SST+ cells can not be infected by AAV-GFAP-mCherry. The experiment is repeated 5 times independently with similar results. Scale bar, 20 μm. (L) Representative image showing that mCherry+NeuN+ cells do not co-localize with Palvabumin. The experiment is repeated 4 times independently with similar results. Scale bar, 20 μm. (M) mCherry+TH+ cells (white arrows) are observed in the intact striatum. Scale bar, 20 μm. (N) Percentage of mCherry+TH+ cells in mCherry+ cells, n=5 mice per group. (O) Typically, mCherry+TH+ cells show very low (upper, white arrows) or undetectable levels (lower, yellow arrows) of NeuN expression. Scale bar, 5 μm. The experiment is repeated 5 times independently with similar results. All values are presented as mean ± SEM; unpaired t-test; *p<0.05, **p<0.01, ***p<0.001.

Figure 12 shows the conversion of astrocytes to dopamine neurons in Parkinson's model mice. (A and B) Experiment overview. 6-OHDA is injected unilaterally into the substantia nigra. After 3 weeks, AAV-GFAP-CasRx-Ptbpl plus AAV-GFAP-mCherry, AAV-GFAP-CasRx plus AAV-GFAP-mCherry or normal saline is injected into the striatum of the ipsilateral (relative to the 6-OHDA injected side) of the rat. (B) Immunostaining is performed approximately 1 or 3 months after AAV injection. (C) Confocal images of converted mCherry+TH+ (yellow arrows) and mCherry+DAT+ (yellow arrows) cells 1 or 3 months after AAV injection. Orange arrows indicate synapses of converted mCherry+TH+DAT+ cells. Note that TH and DAT are dopamine neuron-specific markers. Scale bar, 50 μm or 10 μ m. (D) Percentage of mCherry+TH+ cells in mCherry+ cells. AAV-GFAP-CasRx, 1 month: n=5 mice; AAV-GFAP-CasRx-Ptbpl, 1 month: n=5 mice; AAV-GFAP-CasRx-Ptbpl, 3 months: n= 3 mice. (E) Percentage of mCherry+TH+ cells in TH+ cells, n=5 mice per group. Scale bar, 50 μm. (F) Percentage of mCherry+DAT+ cells in mCherry+ cells. AAV-GFAP-CasRx, 1 month: n=5 mice; AAV-GFAP-CasRx-Ptbpl, 1 month: n=5 mice; AAV-GFAP-CasRx-Ptbpl, 3 months: n= 3 mice. (G) Percentage of mCherry+DAT+TH+ cells in mCherry+TH+ cells. AAV-GFAP-CasRx, 1 month: n=5 mice; AAV-GFAP-CasRx-Ptbpl, 1 month: n=5 mice; AAV-GFAP-CasRx-Ptbpl, 3 months: n= 3 mice. (H) Representative images showing that ALDH1A1 and GIRK2 expression are colocalized with mCherry+TH+ (yellow arrow), whereas Calbindin expression (orange arrow) does not colocalize with mCherry+DAT+. ALDH1A1 and GIRK2 are dopamine neuron-specific markers in the SNc A9 region, while calbindin is a VTA dopamine neuron-specific marker. n=3 mice per group. Scale bar, 50 μm. (I) Percentage of mCherry+ALDH1A1+, mCherry+GIRK2+ and mCherry+calbindin+ cells in mCherry+ cells. n=3 mice per group. (J) Percentage of mCherry+TH+ALDH1A1+ and mCherry+TH+GIRK2+ in mCherry+TH+ cells; and mCherry+DAT+calbindin+ cells in mCherry+DAT+ cells. n=3 mice per group. (K) Whole-cell recordings are performed in sections of striatum (n = 22 cells). The figure shows the ability of neuron-like mCherry+ cells to generate repetitive action potentials (20 of 22 cells) and rectification (4 of 10 cells, green). (L) Image showing spontaneous synaptic currents in induced neurons. (M) Hyperpolarized voltage-gated currents. (N) Representative images showing mCherry+TH+ cells expressing VMAT2 (yellow arrows) and the percentage of mCherry+VMAT2+TH+ cells in mCherry+TH+ cells, n=3 mice. Scale bar, 20 μm. (o) Image showing uptake of FFN206 (blue), a fluorescent dopamine derivative, by mCherry+ cells 1 month after AAV injection. n >= 10 sections per group. Scale bars, 30 μm (upper), 20 μm (lower). (P) FFN206 can be released in mCherry+ cells (yellow arrow). Images show high KCl-induced release of FFN206. Scale bar, 10 μm. (Q) Statistical analysis of KCl-induced FFN206 release in sections injected with AAV-GFAP-mCherry plus AAV-GFAP-CasRx-Ptbpl, n=25 mCherry+blue+ cells. 25 cells are analyzed from 5 sections (n=3 mice). All values are expressed as mean ± SEM. Unpaired t-test; *p<0.05, **p<0.01, ***p<0.001.

Figure 13 shows that knockdown of Ptbpl converts astrocytes into dopamine neurons in a 6-OHDA-induced Parkinson's disease mouse model. (A) Schematic diagram of the experiment. (B) Staining shows the death of TH+ neurons (green) on the ipsilateral substantia nigra (relative to the 6-OHDA-injected side). Scale bar, 100 μm. The experiment is independently repeated 12 times with similar results. (C) DAT staining shows disappearance of dopamine neuronal fibers (green) in the ipsilateral striatum. Scale bar, 500 μm. Experiments are independently repeated >10 times with similar results. (D, E) Representative confocal images showing quantification of mCherry+TH+ and mCherry+DAT+ cells and mCherry+TH+ and mCherry+DAT+ cells at various time points after AAV injection. n>=3 mice per group. Scale bar, 50 μm. Note that the data "GFAP-CasRx, 1 month", "GFAP-CasRx-Ptbpl, 1 month" and "GFAP-CasRx-Ptbpl, 3 months" are also shown in Figures 6C, 6D and 6. Scale bar, 50 μm. (F) Absolute numbers of TH+ cells in Parkinson's disease model mice. (G) Confocal images of mCherry+TH+ cells and percentage of mCherry+TH+ cells in TH+ cells, n=5 mice per group. Scale bar, 50 μm. (H) Percentage of mCherry+TH+ cells in mCherry+ cells. Note that the data are also shown in Figure 6D and S6N. (I) Absolute numbers of DAT+ cells in PD model mice. (J, K) Confocal images of mCherry+DDC+ cells and percentage of mCherry+DDC+ cells in mCherry+ cells, n=5 mice per group. DDC is a dopamine neuron marker. Scale bar, 50 μm. (L, M) Representative images show the co-localization of FOXA2 and mCherry (yellow arrows), and the percentage of mCherry+FOXA2+ cells in cells. n=5 mice per group. FOXA2 is a dopamine neuron marker. Scale bar, 30 μm. (N) Representative images showing that mCherry+TH+ cells do not co-localize with representative striatal interneuron markers PV, SST and CR. Yellow arrows indicate mCherry+TH+ cells and blue arrows indicate PV+, SST+ or CR+ cells. Note that SST is colocalized with mCherry, but not TH, consistent with the results in Figure S1H, suggesting that knockout of Ptbpl sparsely induces SST+ neurons. The experiment is repeated 3 times independently with similar results. Scale bar, 20 μm. (O) Comparison of net conversions 1 month before and after AAV injection. The numbers above the dots indicate the number of mice in each group. Paired t-test. The data for "1 month" is also shown in Figure 7. (P) Net rotation (rev/min) induced by apomorphine injection. Behavior is assessed at 1 and 3 months per mouse, n=3 mice per group. Two-way ANOVA and bonferroni's test. The data for "1 month" is also shown in Figure 7. (Q) Net rotations (revolutions per minute) induced by amphetamine injection, n = 3 mice per group. Two-way ANOVA and bonferroni's test. The data for "1 month" is also shown in Figure 7. All values are presented as mean ± SEM; unpaired t-test; *p<0.05, **p<0.01, ***p<0.001.

Figure 14 shows that induced neurons alleviate motor dysfunction in Parkinson's disease model mice. (A) Experimental design. (B) Net rotation (contralateral-ipsilateral) induced by apomorphine injection. (C) Net rotation (ipsilateral-contralateral) induced by amphetamine injection. (D) Percentage of ipsilateral rotations relative to total rotations (ipsilateral/total rotations) following systemic injection of amphetamine. (E) Percentage of spontaneous ipsilateral touches relative to the total number of touches. (F) Rota-Rod Treadmills test. Results are expressed as the time (in seconds) that the mouse has stayed on the accelerated Rota-rod before falling. The numbers above the bars indicate the number of mice in each group. All values are presented as mean ± SEM. One-way ANOVA followed by Tukey's test. *p<0.05, **p<0.01, ***p<0.001.

Figure 15 shows an off-target detection scheme for the in vitro Ptbpl knockdown tool. The plasmids of the test group are transfected into N2a cells (1), and the cells are harvested 48 hours later, and the transfected positive cells are sorted by flow cytometry (2), and RNA is extracted for transcriptome sequencing (3, RNA-seq) analysis. In order to detect the off-target effect of knockdown of Ptbpl by different tools, we set up a Cas13e-sg (Ptbpl-target) experimental group and a Cas13e-sg (none-target) control group. Meanwhile, for comparison with other gene editing tools, we set up Cas13d-sg (Ptbpl-target) and Cas13d-sg (none-target) controls and shRNA (Ptbpl-target) and shRNA (none-target) controls. The results indicate that the Cas13e-sg (Ptbpl-target) editing tool combination has low off-target effects.

Figure 16 shows the detection scheme for transdifferentiation efficiency following Ptbpl knockdown in vivo. In this experiment, a 6-OHDA-induced Parkinson's mouse model is used to simulate the Parkinson's disease phenotype by injecting 6-OHDA into mouse MFB (Medial Forebrain Bundle) to damage dopamine neurons. After 21 days of induction of the model, the experimental group and the control group are injected with AAV virus in the striatum of mice, respectively and the AAV virus that specifically labels glial cells (GFAP promoter-driven expression of mCherry) is co-injected. 28 or 90 days after virus injection, in vivo transdifferentiation efficiency is verified by behavioral assay analysis and tissue staining analysis. The editing tools involved in this experiment all use AAV vectors. The experiment set up the Cas13e-sg (Ptbpl-target) experimental group, as well as Cas13e-sg (none-target), Cas13d-sg (Ptbpl-target) and Cas13d-sg (none-target) control group. The above four groups of gene editing tools are all driven by the glial cell-specific GFAP promoter to express the Cas13e/d protein, and the expression of the corresponding sg is driven by the U6 promoter. At the same time, shRNA (Ptbpl-target) and shRNA (none-target) control groups are added, and the expression is driven by the U6 promoter. The results indicate that the Cas13e-sg (Ptbpl-target) editing tool combination can more effectively achieve transdifferentiation of glial cells in vivo.

**Detail description**

After extensive and in-depth research, the present inventors have unexpectedly discovered for the first time that inhibiting the expression or activity of the ptbpl gene or RNA or an encoded protein thereof in glial cells can effectively induce the differentiation of glial cells into functional neurons, thereby treating a nervous system disease related to functional neuronal death. On this basis, the present inventors have completed the present invention.

In the present invention, retinal ganglion cell (RGC) degeneration is the main cause of permanent blindness. The transdifferentiation of Müller glial cells (MG) into functional RGCs can help restore vision. The inventors have found that knockdown of *Ptbpl* using the RNA-targeted CRISPR system CasRx in the mature mouse retina directly converts MGs into functional RGCs. Furthermore, in a mouse model of retinal damage induced by NMDA (N-methyl-d-aspartate), RGCs converted from MG have achieved functional projection to the central visual area and improved visual function. Therefore, CasRx-mediated knockdown of Ptbpl will be a promising therapy for retinal diseases caused by neurodegeneration.

The present invention uses CasRx, a recently characterized RNA-targeting CRISPR system, for the inhibition of *Ptbpl.* CasRx-mediated regeneration avoids the occurrence of substantial off-target effects induced by shRNA and the risk of permanently altering genes by DNA-editing nucleases, providing an excellent tool capable of treating a variety of diseases.

As used herein, Müller glial cell (MG) are the main glial cells in retinal tissue, retinal ganglion cells (RGC) are nerve cells located in the innermost layer of the retina, and its dendrites are mainly associated with bipolar cells, Its axons extend to the papilla of optic nerve to form the optic nerve.

Retinal disease is an eye disease. The five most common retinal diseases are: ① vascular and vascular system lesions. Such as retinal vascular occlusion, arteriosclerotic, hypertensive, hematological and diabetic fundus lesions and the like. ② retinal inflammation, closely related to the interaction of choroiditis and optic neuritis. ③ retinal detachment. Refers to the separation of the retinal nervous layer and the pigment epithelium layer. ④ retinal degeneration and malnutrition, having genetic factors. ⑤ retinal tumors. Among them, retinoblastoma is the most common.

In the present invention, the preferred retinal diseases are retinal diseases caused by neurodegeneration, and the symptoms are mainly manifested in visual deterioration or blindness.

In the present invention, the gene editors include DNA gene editors and RNA gene editors. In a preferred embodiment, the gene editor of the present invention includes a gene editing protein and optionally a gRNA.

The terms "reprogramming" or "transdifferentiation" can refer to the process of generating cells of a particular lineage (e.g., neuronal cells) from different types of cells (e.g., fibroblasts) without intermediate differentiation.

**Nervous system disease related to functional neuronal death**

In the present invention, a nervous system disease related to functional neuronal death mainly includes Parkinson's disease and visual impairment caused by optic ganglion death.

In a preferred embodiment, a nervous system disease related to functional neuronal degeneration includes, but is not limited to, glaucoma, age-related RGC loss, injury of optic nerve, retinal ischemia, Leber hereditary optic neuropathy, Alzheimer's disease, Huntington's disease, schizophrenia, depression, drug taking, movement disorders (such as chorea, hypercholesterolemia, and movement disorders), motor neuron injury diseases (such as amyotrophic lateral sclerosis, spinal cord injury), bipolar disorder, autism spectrum disorder (ASD), dysfunction, Parkinson's disease.

**astrocytes**

Astrocytes are the most abundant type of cells in the mammalian brain. They perform many functions, including biochemical support (such as forming the blood-brain barrier), providing nutrients to neurons, maintaining extracellular ion homeostasis, and participating in repair and scarring following brain and spinal cord injury. According to the content of glial filaments and the shape of cytoplasmic process, astrocytes can be divided into two types: fibrous astrocytes, which are mostly distributed in the white matter of the brain and spinal cord, with elongated cytoplasmic process and fewer branches, contains a lot of glial filaments in the cytoplasm; protoplasmic astrocytes, which are mostly distributed in the gray matter, with thick and short cytoplasmic process and many branches.

The astrocytes that can be used in the present invention are not particularly limited, including various astrocytes derived from the central nervous system of mammals, such as those derived from the striatum, spinal cord, dorsal midbrain or cerebral cortex, preferably, from the striatum.

**functional neuron**

In the present invention, functional neurons may refer to neurons capable of sending or receiving information through chemical or electrical signals. In some embodiments, functional neurons exhibit one or more functional properties of mature neurons present in the normal nervous system, including, but not limited to: excitability (e.g., the ability to exhibit action potentials, such as rapid rise and subsequent fall) (voltage or membrane potential across cell membranes), formation of synaptic connections with other neurons, presynaptic neurotransmitter release, and postsynaptic responses (e.g., excitatory postsynaptic currents or inhibitory postsynaptic currents).

In some embodiments, functional neurons are characterized in that they express one or more markers of functional neurons, including but not limited to synapsin, synaptophysin, glutamate decarboxylase 67 (GAD67), glutamate decarboxylase 67 (GAD65), parvalbumin, dopamine- and cAMP-regulated neuronal phosphoprotein 32 (DARPP32), vesicular glutamate transporter 1 (vGLUTI), vesicular glutamate transporter 2 (vGLUT2) , acetylcholine, tyrosine hydroxylase (TH), dopamine, vesicular GABA transporter (VGAT) and $\gamma$ -aminobutyric acid (GABA).

**dopamine neurons**

Dopaminergic neurons contain and release dopamine (DA) as a neurotransmitter neuron. Dopamine is a catecholamine neurotransmitter and plays an important biological role in the central nervous system. The dopaminergic neurons in the brain are mainly concentrated in the substantria nigra pars compacta (SNc) of the midbrain and ventral tegmental area (VTA), hypothalamus and periventricular. Many experiments have confirmed that dopaminergic neurons are closely related to a variety of human diseases, the most typical of which is Parkinson’s disease.

**neurodegenerative disease**

Neurodegenerative diseases are diseases caused by the loss of neurons in the brain and spinal cord. Neuron is the most important part of the nervous system and its death will eventually lead to the dysfunction of the nervous system. After the patient suffers from neurodegenerative disease, there will be movement or cognitive disorder, and the development of the disease often leads to many complications, causing serious damage to the patient’s life. Clinically, neurodegenerative diseases mainly include Alzheimer’s disease, Parkinson’s disease, Huntington’s disease, amyotrophic lateralizing sclerosis, and multiple sclerosis and the like. At present, neurodegenerative diseases can only be alleviated or delayed the progression of the disease, but cannot be completely cured. Parkinson’s disease (PD) is a severe neurodegenerative disorder characterized by the loss of midbrain substantia nigra dopamine neurons.

**gene editor**

In the present invention, the gene editors include DNA gene editors and RNA gene editors. In a preferred embodiment, the gene editor of the present invention includes a gene editing protein and optionally a gRNA.

**gene editing protein**

In the present invention, the nucleotides of the gene editing protein can be obtained by genetic engineering techniques, such as genome sequencing, polymerase chain reaction (PCR), etc., and the amino acid sequence thereof can be derived from the nucleotide sequence. The source of the wild-type gene editing protein includes (but is not limited to): *Ruminococcus Flavefaciens, Streptococcus pyogenes, Staphylococcus aureus, Acidaminococcus sp, Lachnospiraceae bacterium*.

In a preferred embodiment of the present invention, the gene editing proteins include, but are not limited to, Cas13d, CasRx, Cas13e, CRISPR/Cas9, Cpfl, Cas9, Cas13a, Cas13b, Cas13c, Cas13f, and RNA-targeted gene editing proteins.

**ptbp1 protein and polynucleotides**

In the present invention, the terms "protein of the present invention", "ptbpl protein", "ptbpl polypeptide" and "PTB" are used interchangeably, and all refer to a protein or polypeptide having an amino acid sequence of ptbpl. They include the ptbpl protein with or without the starting methionine. In addition, the term also includes full-length ptbpl and fragments thereof. The ptbpl protein referred to in the present invention includes its complete amino acid sequence, its secreted protein, its mutants and its functionally active fragments.

The ptbpl protein is polypyrimidine tract binding protein 1, an RNA-binding protein that regulates the regulation of RNA splicing. At the same time, it also plays a very critical role in other functions of RNA.

In the present invention, the terms "ptbpl gene", "ptbpl polynucleotide" and "PTB gene" are used interchangeably, and all refer to a nucleic acid sequence having a ptbpl nucleotide sequence.

The full-length genome of the human ptbpl gene is 14936 bp (NCBI GenBank accession number is 5725).

The full-length genome of the mouse ptbpl gene is 10004 bp (NCBI GenBank accession number is 19205).

Human and murine ptbpl share 88% similarity at the DNA level and 84% protein sequence similarity. It is understood that substitution of nucleotides in codons is acceptable when encoding the same amino acid. It is also to be understood that nucleotide changes are also acceptable when conservative amino acid substitutions result from nucleotide substitutions.

When the amino acid fragment of ptbpl is obtained, a nucleic acid sequence encoding it can be constructed based on it, and a specific probe can be designed based on the nucleotide sequence. The full-length nucleotide sequence or its fragment can usually be obtained by PCR amplification method, recombinant method or artificial synthesis method. For the PCR amplification method, primers can be designed according to the nucleotide sequence of ptbpl disclosed in the present invention, especially the open reading frame sequence, and using a commercially available cDNA library or a cDNA library prepared by conventional methods known to those skilled in the art as a template to amplify the relevant sequences. When the sequence is long, it is often necessary to perform two or more PCR amplifications, and then splicing the amplified fragments together in the correct order.

Once the relevant sequences have been obtained, recombinant methods can be used to obtain the relevant sequences in bulk. This is usually done by cloning it into a vector, transferring it into a cell, and isolating the relevant sequence from the propagated host cell by conventional methods.

In addition, synthetic methods can also be used to synthesize the relevant sequences, especially when the fragment length is short. Often, fragments of very long sequences are obtained by synthesizing multiple small fragments followed by ligation.

At present, the DNA sequences encoding the proteins of the present invention (or fragments or derivatives thereof) can be obtained completely by chemical synthesis. This DNA sequence can then be introduced into various existing DNA molecules (e.g., vectors) and cells known in the art.

The polynucleotide sequences of the invention can be used to express or produce recombinant ptbpl polypeptides by conventional recombinant DNA techniques. Generally there are the following steps:

(1). A suitable host cell is transformed or transduced with the polynucleotide (or variant) encoding the human ptbpl polypeptide of the present invention, or with a recombinant expression vector containing the polynucleotide;
(2). Host cells cultured in a suitable medium;
(3). Separation and purification of proteins from culture medium or cells.

In the present invention, the ptbpl polynucleotide sequence can be inserted into a recombinant expression vector. In short, any plasmid and vector can be used as long as it is replicable and stable in the host. An important feature of expression vectors is that they typically contain an origin of replication, a promoter, marker genes and translational control elements.

Methods well known to those skilled in the art can be used to construct expression vectors containing the ptbpl-encoding DNA sequence and appropriate transcriptional/translational control signals. These methods include in vitro recombinant DNA technology, DNA synthesis technology, in vivo recombinant technology, and the like. The DNA sequence can be operably linked to an appropriate promoter in an expression vector to direct mRNA synthesis. Expression vectors also include a ribosome binding site for translation initiation and a transcription terminator.

In addition, the expression vector preferably contains one or more selectable marker genes to provide phenotypic traits for selection of transformed host cells, such as dihydrofolate reductase for eukaryotic cell culture, neomycin resistance, and green fluorescent protein (GFP), or for tetracycline or ampicillin resistance in E. coli.

Vectors comprising the appropriate DNA sequences as described above, together with appropriate promoter or control sequences, can be used to transform appropriate host cells so that they can express the protein.

Host cells can be prokaryotic cells, such as bacterial cells; or lower eukaryotic cells, such as yeast cells; or higher eukaryotic cells, such as mammalian cells. Representative examples are: Escherichia coli, bacterial cells of Streptomyces; fungal cells such as yeast; plant cells; insect cells; animal cells and the like.

Transformation of host cells with recombinant DNA can be performed using conventional techniques well known to those skilled in the art. When the host is a prokaryote such as E. coli, competent cells that can take up DNA can be harvested after exponential growth phase and treated with the $CaCl_2$ method using procedures well known in the art. Another way is to use $MgCl_2$. If desired, transformation can also be performed by electroporation. When the host is a eukaryote, the following DNA transfection methods can be used: calcium phosphate co-precipitation method, conventional mechanical methods such as microinjection, electroporation, liposome packaging, etc.

The obtained transformants can be cultured by conventional methods to express the polypeptides encoded by the genes of the present invention. The medium used in the culture can be selected from various conventional culture medium depending on the host cells used. Cultivation is carried out under conditions suitable for growth of the host cells. After the host cells have grown to an appropriate cell density, the selected promoter is induced by a suitable method (e.g., temperature switching or chemical induction), and the cells are cultured for an additional period of time.

The recombinant polypeptide in the above method can be expressed intracellularly, or on the cell membrane, or secreted outside the cell. If desired, recombinant proteins can be isolated and purified by various isolation methods utilizing their physical, chemical and other properties. These methods are well known to those skilled in the art. Examples of these methods include, but are not limited to: conventional renaturation treatment, treatment with protein precipitants (salting-out method), centrifugation, osmotic disruption, ultratreatment, ultracentrifugation, molecular sieve chromatography (gel filtration), adsorption chromatography, ion exchange chromatography, high performance liquid chromatography (HPLC) and various other liquid chromatography techniques and combinations of these methods.

**adeno-associated virus**

Because adeno-associated virus (AAV) is smaller than other viral vectors, non-pathogenic, and can transfect both dividing and non-dividing cells, AAV vector-based gene therapy approaches for inherited diseases have received extensive attention.

Adeno-associated virus (AAV), also known as adeno associated virus, belongs to the Dependovirus of the Parvoviridae, and is the simplest single-stranded DNA-deficient virus discovered so far, requiring a helper virus (usually adenovirus) for replication. It encodes the cap and rep genes in 2 inverted terminal repeats (ITR). ITRs play a decisive role in viral replication and packaging. The cap gene encodes the viral capsid protein, and the rep gene is involved in the replication and integration of the virus. AAV can infect a variety of cells.

Recombinant adeno-associated virus vector (rAAV) is derived from non-pathogenic wild-type adeno-associated virus, due to its good safety, wide range of host cells (dividing and non-dividing cells), low immunogenicity, and long time to express foreign genes in vivo, it is regarded as one of the most promising gene transfer vectors and has been widely used in gene therapy and vaccine research worldwide. After more than 10 years of research, the biological characteristics of recombinant adeno-associated virus have been deeply understood, especially its application effect in various cells, tissues and in vivo experiments has accumulated a lot of information. In medical research, rAAV is used for gene therapy research of various diseases (including in vivo and in vitro experiments); at the same time, as a characteristic gene transfer vector, it is also widely used in gene function research, construction of disease models, and preparation of gene knockout mice.

In a preferred embodiment of the present invention, the vector is a recombinant AAV vector. AAVs are relatively small DNA viruses that can integrate into the genomes of the cells they infect in a stable and site-specific manner. They are capable of infecting a wide range of cells without any effect on cell growth, morphology or differentiation, and they do not appear to be involved in human pathology. The AAV genome has been cloned, sequenced and characterized. AAV contains an inverted terminal repeat (ITR) region of approximately 145 bases at each end, which serves as the viral origin of replication. The rest of the genome is divided into two important regions with encapsidation functions: the left part of the genome containing the rep gene involved in viral replication and viral gene expression; and the right part of the genome containing the cap gene encoding the viral capsid protein.

AAV vectors can be prepared using standard methods in the art. Any serotype of adeno-associated virus is suitable. Methods for purifying vectors can be found, for example, in US Patent Nos. 6,566,118, 6,989,264, and 6,995,006, the disclosures of which are incorporated herein by reference in their entirety. The preparation of hybrid vectors is described, for example, in PCT Application No. PCT/US2005/027091, the disclosure of which is incorporated herein by reference in its entirety. The use of AAV-derived vectors for gene transfer in vitro and in vivo has been described (see, e.g., International Patent Application Publication Nos. WO91/18088 and WO93/09239; US Patent Nos. 4,797,368, 6,596,535 and 5,139,941, and European Patent No. 0488528, all of which are incorporated herein by reference in their entirety). These patent publications describe various AAV-derived constructs in which the rep and/or cap genes are deleted and replaced by genes of interest, as well as the use of these constructs to transfer genes of interest in vitro (into cultured cells) or in vivo (directly into organisms). Replication-deficient recombinant AAVs can be prepared by co-transfection of the following plasmids into cell lines infected with human helper virus (e.g., adenovirus): a plasmid containing a nucleic acid sequence of interest flanked by two AAV inverted terminal repeat (ITR) regions, and a plasmid carrying the AAV encapsidation genes (rep and cap genes). The resulting AAV recombinants are then purified by standard techniques.

In some embodiments, the recombinant vector is encapsidated into a virion (e.g., including, but not limited to, AAV virions of AAV1, AAV2, AAV3, AAV4, AAV5, AAV6, AAV7, AAV8, AAV9, AAV10, AAV11, AAV12, AAV13, AAV14, AAV15, and AAV16). Accordingly, the present disclosure includes recombinant virions (recombinant because they contain recombinant polynucleotides) containing any of the vectors as described herein. Methods of producing such particles are known in the art and described in US Patent No. 6,596,535.

**ptbp1 inhibitors and pharmaceutical compositions**

Using the protein of the present invention, through various conventional screening methods, substances that interact with the ptbpl gene or protein, especially inhibitors, can be screened.

The ptbpl inhibitor (or antagonist) that can be used in the present invention includes any substance that can inhibit the expression and/or activity of the ptbpl gene or its encoded protein.

For example, the inhibitor of ptbpl includes an ptbpl antibody, an antisense RNA of ptbpl nucleic acid, siRNA, shRNA, miRNA, a gene editor, or an active inhibitor of ptbpl. A preferred inhibitor of ptbpl refers to a gene editor capable of inhibiting the expression of ptbpl.

Preferably, the ptbpl inhibitors of the present invention include inhibitors targeting positions 4758-4787 and/or 5381-5410 of the ptbpl gene sequence. The objects on which the ptbpl inhibitor of the present invention acts include astrocytes or MG cells.

In a preferred embodiment, the methods and steps for inhibiting ptbpl include neutralizing its protein with an antibody against ptbp1, and silencing the ptbpl gene with shRNA or siRNA or a gene editor carried by a virus (such as adeno-associated virus).

The inhibition rate of ptbpl is generally at least 50% inhibition, preferably 60%, 70%, 80%, 90%, 95% inhibition, the inhibition rate of ptbpl can be controlled and detected based on conventional techniques, such as flow cytometry, fluorescence quantitative PCR or Western blot.

The inhibitors of the ptbpl protein of the present invention (including antibodies, antisense nucleic acids, gene editors and other inhibitors), when administered therapeutically (dosing), can inhibit the expression and/or activity of the ptbpl protein, thereby inducing differentiation of glial cells into functional neurons to treat nervous system diseases associated with functional neuronal degeneration. Generally, these materials can be formulated in a non-toxic, inert and pharmaceutically acceptable aqueous carrier medium, usually at a pH of about 5-8, preferably at a pH of about 6-8, although pH can vary depending on the nature of the substance being formulated and the condition being treated. The formulated pharmaceutical compositions can be administered by conventional routes, including (but not limited to): topical, intramuscular, intraperitoneal, intravenous, subcutaneous, intradermal, topical administration, and infusion of autologous cells after extraction and culture and the like.

The present invention also provides a pharmaceutical composition, which contains a safe and effective amount of the inhibitor of the present invention (such as an antibody, a gene editor, an antisense sequence (such as siRNA), or an inhibitor) and a pharmaceutically acceptable carrier or excipient. Such carriers include, but are not limited to, saline, buffers, dextrose, water, glycerol, ethanol, and combinations thereof. The drug formulation should match the mode of administration. The pharmaceutical composition of the present invention can be prepared in the form of injection, for example, prepared by conventional methods with physiological saline or an aqueous solution containing glucose and other adjuvants. Pharmaceutical compositions, such as tablets and capsules, can be prepared by conventional methods. Pharmaceutical compositions such as injections, solutions, tablets and capsules are preferably manufactured under sterile conditions. The active ingredient is administered in a therapeutically effective amount, e.g., about 1 microgram to 10 mg/kg body weight per day.

**The main advantages of the present invention include:**

(1) The present invention has discovered for the first time that reducing the expression or activity of the Ptbpl gene or its encoded protein in astrocytes can induce the differentiation of astrocytes into dopamine neurons, thereby preventing and/or treating neurodegenerative disease (e.g. Parkinson's disease).

(2) The present invention has discovered for the first time that the use of gene editors (including gene editing proteins and gRNAs) to inhibit the expression of ptbpl in astrocytes can transdifferentiate astrocytes into dopamine neurons, which provides a potential approach for Parkinson's treatment.

(3) The present invention has discovered for the first time that the induction of dopamine neurons alleviates the motor dysfunction in the Parkinson's mouse model.

(4) The present invention has discovered for the first time that the RNA-targeted CRISPR system CasRx can avoid the risk of permanent DNA changes caused by traditional CRISPR-Cas9 editing. Therefore, CasRx-mediated RNA editing provides an effective means for the treatment of various diseases.

(5) The present invention directly converts MGs into functional RGCs by inhibiting the expression of Ptbpl in the retina.

(6) Regenerated RGCs can be integrated into the visual pathway and improve visual function in a mouse model of RGC injury.

(7) The present invention uses the RNA-targeted CRISPR system CasRx to knock down *Ptbpl,* avoids the occurrence of substantial off-target effects induced by shRNA and the risk of permanently altering the gene, and provides an excellent tool capable of treating various diseases.

The present invention will be further described below in conjunction with specific embodiments. It should be understood that these examples are only used to illustrate the present invention and not to limit the scope of the present invention. The experimental method of unreceipted specific conditions in the following examples, usually according to conventional conditions, such as people such as Sambrook, molecular cloning: conditions described in laboratory manual (New York: Cold Spring Harbor Laboratory Press, 1989), or according to manufacture conditions recommended by the manufacturer. Percentages and parts are weight percentages and parts unless otherwise specified.

Unless otherwise specified, the materials and reagents used in the examples of the present invention are all commercially available products.

**General method**

**Animal Ethics:** Animals were used and housed in accordance with the guidelines of the Biomedical Research Ethics Committee of the Institute of Neuroscience, Chinese Academy of Sciences.

**guide RNA sequence**

Guide 1: 5'-tttgtaccgactgctatgtctgggacgat-3' (SEQ ID NO: 1);
Guide 2: 5'-ggctggctgtctccagagggcaggtcaggt-3' (SEQ ID NO: 2);
Guide 3: 5'-gtatagtagttaaccatagtgttggcagcc-3' (SEQ ID NO: 3);
Guide 4: 5'-gctgtcggtcttgagctctttgtggttgga-3' (SEQ ID NO: 4);
Guide 5: 5'-tgtagatgggctgtccacgaagcactggcg-3' (SEQ ID NO: 5);
Guide 6: 5'-gcttggagaagtcgatgcgcagcgtgcagc-3' (SEQ ID NO: 6).

**Transient transfection of astrocytes and qPCR:** Astrocytes were isolated and cultured as previously described [1]. Briefly, astrocytes were seeded in 6-well plates. Transiently transfected with 3 μg of a vector expressing gRNA-CasRx-GFP using Lipofectamine 3000 (Thermo Fisher Scientific) following standard procedures. The control plasmid expressed the non-target guide. 1-2 days after transient transfection, GFP-positive cells were collected by Fluorescence activated Cell Sorting (FACS) and lysed for qPCR analysis: RNA was first extracted using Trizol (Ambion), RNA was then reverse transcribed to cDNA using a reverse transcription kit (HiScript Q RT SuperMix for qPCR, Vazyme, Biotech). Amplification was followed by AceQ qPCR SYBR Green Master Mix (Vazyme, Biotech). Ptbpl qPCR primers were: forward, 5'-AGAGGAGGCTGCCAACACTA-3' (SEQ ID NO.: 13); reverse, 5'-GTCCAGGGTCACTGGGTAGA-3' (SEQ ID NO. 14).

**Stereotactic injection:** AAV8 (Figure 1) and AAV-PhP.eb (Figures 2 and 3) were used in this study. Stereotactic injection (C57BL/6, 1-3 months old) was performed as previously described [2]. The titers of AAV-CasRx-Ptbp1 in Figures 1 and 2, 3 were about 5 × 10e12 (2 μl per injection) and 1.6 × 10e13 (2-3 μl per injection), respectively. AAVs were injected into the striatum (AP+0.8mm, ML±1.6mm and DV-2.8mm).

**Immunofluorescence staining**: Immunofluorescence staining was performed 5-6 weeks after injection (Figure 1) or 3-4 weeks (Figures 2 and 3). Brains were harvested after perfusion of mice and fixed with 4% paraformaldehyde (PFA) overnight and kept in 30% sucrose for at least 12 h. Frozen section after embedding with a section thickness of 35 μm. Brain sections were rinsed thoroughly with 0.1 M phosphate buffer (PB) prior to immunofluorescence staining. Primary antibodies: rabbit polyclonal NeuN antibody (Brain, 1:500, #ABN78, Millipore), rabbit polyclonal Tbr1 antibody (#NG1854874, Millipore), mouse TH antibody (1:300, MAB318, Millipore) and rabbit DAT antibody (1:100, MAB369, Millipore). Secondary antibodies: Donkey anti-mouse (#715-545-150, Jackson ImmunoResearch), donkey anti-rabbit (#711-545-152, Jackson ImmunoResearch) were used in this study. Following antibody incubation, sections were washed and covered with mounting medium (Life Technology).

**Electrophysiological recordings:** Electrophysiological recordings were performed 5-6 weeks after AAV injection, as described previously [3]. Briefly, mice were anesthetized and transcardially perfused and brains were placed in carbon dioxide-filled NMDG artificial cerebrospinal fluid (aCSF) [NMDG aCSF (mM): NMDG 92, potassium chloride 2.5, sodium dihydrogen phosphate 1.25, Sodium Bicarbonate 30, HEPES 20, Glucose 25, Thiourea 2, Sodium Ascorbate 5) at room temperature, Sodium Pyruvate 3, Calcium Chloride 0.5, Magnesium Sulfate 10]. Brains were extracted after perfusion and placed in ice-cold NMDG aCSF solution for 30 s. Brains were trimmed and sliced at a thickness of 250-350 μm at a speed of 0.04-0.05 mm/s. Brain slices were transferred into dishes filled with carbon dioxide NMDG aCSF and kept at 32-34 ° C for ≤ 12 min. Transfer the slices to a new dish of $CO_2$-filled HEPES aCSF at room temperature [HEPES containing aCSF (mM): NaCl 92, KCl 2.5, Sodium Dihydrogen Phosphate 1.25, Sodium Bicarbonate 30, HEPES 20, Glucose 25, Thiourea 2, Sodium Ascorbate 5, Sodium Pyruvate 3, Calcium Chloride 2, Magnesium Sulfate 2]. After 1 hour, transfer the slices to a recording dish containing recording buffer [recording aCSF (mM): NaCl 119, KCl 2.5, sodium dihydrogen phosphate 1.25, Sodium Bicarbonate 24, Glucose 12.5, Chloride Calcium 2, Magnesium Sulfate 2]. Neuron-like mCherry-positive cells were recorded under a microscope (Olympus BX51WI) and data were acquired using Clampex 10.

**6-OHDA PD mouse model**

The research process was based on previous research[4]. Injecting adult C57BL/6 mice (7-10 weeks) intraperitoneally. 25 mg/kg desipramine hydrochloride (D3900, Sigma-Aldrich) was injected half an hour before anesthesia. After anesthesia, mice were injected with 3 μg of 6-OHDA (H116, Sigma-Aldrich) or normal saline into the right medial forebrain bundle according to the following coordinates: anteroposterior position (A/P) = -1.2 mm, mediolateral (M/L) = -1.1 mm, dosral-ventral (D/V) = -5 mm. All mice were injected subcutaneously with 1 ml of 4% glucose-saline solution 1 hour after surgery. Mice consumed the soaked food pellets for the following 3 weeks.

**Apomorphine-induced Rotatory Test**

Mice were intraperitoneally injected with 0.5 mg/kg apomorphine (A4393, Sigma-Aldrich) 10 min before testing. Afterwards, they were each placed in an opaque cylinder (30 cm in diameter) over which the camera was recorded for 20 min. Rotation is defined as full body turning with one hind paw as the center and no switching of head orientation. Count the number of rotations on the side of the injection and on the contralateral side. Data were quantified as the number of contralateral reversals over 20 min.

**cylinder test**

Each mouse was gently placed into a glass beaker (1000 ml) and recorded with a camera in front of it for 10 min. The number of paw touches on the injected and contralateral sides were counted separately, and the data were quantified as the ratio of the number of touches on the same side to the total number of touches.

**Rota-Rod Test**

All mice were trained for 2 days and tested on day 3. On day 1, mice were trained 4 times for 300 s on the rotarod at a fixed speed of 4 laps/min. On days 2 and 3, mice were trained or tested 4 times with an acceleration of 4 to 40 laps/min. The time the mice stayed on the rod before falling off was recorded as the dwell period and the average of the 3 longest dwell periods was used for analysis.

**Statistical analysis:** Error bars were set by s.e.m. and statistical significance was calculated by unpaired two-tailed t-test or one-way ANOVA ($p < 0.05$). All experiments were randomized and no statistical methods were used to predetermine sample size, but our sample size was based on reports in the previous literature[5]. It is assumed that the data distribution is normal but not formally tested. Data collection and analysis were not performed under blind experimental conditions.

**Transient transfection, qPCR and RNA-seq of N2a cells**

N2a cells were seeded in 6-well plates. Lipofectamine 3000 (Thermo Fisher Scientific) was used according to standard procedures and cells were transfected with 7 μg of the vector expressing gRNA-CasRx-GFP. Control plasmids did not express gRNA. Two days after transfection, approximately 50,000 GFP-positive cells were collected per sample by fluorescence-activated cell sorting (FACS) and lysed for qPCR analysis. At the same time retinas were isolated to determine AAV expression. RNA was extracted using Trizol (Ambion) and converted to cDNA using a reverse transcription kit (HiScript Q RT SuperMix for qPCR, Vazyme, Biotech). The amplification process was followed using AceQ qPCR SYBR Green Master Mix (Vazyme, Biotech).

*Ptbpl* qPCR primers are: forward primer, 5'-AGAGGAGGCTGCCAACACTA-3' (SEQ ID NO: 7);
reverse primer, 5'-GTCCAGGGTCACTGGGTAGA-3' (SEQ ID NO: 8).
CasRx qPCR primer: forward primer, 5'-CCCTGGTGTCCGGCTCTAA-3' (SEQ ID NO: 9);
reverse primer, 5'-GGACTCGCCGAAGTACCTCT-3' (SEQ ID NO: 10).

For RNA-seq, N2a cells were cultured in 15-cm dishes and transiently transfected with 70 μg of plasmid. ~500,000 GFP-positive (top 20% GFP) N2a cells were collected by FACS, RNA was extracted, converted to cDNA, and then used for whole transcriptome RNA-seq.

## Intravitreal and subretinal injections

NMDA and AAV (AAV-PHP.eB) were introduced by intravitreal and subretinal injection, respectively, as previously described. For subretinal injection, high titer (>1 $\times$ $10^{13}$) AAV was injected into the eye with a Hamilton syringe (32G needle) under an Olympus microscope (Olympus, Tokyo, Japan). To determine reprogramming in intact retina, a total of 1 $\mu$l of GFAP-GFP-Cre (0.2 $\mu$l) and GFAP-CasRx-Ptbp 1 (0.8 $\mu$l), or GFAP-Cre-GFP (0.2 $\mu$l) and GFAP-CasRx (0.8 $\mu$l) via subretinal injection (Ai9 and C57BL/6 mice, 5 weeks old) were delivered to the retina. To determine reprogramming in damaged retinas, NMDA was dissolved in PBS to a concentration of 200 mM, and then 1.5 $\mu$l of NMDA solution was injected into the eyes of 4-8 week old Ai9 mice or 5-6 week old C57BL/6 mice by intravitreal injection (for VEP and B&W scene preference test). 2-3 weeks after NMDA injection, GFAP-GFP-Cre was co-delivered to the retina by subretinal injection together with GFAP-CasRx-Ptbpl or GFAP-CasRx. To assess functional rescue of damaged retinas (VEP and light-dark box shuttling experiments), 5-6 week old mice (C57BL/6) were injected with NMDA to induce retinal damage, and a mixture of GFAP-mCherry (0.2 $\mu$l) and GFAP-CasRx-Ptbpl (0.8 $\mu$l) or GFAP-CasRx (0.8 $\mu$l) was delivered 2-3 weeks after injection.

## Immunofluorescence staining

1 month after AAV injection, eyes, optic nerves and brains were removed, fixed with 4% paraformaldehyde (PFA) for 2 hours (eyes and optic nerves) or 24 hours (brain), and then preserved in 30% sucrose solution for 2 (eyes and optic nerve) or 24 (brain) hours. After embedding and freezing, eyes and brains were sectioned at 30 $\mu$m thickness. Primary antibodies for immunofluorescence staining: mouse anti-Brn3a (1:100, MAB1585, Millipore), rabbit anti-RBPMS (1:500, 15187-1-AP, Proteintech), rabbit anti-Sox9 (1:500, AB5535, Millipore), rabbit anti-Pax6 (1:500, 901301, Biolegent), rabbit anti-Proxl (1:500, AB5475, Millipore), and secondary antibody: Cy™5 AffiniPure Donkey mouse anti-IgG (H + L) (H + L) (1:500,715-175-150, Jackson ImmunoResearch), Cy™ 5 AffiniPure Donkey Rabbit Anti-IgG (H+L) (1:500, 711-175-152, Jackson ImmunoResearch). After applying the antibody, the slides were washed and mounted. Imaging was performed using an Olympus FV3000 microscope.

## Electrophysiology

Mice were dark-adapted overnight before euthanasia. Under the infrared microscope at room temperature, retina anatomy was performed in oxygenated (95% O2 / 5% CO2) artificial cerebrospinal fluid (ACSF) containing 126 mM NaCl, 2.5 mM KCl, 1.25 mM NaH2PO4, 2 mM CaCl2, 2 mM NaHCO3, and 10 mM glucose. On the stage of an Upright Microscope, place the RGCs of the retina facing the cell recording slot. Identify tdTomato-positive cells in the ganglion cell layer using two-photon ($\lambda$ = 1030 nm) microscopy and they were cell-attached recordings under infrared light. A pipette (4-7 M$\Omega$) for recording was supplemented with ASCF and 0.25 mM Alexa488 for the hydration. Recordings were performed using a Multiclamp 700A amplifier and pClamp10 software suite (Molecular Devices). The signal was low pass filtered at 1 kHz and digitized at 10 kHz. Full-field photostimulation was delivered with white LED light. After recording, current pulses were injected to populate the cells to visualize their morphology.

## visual evoked potential

Mice were injected intraperitoneally with a mixture of fentanyl (0.05 mg/kg), midazolam (5 mg/kg) and medetomidine (0.5 mg/kg). The mice were head-fixed in a brain stereotaxic apparatus and their body temperature was maintained at 37°C by a heating blanket. A craniotomy (approximately 1 mm in diameter) was performed over both sides of the primary visual cortex (VI) (AP -3.6 to -3.9 mm, ML 2.2 mm), and the endocranium was removed. Visual stimulation were delivered by a 17-inch liquid crystal display (Dell P170S, maximum brightness of 69 cd/m2), which was 8 cm from the eye on the recording end, and the side of the eye on the same side of the recording end is shielded from visual stimulation. We performed 100 2-second repetitive flash stimuli (full field, 100% contrast) with interval of 2 seconds. Recordings were performed at VI (AP -3.6 to -3.9 mm, ML 2.2 mm) using a multi-site silicon probe (A1$\times$16-5mm-50-177, NeuroNexus Technologies), the cortical depth reached by the electrode tip for each recording was approximately 900 $\mu$m. Both the reference line and the ground lead were placed within a small craniotomy at least 3 mm from the recording site. Neural responses were amplified and filtered using a Cerebus 32-channel system (Blackrock microsystems). The local field potential (LFP) signal was sampled at 2 kHz or 10 kHz using a broadband front-end filter (0.3 to 500 Hz). LFP responses to full-screen flash stimuli were used for current source density (CSD) analysis to determine the location of cortical layer 4[3]. To generate the CSD distribution profile, we calculated the second-order spacial derivatives of the LFP with the following equation:

$$\frac{\partial^2 \varnothing}{\partial z^2} \approx \frac{\varnothing(z-n\Delta z)+\varnothing(z+n\Delta z)-2\varnothing(z)}{(n\Delta z)^2},$$

wherein $\varnothing$ is LFP, z is the coordinates of the recording ends, $\Delta z$ is the distance between adjacent recording ends, and $n\Delta z$ is the differentiation grid (n = 2). Layer 4 (the granular layer) is defined as those recording locations at the initial current receiver. We used the layer 4 channel showing the largest mean amplitude to analyze visually evoked responses in each mouse.

**Black and White Box Preference Test**

The equipment used for the light-dark box shuttle experiment comprised a box with a door, which was divided into a small (one-third) dark box section and a large (two-thirds) illuminated section (550 lumens). Mice were free to move between the two compartments for 10 minutes. The time the mice spent in each compartment was recorded by a camera and then analyzed using the Ethovision XT. After each trial, washing the compartment with 70% ethanol to avoid olfactory cues.

**Statistical Analysis**

All values are shown as mean $\pm$ s.e.m.. Unpaired t-test was used to determine statistical significance ($p < 0.05$). All experiments were randomized and no statistical methods were used to estimate sample size, but our sample sizes were similar to those reported in previous publications. The data distribution is assumed to be normal but not formally tested.

**Example 1 Using CasRx to specifically knock down Ptbpl in vitro**

To determine the efficiency of CasRx-mediated knockdown of Ptbpl, six gRNAs targeting Ptbp1 were first designed and their inhibitory efficiencies were compared in cultured N2a cells and astrocytes (Figure 1A and 1B). It is found that co-transfection of two gRNAs targeting regions IV and VII of Ptbpl exons (combination of 5 and 6), a reduction of 87% $\pm$ 0.4% and 76% $\pm$ 4% can be achieved in N2a cells and astrocytes, respectively. decreased (Figures 1C and 1D). Data from RNA whole transcriptome sequencing further shows that this knockdown is very specific (Figures IE, IF and 2A).

**Example 2 Conversion of Müller glial cell to retinal ganglion cell in the mature retina**

Previous studies have found that knockdown of Ptbpl by shRNA can convert cultured mouse fibroblasts and N2a cells into functional neurons, and we have next investigated whether Ptbpl knockdown converts Müller glial cell into retinal ganglion cell in vivo in mature retinas. To specifically and permanently label retinal Müller glial cell, we injected AAV-GFAP-GFP-Cre into eyes of Ai9 mice (Rosa-CAG-LSL-tdTomato-WPRE) to specifically induce tdTomato expression in Müller glial cell (Figures 2B and 2C). We have also constructed AAV-GFAP-CasRx-Ptbpl (gRNA 5+6) driven by the Müller glial cell-specific promoter GFAP, hoping to specifically knock down Ptbpl in Müller glial cells and we have also constructed a control virus AAV-GFAP-CasRx that does not target Ptbpl (Figures 3A and 2D). We first co-injected AAV-GFAP-CasRx-Ptbpl and AAV-GFAP-Cre-GFP subretinal in Ai9 mice aged approximately 5 weeks, and after 1 month, we have found that many tdTomato-positive cells are co-labeled with the retinal ganglion cells-specific antibodies Brn3a or Rbpms in the retinal ganglion cell layer (GCL), but we found no such cells in retinas injected with control AAV vectors (Figure 3B-3E). These results demonstrate that knockdown of Ptbpl in mature retina converts Müller glial cell into retinal ganglion cell. Notably, since the converted retinal ganglion cells are no longer Müller glial cells, the expression of GFP driven by GFAP is also reduced in the induced retinal ganglion cells (Fig. 2E). We have also demonstrated this existence by other methods (Fig. 4). In addition to retinal ganglion cells, we have also found amacrine neurons (Fig. 5).

**Example 3 Conversion of MG to RGC in a mouse model of retinal injury**

To explore whether MG-induced RGCs can complement lost RGCs in a mouse model of retinal injury, we injected NMDA (200 mM) intravitreally into Ai9 mice (NMDA, 200 mM) aged 4 to 8 weeks, resulting in the death of the vast majority of RGCs and a reduction in the thickness of the inner plexiform layer (IPL). Two to three weeks after NMDA injection, eyes were injected with AAV-GFAP-CasRx-Ptbpl plus AAV-GFAP-GFP-Cre or control AAV virus (Figures 6A and 6B). One month after AAV injection, we have found that the number of Brn3a or Rbpms-positive cells in the retinal ganglion cell layer is significantly increased in AAV-GFAP-CasRx-Ptbp1-injected retinas. Interestingly, most of these cells are tdTomato positive. Whereas in the retinal ganglion cell layer injected with control AAV we have found no increase (Fig. 6C-6F). To determine whether MG-induced RGCs are integrated into retinal circuits and have the capacity to receive visual information, we performed extracellular recordings of MG-induced RGCs under two-photon microscopy to monitor light stimulation-evoked responses (Fig. 6G). We have found that, out of the 8 cells examined, 6 cells have shown photostimulation-induced action potentials (Fig. 6H). Among these cells, five cells are ON cells and one cell is OFF (Fig. 6H). These results suggest that functional RGCs can convert MGs by reducing the expression of Ptbpl in Müller glial cell in damaged retinas.

**Example 4 Induced retinal ganglion cells partially restore visual function**

In the visual system, visual information is projected through the RGCs to the dorsal lateral geniculate nucleus and superior colliculus of the brain (Fig. 8A). In the permanently damaged retina, we have observed a large number of tdTomato-positive axons in the retina and optic nerve by treatment, but not in the control group (Fig. 8B and 8C). Notably, we have also found a large number of tdTomato-positive axons in the dorsal geniculate nucleus and superior colliculus, and the distribution on the contralateral side of the brain is much more abundant than on the ipsilateral side of the brain (Figures 8D and 8E), which is consistent with the correct projection of RGCs in normal mouse granules. To investigate whether impaired visual function can be improved by induced RGCs. We recorded visual evoked potentials (VEPs) in the primary visual cortex (VI) of anesthetized mice 1 month after AAV virus injection (Fig. 8F). In retinal-injured mice treated with AAV-GFPA-CasRx-Ptbp1 and AAV-GFAP-mCherry, we have found very obvious evoked potentials with amplitudes similar to those found in uninjured mice. In the control group, we have observed only very weak responses (Figures 8G and 8H). These results support that induced optic nerve cells partially restore the transmission of visual information to the primary visual cortex, presumably by establishing synaptic connections with existing functional dorsal lateral geniculate nucleus neurons in the brain. From a behavioral perspective, we have explored whether CasRx-mediated conversion of MGs to RGCs can restore visual behaviors lost due to retinal damage (Fig. 8I). First we injected NMDA in both eyes of mice and then AAV virus. In the light/dark preference test, untreated mice have shown no apparent tendency to stay in the dark room, consistent with vision loss due to retinal damage. In contrast, the duration of time spent in the dark room in the treated mice is significantly increased, even approaching the level of healthy control mice (Fig. 8J), suggesting that induced optic nerve cells have improved visual behavior in visually impaired mice.

**Example 5 Projection process of retinal ganglion cells to the brain**

To explore when to start seeing Müller glial cell-induced retinal ganglion cells, we set five different time points (1 week, 1.5 weeks, 2 weeks, 3 weeks, and 1 month). We have found that tdTomato+Rbpms+ and tdTomato+Brn3a+ cells are first identified in the retina at 1.5 weeks after AAV injection, and the numbers of these cells are gradually increased over time (Figures 7A and 7B). Our results also indicate that at 1.5 weeks after AAV injection, there is an intermediate phase in which induced RGCs migrate from the INL to the optic nerve cell layer (Fig. 7C). These findings are also confirmed in NMDA-induced permanently damaged retinas (Fig. 7D). As to when to start projecting to the correct brain region, we have first found that tdTomato-positive axons in the optic nerve are increased significantly over time (Fig. 9A). In the brain, we do not find any projections in the brain at 1 week, while at 1.5 weeks of AAV injection we begin to find tdTomato-positive axons in the contralateral dorsal geniculate nucleus, this projection does not occur on the same side (Figure 9B). At 2 weeks of AAV injection, we begin to find induced retinal ganglion cell projections in the contralateral inferior colliculus (Fig. 5C), and at 3 weeks of AAV injection, we have found projections of induced retinal ganglion cells in the contralateral and ipsilateral dorsal geniculate nucleus and superior colliculus, and the density of this projection is further increased at 1 month (Fig. 9B-9D). Similar results are observed in NMDA-injected damaged retinas (Figures 10A-10C).

**Example 6 Conversion of astrocytes into neurons**

To further explore the potential use of CasRx-induced glial-neuron conversion in other systems, we have further investigated whether knockdown of Ptbpl expression in striatal astrocytes can locally convert astrocytes into dopamine neurons, a cell type closely associated with the development of Parkinson's disease (PD). We first injected AAV-GFAP-mCherry into the striatum of wild-type mice to label astrocytes, and co-injected AAV-GFAP-CasRx-Ptbpl (gRNA: 5+6) to down-regulate the expression of Ptbpl in astrocytes (Figures 11A and 11B). As a control, we used AAV-GFAP-CasRx without Ptbpl gRNA. We have found that both mCherry and CasRx are abundantly expressed in astrocytes and shows high co-infection efficiency in the striatum, wherein 99%±1% of mCherry+ cells express CasRx (82%±2% of GFAP+ cells express mCherry and 95%±1% of mCherry+ cells express GFAP) (Figures 11C and 11D). We have found down-regulation of Ptbpl expression in astrocytes 1 week after AAV injection into the striatum (Figures 11E and 11F). One month after AAV virus injection, we have found that a high proportion of mCherry+ cells express the mature neuronal marker NeuN (48% ± 10%, SEM, n = 6 mice), but no such expression is found in the control group injected with AAV-GFAP-mCherry and AAV-GFAP-CasRx (0.97%±0.45%, SEM, n=6) (Figures 11G and 11H). To further explore the specific types of these converted neurons, we performed immunostaining with neuron-specific antibodies. We have found that approximately 50% of the induced neurons express glutaminase (Figures 11I and 11J), a marker for excitatory glutamatergic neurons, and we have also found that a small fraction of induced neurons express one interneuron subtype marker, SST, and no cells express another interneuron subtype marker, PV (Figures 11K and 11L). We have also used co-staining of the dopamine neuron marker tyrosine hydroxylase (TH) with NeuN to determine whether there are dopamine neurons in the converted neurons, and we have found that a fraction (7.5% ± 3%, SEM) of mCherry+ cells express TH (Figures 11M-11O). Furthermore, we have found that mCherry expression in converted neurons has persisted for at least 1 month after infection (Fig. 11G), a phenomenon consistent with previous reports.

**Example 7 Induction of dopamine neurons**

To explore the potential clinical applications of CasRx-mediated neuronal conversion in the striatum, we induced a mouse model of Parkinson by unilateral infusion of 6-hydroxydopamine (6-OHDA) into the substantia nigra. This infusion caused the death of dopamine neurons in the substantia nigra and the degeneration of dopaminergic projections in the ipsilateral striatum (Figures 13A-13C). Three weeks after injection of 6-OHDA, AAV-GFAP-CasRx-Ptbpl (or AAV-GFAP-CasRx as a control) was co-injected with AAV-GFAP-mCherry into the ipsilateral striatum. The conversion of striatum cells are also analyzed at different time points (Figures 12A and 12B). One month after virus injection, we have found that a high proportion of mCherry cells expressing TH, a marker for dopamine neurons, has appeared in striatum injected with AAV-GFAP-CasRx-Ptbpl and AAV-GFAP-mCherry in mice with 6-OHDA-induced injury, and this percentage increases at 3 months of AAV injection (19% ± 0.4%, SEM, n=5 mice at one month; 32% ± 7%, SEM, n=3 mice mice, at 3 months) (Figures 12C, 12D, 13D and 13E). In the control group, such cells are rarely observed (Figures 12C, 12D and 13D-13F). Additionally, about 80% of TH+ cells in the virus injection area are mCherry+ (Figures 12E and 13G), indicating that these dopamine cells are mainly converted from astrocytes. We have noticed that the percentage of mCherry+ TH+ cells in wild-type (without 6-OHDA injury) mice is lower than that in 6-OHDA-injured mice (Fig. 13H), suggesting that repair mechanisms after injury may promote TH+ neurons induction. We have also found that the induced dopamine neurons express the mature dopamine neuron marker dopamine transporter Slc6a3 (DAT), a protein not present in striatal neurons transiently expressing TH. We have found that a high proportion of mCherry-positive cells expresses DAT (10% ± 3%, SEM, n = 5, 1 month; in AAV-GFAP-CasRx-Ptbp1-injected striatum, 31%±7% in mCherry+DAT+ cells, at 3 months, n=3 mice), while almost no such cells are found in the striatum injected with control virus (Figures 12C, 12F, 13D and 13I). Co-immunostaining of TH and DAT has further revealed that the majority of mCherry+TH+ cells express DAT (Figures 12C, 12G and 13D), indicating that most of the induced dopamine neurons express mature dopaminergic markers. In addition, mCherry+ cells have also expressed two additional midbrain dopamine neuron markers, dopa decarboxylase (DDC) and forkhead box A2 (FOXA2) (Figures 13J-13M), further verifying that the converted neurons are dopaminergic neurons. Previous studies indicated that 6-OHDA injury transiently induces TH expression in some interneurons in the mouse striatum. Here we have assessed this by co-staining interneurons for the markers PV+, SST+ and calretinin+ (CR+) 3 months after AAV injection. We have found that none of these interneuron markers are co-localized with mCherry+TH+ cells, indicating that converted TH+ neurons are not transiently induced TH+ neurons (Fig. 13N). To explore the subtypes of induced dopamine neurons, we co-stained TH with two substantia nigra A9 region-specific dopamine neuron markers, ALDH1A1 and GIRK2, and DAT with the ventral tegmental area (VTA) dopamine neuron-specific marker, Calbindin, respectively. Our results show that almost all induced neurons express ALDH1A1 and GIRK2, but not calbindin (Fig. 12H-12J), implying that the induced dopamine neurons are very similar to those of the substantia nigra. Next, we did whole-cell electrophysiological recordings on brain slices. We have found that the majority of neuron-like mCherry-positive cells (20 out of 22 cells) are able to respond to depolarizing current-induced action potentials in current-clamp mode (Fig. 12K). We have also observed spontaneous postsynaptic currents (Vc = -70 mV) in voltage-clamp mode, indicating that converted neurons are capable of receiving functional synaptic input (Figures 12L and 12M). Furthermore, in 4 out of 10 neurons examined, we have observed delayed voltage rectification induced by hyperpolarization activated current (Ih) (Fig. 12K), which is also one of the markers of midbrain dopamine neurons. Next to determine whether induced dopamine neurons can release dopamine, we performed immunostaining and have found that most induced dopamine cells express vesicular monoamine transport protein 2 (VMAT2) (Fig. 12N), which is a protein that regulates the packaging, storage and release of dopamine. We have also found that many cells in the virus-injected striatum take up a fluorescent dopamine derivative (FFN206), a VMAT2 substrate, that can determine whether the expressed VMAT2 is functional. Under high potassium chloride treatment, we have found a decrease in the fraction of fluorescence, indicating that the converted cells have a dopamine release function (Figures 12O-12Q). Our results demonstrate that CasRx-mediated knockdown of Ptbpl can efficiently induce dopaminergic neurons in the striatum of PD model mice.

**Example 8 Improvement of motor function in Parkinson's disease model mice**

We further examined whether induced dopamine neurons in the striatum could alleviate motor symptoms in a mouse model of 6-OHDA-induced Parkinson's disease (Fig. 14A). We performed both drug- and drug-free-induced assessments of motor function. For drug-induced activity, we first investigated apomorphine-induced contralateral rotational behavior, which is widely used to test behavioral changes resulting from unilateral dopamine neuron loss. We have found that compared with control mice (AAV-GFAP-CasRx and AAV-GFAP-mCherry or saline injection), Ptbpl knockdown mice (injected with AAV-GFAP-CasRx-Ptbpl and AAV-GFAP-mCherry) have significantly reduced apomorphine-induced net rotation numbers (counted as contralateral shear ipsilateral rotation numbers), at levels comparable to uninjured healthy mice (Figures 14B, 13O and 13P). In another test of ipsilateral rotational behavior induced by systemic amphetamine administration, intercellular dopamine concentrations were increased by inhibiting DAT uptake of dopamine in the striatum. Compared with control mice, AAV-GFAP-CasRx-Ptbp1-injected mice have significantly lower net rotation numbers (measured as ipsilateral-to-contralateral rotation numbers) and ipsilateral rotation ratios (measured as ipsilateral/total rotation numbers) (Figures 14C, 14D and 13Q). These results suggest that induced neurons in the striatum can release sufficient dopamine to alleviate drug-induced motor dysfunction in PD model mice. In addition, we examined whether two drug-free motor functions improved separately, namely asymmetry in forelimb use and motor coordination. We have found that compared to control mice, mice injected with AAV-GFAP-CasRx-Ptbpl have a significantly lower percentage of ipsilateral touches on the cylinder and longer duration on the rotating tripod (Figures 14E and 14F). Taken together, these results suggest that astrocyte Ptbpl knockdown-mediated dopamine neuron conversion in the striatum alleviates motor dysfunction in Parkinson's disease model mice.

**Example 9 Detection of off-target Ptbpl knockdown tools in vitro**

In order to explore whether the subsequent Cas13Rx and Cas13e can be used in clinical in vivo treatment, the present invention attempts to use an in vitro system to perform off-target detection of these RNA editing tools in the process of knocking down Ptbpl. First, we constructed a plasmid containing CasRx-sgRNA (this sgRNA combination comes from Cell, DOI: 10.1016/j.cell.2020.03.024) and Cas13e-sgRNA (this sgRNA combination is a combination obtained by new screening, respectively sgRNA1: TGTGGTTGGAGAACTGGATGTAGATGGGCT (SEQ ID NO.15), sgRNA2: GAGCCCATCTGGATCAGTGCCATCTTGCGG (SEQ ID NO.: 16); sgRNA3: AGTCGATGCGCAGCGTGCAGCAGGCGTTGT (SEQ ID NO.: 17)) and a plasmid containing Cas13e-NT, CasRx-NT, U6-shPTB (shPTB is derived from Nature, DOI: 10.1038/s41586-020-2388-4) and U6-shNT are used as controls (Figure 15) and they were transferred into N2a cells by lipofection, respectively. Since these vector plasmids all carry the mcherry reporter gene, 48 hours after transfection, we used flow sorting to select mcherry-positive cells, and approximately 50,000 cells were collected per group and subjected to RNA whole transcriptome sequencing. Analysis of RNA whole transcriptome sequencing results show that the off-target rate of Cas13e-sgRNA is lower than that of CasRx-sgRNA, and the off-target rates of both Cas13e-sgRNA and CasRx-sgRNA are much lower than those of U6-shPTB group. The results show that CRISPR-mediated RNA editing tools, especially Cas13e, are superior to traditional shRNA tools in off-target effects.

**Example 10 Detection of transdifferentiation efficiency after Ptbpl knockdown in vivo**

To further explore the potential use of Cas13e in inducing the differentiation of glial cells into neurons, we examined whether Cas13e converts astrocytes into dopamine neurons after knockdown of Ptbpl in astrocytes in striatum and compared it with two tools, CasRx and shRNA. To this end, the plasmids of Cas13e-sgRNA expressed and driven by GFAP promoter was constructed (the sgRNA combination is a combination obtained by new screening, respectively sgRNA1: TGTGGTTGGAGAACTGGATGTAGATGGGCT (SEQ ID NO. 15), sgRNA2: GAGCCCATCTGGATCAGTGCCATCTTGCGG (SEQ ID NO.: 16); sgRNA3: AGTCGATGCGCAGCGTGCAGCAGGCGTTGT (SEQ ID NO.: 17)) and CasRx-sgRNA (this sgRNA combination is derived from Cell, DOI: 10.1016/j.cell.2020.03.024) and the plasmids containing Cas13e-NT, CasRx-NT, U6-shPTB (shPTB derived from Nature, DOI: 10.1038/s41586-020-2388-4) and U6-shNT are used as controls (Figure 16). These plasmids were subsequently subjected to packaging and purification of adeno-associated virus AAV. We induced a mouse model of Parkinson's disease by unilateral infusion of 6-hydroxydopamine (6-OHDA) into the substantia nigra three weeks before the virus injection experiment, and these AAV viruses were then injected into the striatum of these Parkinson's mice, respectively and AAV-GFAP-mCherry virus was co-injected for labeling of astrocytes (Figure 16). 28 days and 90 days after virus injection, DAT/TH staining, electrophysiological and mouse behavioral detection were performed respectively to verify the transdifferentiation efficiency in vivo from various aspects. We have found that the combination of Cas13e-sgRNA can also effectively knock down Ptbpl in mice, and lead to the differentiation of astrocytes into dopamine neurons, and improve the motor function of Parkinson's disease model mice, it also has potential prospects for application to clinical treatment.

## Discussion

In this work, our study shows that glial cells can be efficiently converted into neurons through CasRx-mediated downregulation of Ptbpl. In an NMDA-induced retinal injury model, Ptbpl knockdown induces the conversion of MGs to RGCs in the injured retina, partially restoring visual responses and vision-dependent behaviors. In a mouse model of 6-OHDA-induced Parkinson's disease, it induces striatal astrocytes into dopamine neurons and alleviates motor dysfunction associated with loss of substantia nigra dopamine neurons. These results demonstrate that downregulating the expression of a single RNA-binding protein, Ptbpl, is sufficient to convert glial cells into lost specific types of neurons in different nervous systems, providing a new approach for future therapeutic applications. Short hairpin RNA (shRNA) technology enables cleavage or repression of desired transcripts, but it has very serious off-target effects. Cas13-mediated knockdown is not only more efficient than RNAi, but also reduces off-target effects to a large extent, with greater potential for its therapeutic applications. Furthermore, CasRx is the smallest of the Cas13 protein family and can be packaged in an AAV with CRISPR arrays (encoding multiple guide RNAs). In the field of gene editing, the RNA-targeting CRISPR system CasRx can avoid the risk of permanent DNA alterations caused by the DNA-targeting CRISPR-Cas9 editing system, making it safer for clinical applications.

References:

1. Mccarthy, K.D. & Devellis, J. Preparation Of Separate Astroglial And Oligodendroglial Cell-Cultures From Rat Cerebral Tissue. Journal Of Cell Biology 85, 890-902 (1980).
2. Zhou, H. et al. Cerebellar modules operate at different frequencies. Elife 3, e02536 (2014).
3. Xu, H.T. et al. Distinct lineage-dependent structural and functional organization of the hippocampus. Cell 157, 1552-1564 (2014).
4. Su, J. et al. Reduction of HIP2 expression causes motor function impairment and increased vulnerability to dopaminergic degeneration in Parkinson's disease models. Cell Death Dis 9, 1020 (2018).
5. Chavez, A. et al. Comparison of Cas9 activators in multiple species. Nat Methods 13, 563-567 (2016).
6. Qian, Hao et al. Reversing a model of Parkinson's disease with in situ converted nigral neurons. Nature 582, 550-556 (2020).
7. Zhou, Haibo et al. Glia-to-Neuron Conversion by CRISPR-CasRx Alleviates Symptoms of Neurological Disease in Mice. Cell 181,590-603.e16 (2020).

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. Use of an inhibitor of Ptbpl gene or RNA or an encoded protein thereof for the preparation of a composition or preparation for the prevention and/or treatment of a nervous system disease related to functional neuronal death.

2. The use of claim 1, wherein the composition or preparation is also used for one or more purposes selected from the group consisting of:

   (a1) inducing the transdifferentiation of astrocytes into dopamine neurons;
   (b1) treating movement disorders in mammalian Parkinson's disease;
   (c1) inducing the conversion or differentiation of glial cells into functional neurons;
   (d1) treating nervous system diseases related to retinal ganglion cell (RGC) degeneration;
   (e1) preventing or treating retinal diseases;
   (f1) preventing and/or treating neurodegenerative diseases;
   (g1) transdifferentiation of Müller glial cells into retinal ganglion cells;
   (h1) treating visual impairment caused by the death of the optic ganglia in mammals.

3. The use of claim 2, wherein the astrocyte is derived from the striatum, substantia nigra, spinal cord, dorsal midbrain or cerebral cortex, preferably, the astrocyte is derived from the striatum.

4. The use of claim 1, wherein the functional neuron is selected from the group consisting of: RGC neuron, dopamine neuron, and a combination thereof.

5. The use of claim 1, wherein the inhibitor is selected from the group consisting of an antibody, small molecule compound, microRNA, siRNA, shRNA, antisense oligonucleotide, aptamer, gene editor, and a combination thereof.

6. A composition comprising:

(a) a gene editing protein, or an expression vector thereof, selected from the group consisting of CasRx, CRISPR/Cas9, Cpfl, Cas9, Cas13a, Cas13b, Cas13c, RNA-targeted gene editing protein, and a combination thereof; and
(b) a gRNA or an expression vector thereof, the gRNA guides the gene editing protein to specifically bind to the DNA or RNA of the Ptbpl gene.

7. A kit, comprising:

(a1) a first container, and a gene editing protein or an expression vector thereof located in the first container, or a drug containing a gene editing protein or an expression vector thereof, the gene editing protein is selected from the group consisting of CasRx, CRISPR/Cas9, Cpfl, Cas9, Cas13a, Cas13b, Cas13c, RNA-targeted gene editing protein, and a combination thereof;
(b1) a second container, and a gRNA or an expression vector thereof located in the second container, or a drug containing a gRNA or an expression vector thereof, the gRNA guides the gene editing protein to specifically bind to the DNA or RNA of the Ptbpl gene.

8. Use of the composition of claim 6 or the kit of claim 7 for the preparation of a medicament for the prevention and/or treatment of a nervous system disease associated with functional neuronal death.

9. A method for promoting the differentiation of a glial cell into a functional neuron, comprising the steps of:
In the presence of the inhibitor of Ptbpl gene or RNA or an encoded protein thereof or the composition of claim 6, culturing the glial cell, thereby promoting the differentiation of the glial cell into the functional neuron.

10. A method for screening a candidate compound for preventing and/or treating a nervous system disease associated with functional neuron death, comprising the steps of:

(a) in the test group, adding a test compound to the cell culture system, and observing the expression level (E1) and/or activity (A1) of Ptbpl in the cell of the test group; in the control group, a test compound that does not target Ptbpl is added to the culture system of the same cell, and the expression (E0) and/or activity (A0) of Ptbpl in the cell of the control group are observed;

wherein, if the expression level (E1) and/or activity (A1) of Ptbpl in the cell in the test group is significantly lower than that in the control group, indicating that the test compound is a candidate compound for preventing and/or treating a nervous system disease associated with functional neuron death with an inhibitory effect on the expression and/or activity of Ptbpl.

11. An AAV vector, comprising:

(a) a coding sequence for a gene editing protein selected from the group consisting of CasRx, CRISPR/Cas9, Cpfl, Cas9, Cas13a, Cas13b, Cas13c, an RNA-targeted gene editing protein, and a combination thereof; and
(b) a gRNA that guides the gene editing protein to specifically bind to the DNA or RNA of the Ptbpl gene.

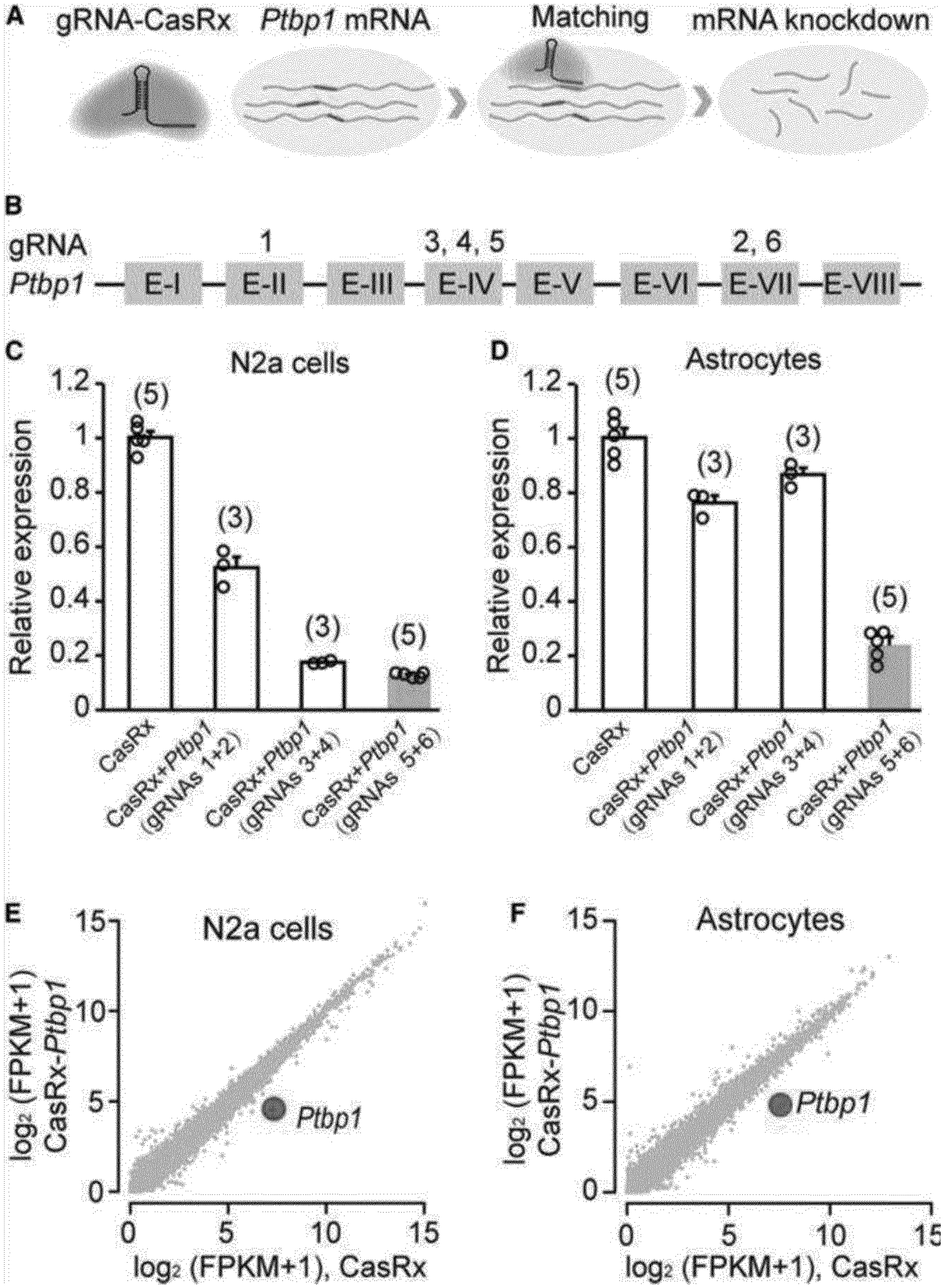
A
gRNA-CasRx
Ptbp1 mRNA
Matching
mRNA knockdown
B
gRNA
1
3, 4, 5
2, 6
Ptbp1
E-I
E-II
E-III
E-IV
E-V
E-VI
E-VII
E-VIII
C
N2a cells
Relative expression
1.2
1
0.8
0.6
0.4
0.2
0
(5)
(3)
(3)
(5)
CasRx
CasRx+Ptbp1 (gRNAs 1+2)
CasRx+Ptbp1 (gRNAs 3+4)
CasRx+Ptbp1 (gRNAs 5+6)
D
Astrocytes
Relative expression
(5)
(3)
(3)
(5)
CasRx
CasRx+Ptbp1 (gRNAs 1+2)
CasRx+Ptbp1 (gRNAs 3+4)
CasRx+Ptbp1 (gRNAs 5+6)
E
N2a cells
log2 (FPKM+1) CasRx-Ptbp1
Ptbp1
log2 (FPKM+1), CasRx
0
5
10
15
F
Astrocytes
log2 (FPKM+1) CasRx-Ptbp1
Ptbp1
log2 (FPKM+1), CasRx

Figure 1

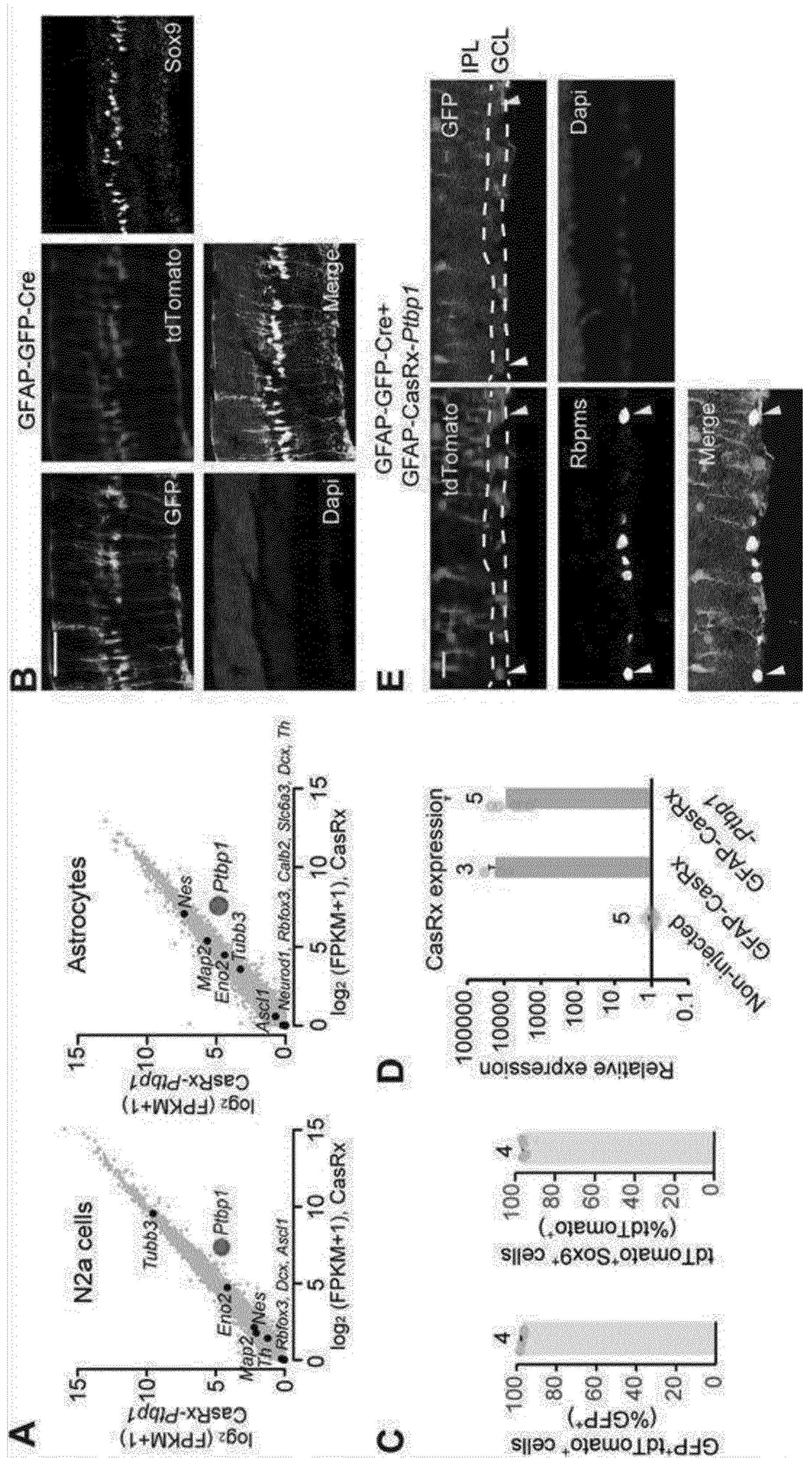
A
N2a cells
Astrocytes
log2 (FPKM+1) CasRx-Ptbp1
log2 (FPKM+1), CasRx
Tubb3
Ptbp1
Eno2
Map2
Nes
Th
Rbfox3, Dcx, Ascl1
Tubb3
Ascl1
Neurod1, Rbfox3, Calb2, Slc6a3, Dcx, Th
B
GFAP-GFP-Cre
GFP
tdTomato
Sox9
Dapi
Merge
C
GFP+tdTomato+ cells (%GFP+)
tdTomato+Sox9+ cells (%tdTomato+)
D
CasRx expression
Relative expression
Non-injected
GFAP-CasRx
GFAP-CasRx-Ptbp1
E
GFAP-GFP-Cre+
GFAP-CasRx-Ptbp1
tdTomato
GFP
IPL
GCL
Rbpms
Dapi
Merge

Figure 2

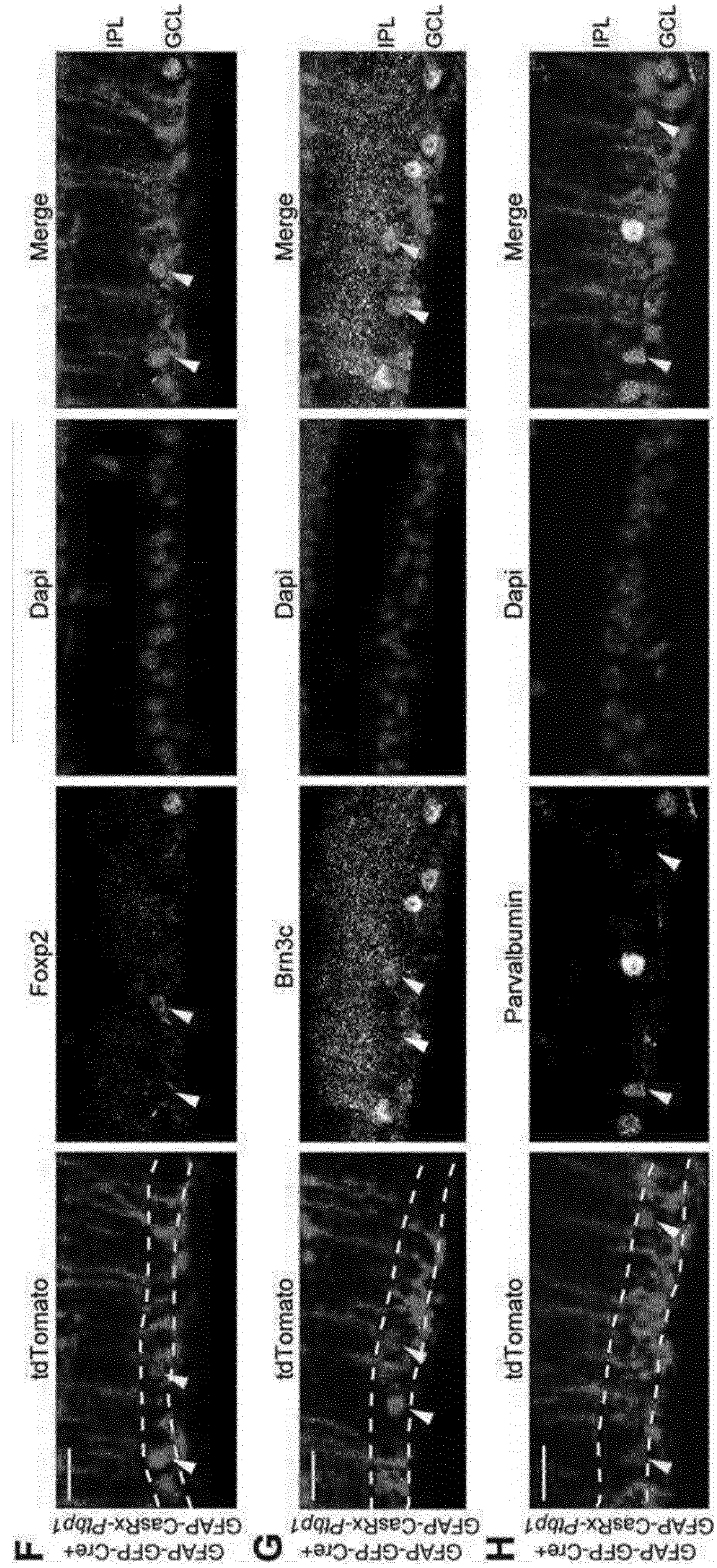


Figure 2 (continued)

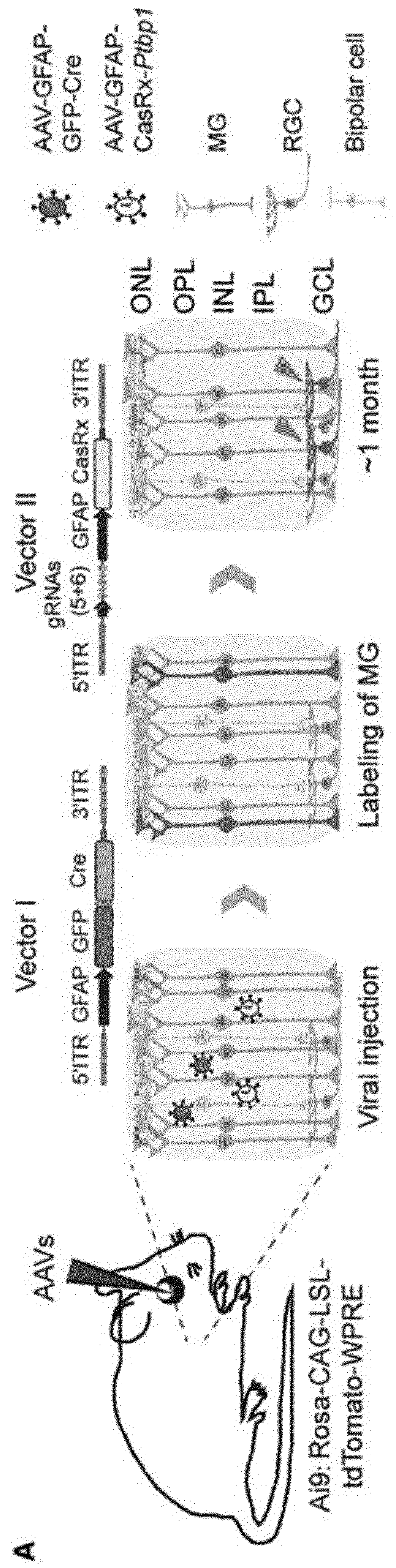
A
AAVs
Ai9: Rosa-CAG-LSL-tdTomato-WPRE
Vector I
5'ITR GFAP GFP Cre 3'ITR
Viral injection
Labeling of MG
Vector II
5'ITR gRNAs (5+6) GFAP CasRx 3'ITR
~1 month
ONL
OPL
INL
IPL
GCL
AAV-GFAP-GFP-Cre
AAV-GFAP-CasRx-Ptbp1
MG
RGC
Bipolar cell

Figure 3

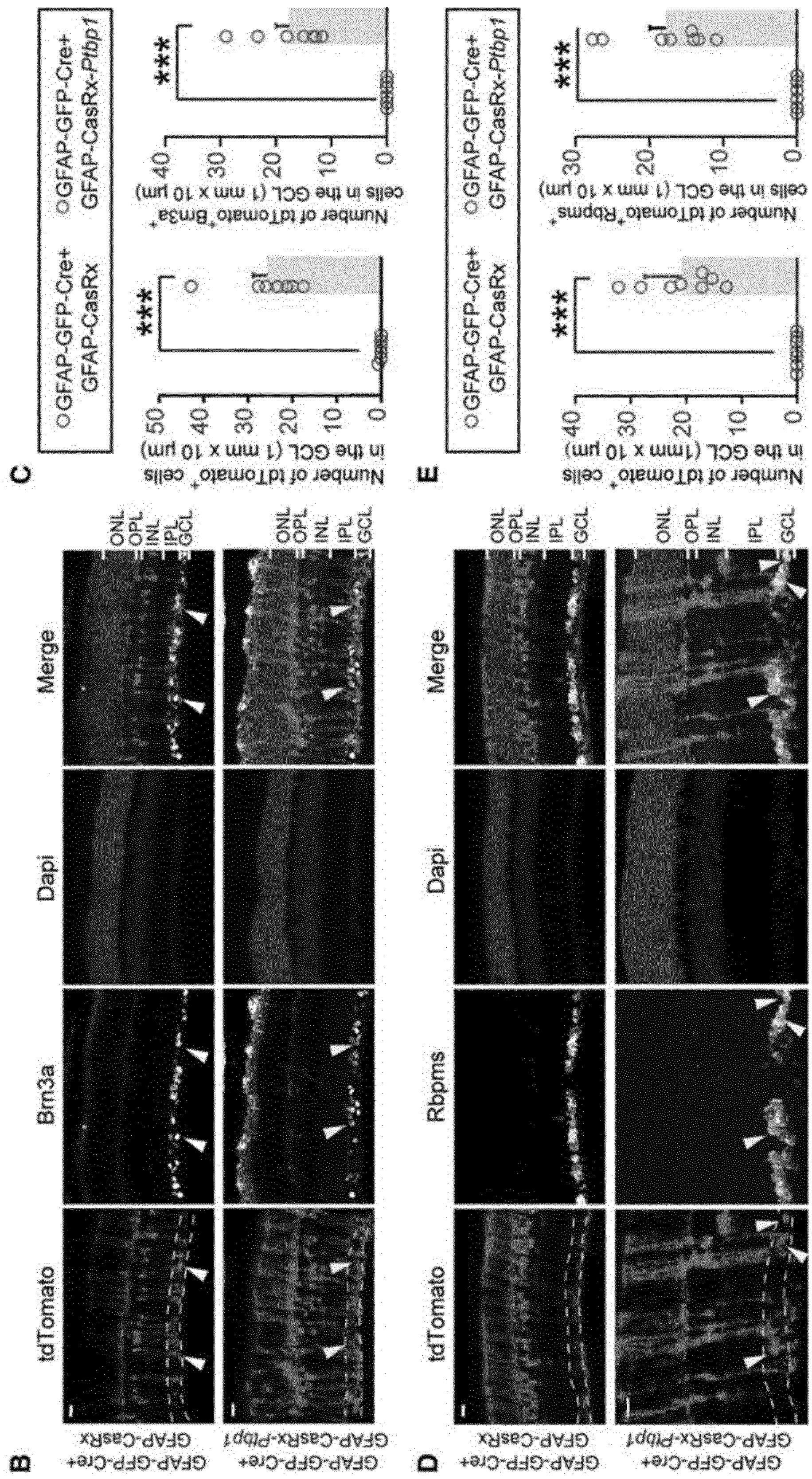


Figure 3 (continued)

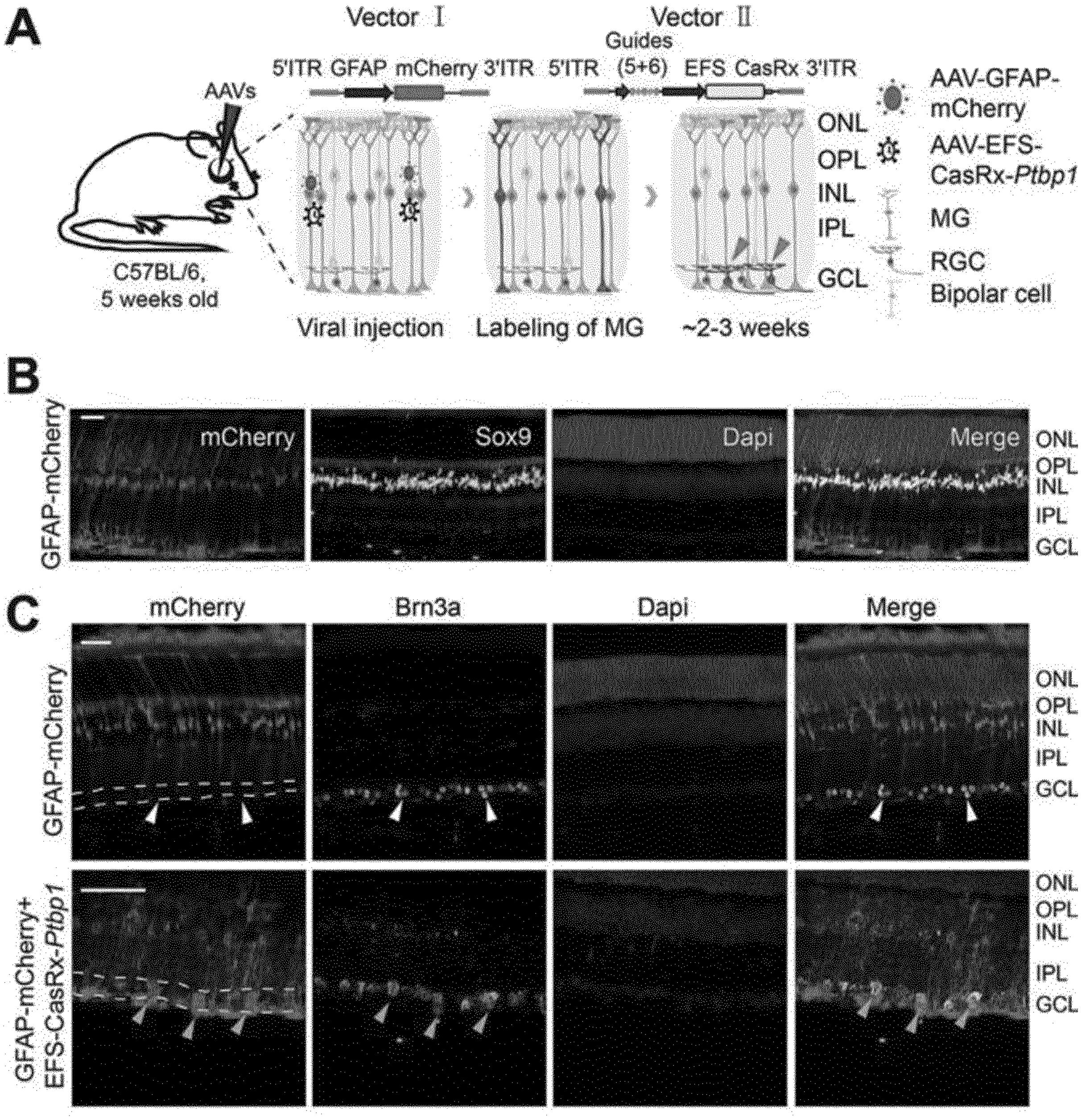
A
Vector Ⅰ
Vector Ⅱ
Guides
5'ITR GFAP mCherry 3'ITR 5'ITR (5+6) EFS CasRx 3'ITR
AAVs
C57BL/6,
5 weeks old
Viral injection
Labeling of MG
~2-3 weeks
ONL
OPL
INL
IPL
GCL
AAV-GFAP-mCherry
AAV-EFS-CasRx-Ptbp1
MG
RGC
Bipolar cell
B
GFAP-mCherry
mCherry
Sox9
Dapi
Merge
C
Brn3a
GFAP-mCherry+
EFS-CasRx-Ptbp1

Figure 4

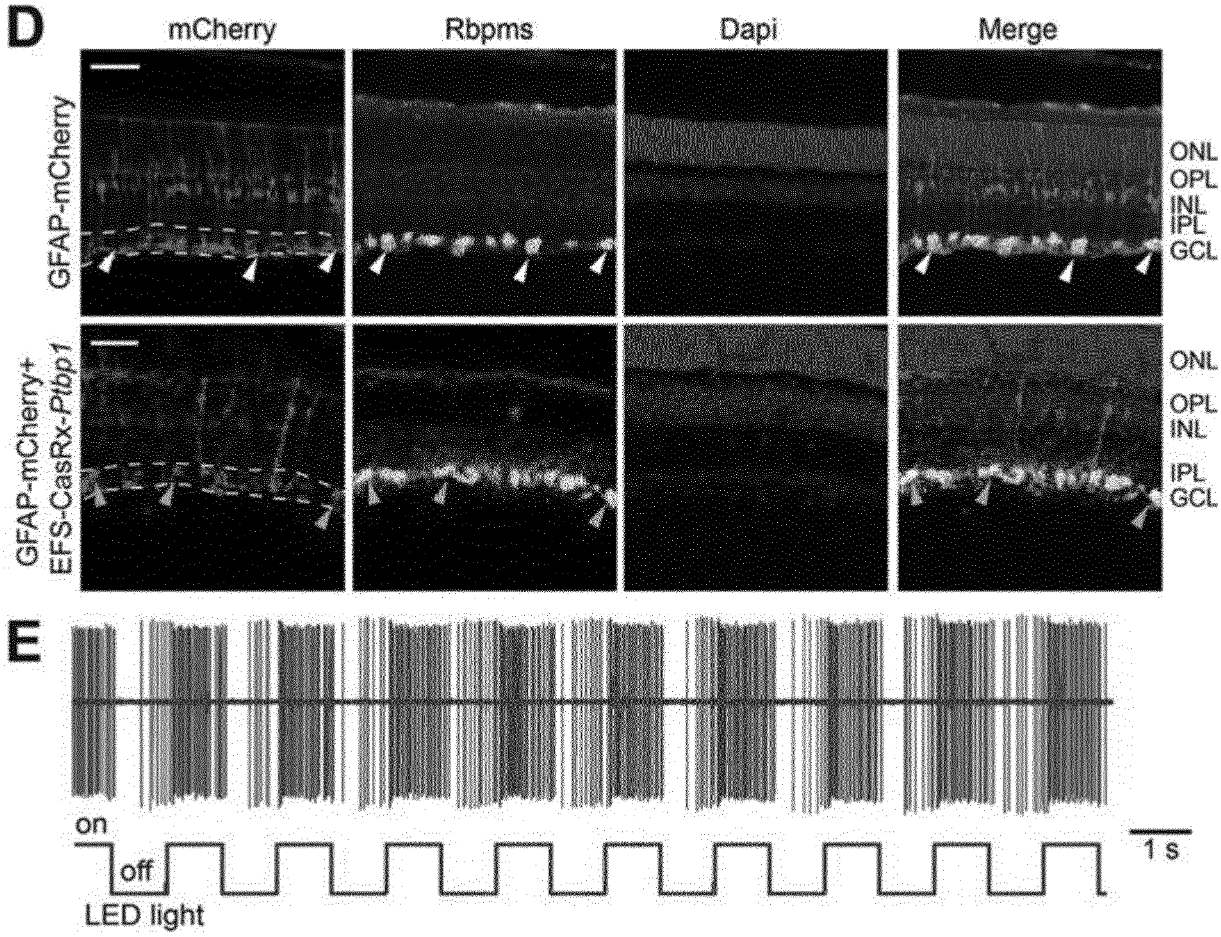


Figure 4 (continued)

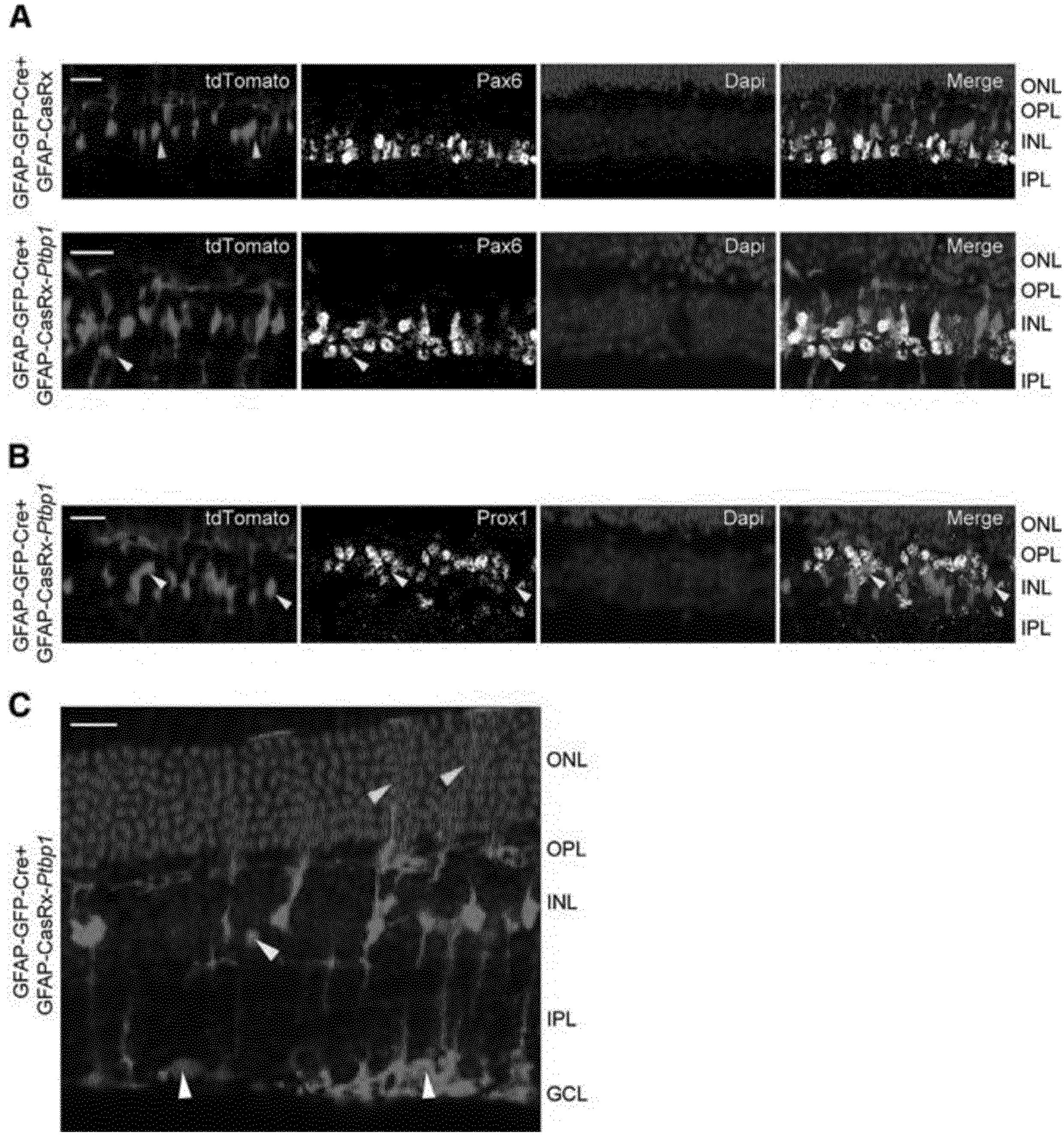
A
GFAP-GFP-Cre+
GFAP-CasRx
tdTomato
Pax6
Dapi
Merge
ONL
OPL
INL
IPL
GFAP-GFP-Cre+
GFAP-CasRx-Ptbp1
B
Prox1
C
GCL

Figure 5

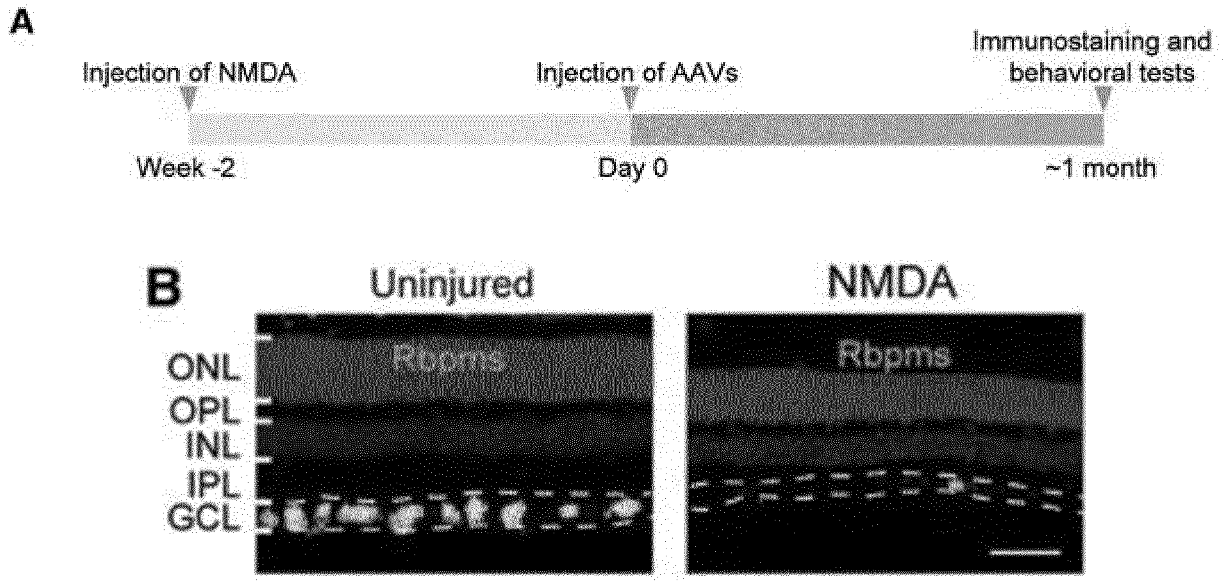
A
Injection of NMDA
Injection of AAVs
Immunostaining and behavioral tests
Week -2
Day 0
~1 month
B
Uninjured
NMDA
Rbpms
Rbpms
ONL
OPL
INL
IPL
GCL

Figure 6

C

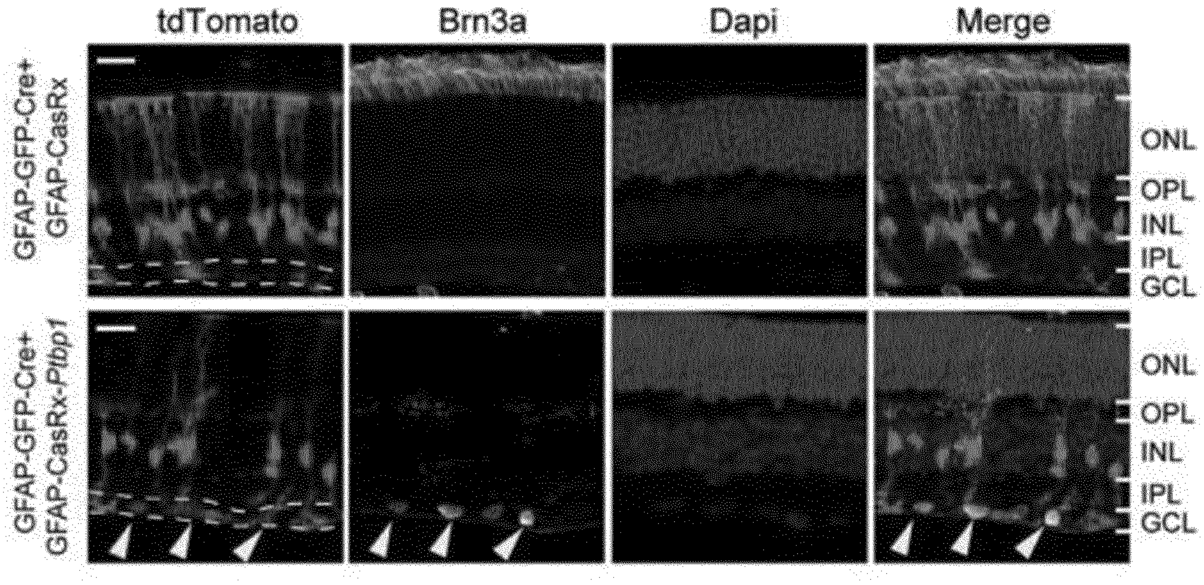


D

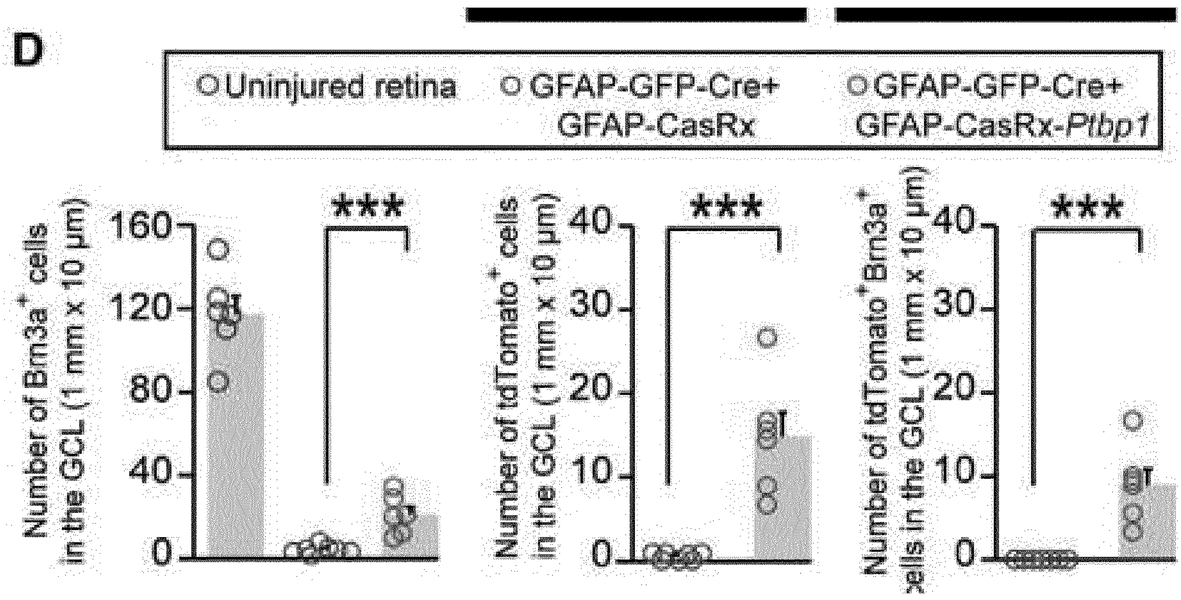


Figure 6 (continued)

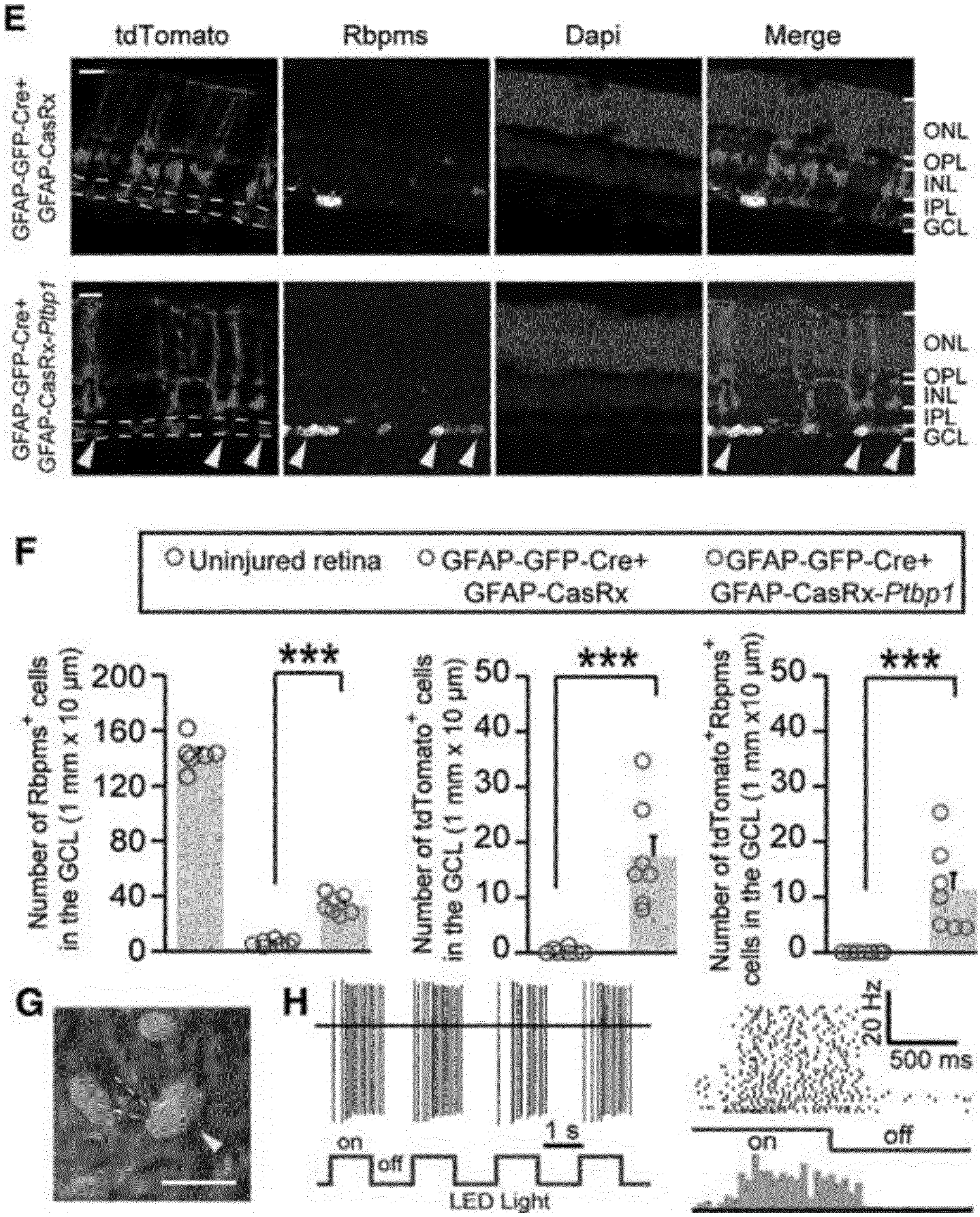


Figure 6 (continued)

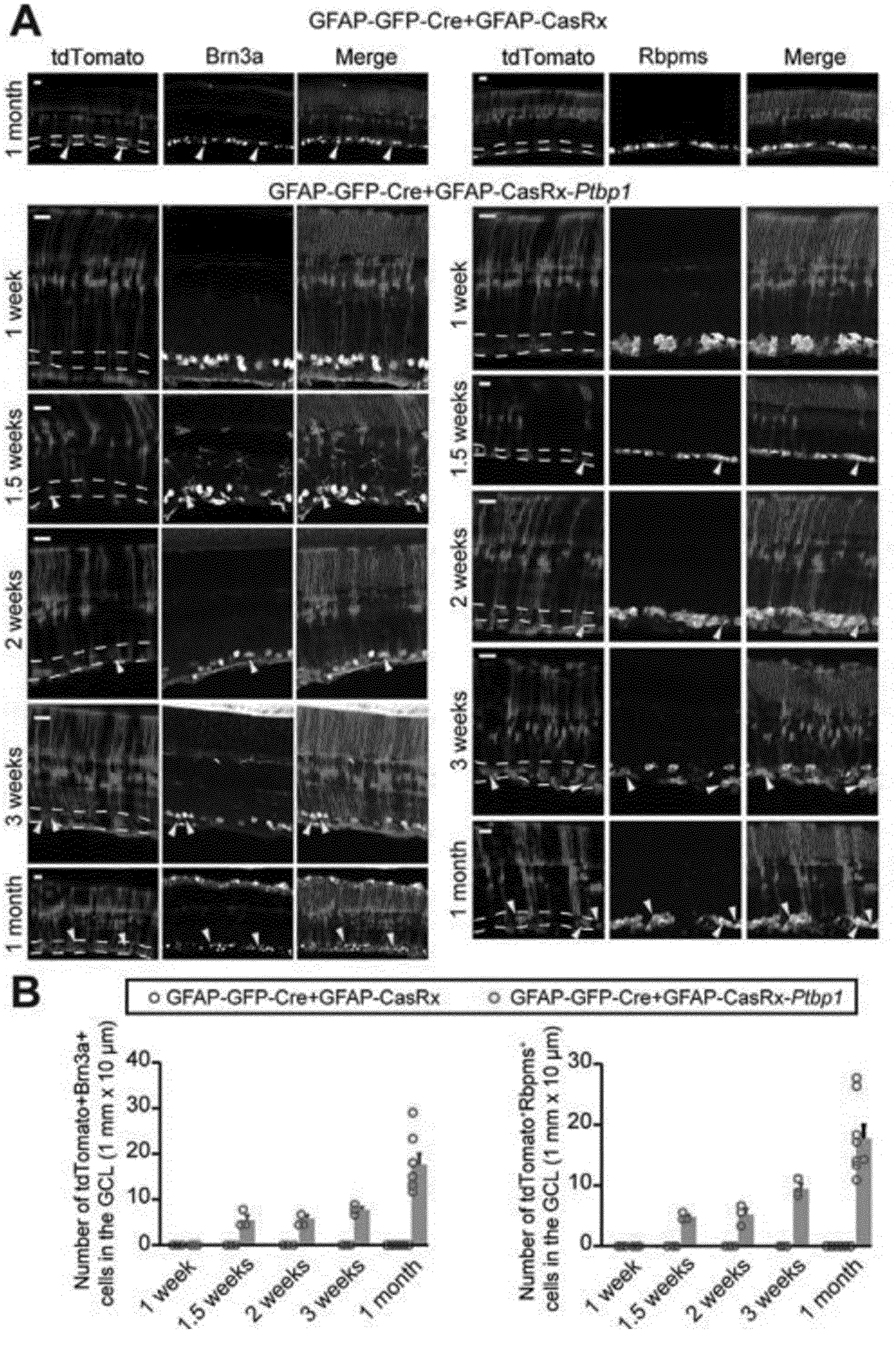
A
GFAP-GFP-Cre+GFAP-CasRx
tdTomato
Brn3a
Merge
tdTomato
Rbpms
Merge
1 month
GFAP-GFP-Cre+GFAP-CasRx-Ptbp1
1 week
1.5 weeks
2 weeks
3 weeks
1 month
B
GFAP-GFP-Cre+GFAP-CasRx
GFAP-GFP-Cre+GFAP-CasRx-Ptbp1
Number of tdTomato+Brn3a+ cells in the GCL (1 mm x 10 μm)
Number of tdTomato+Rbpms+ cells in the GCL (1 mm x 10 μm)
40
30
20
10
0
1 week
1.5 weeks
2 weeks
3 weeks
1 month

Figure 7

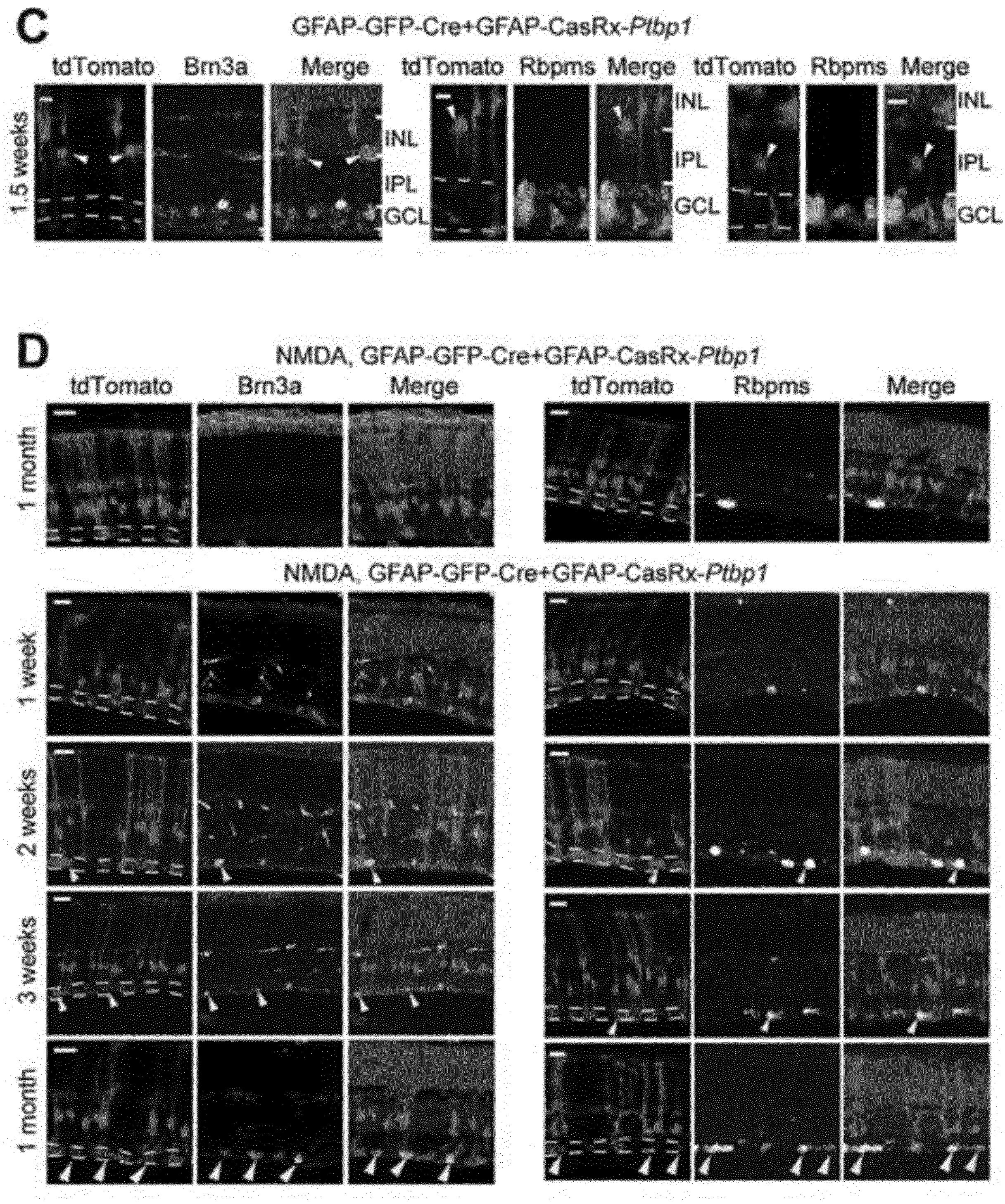


Figure 7 (continued)

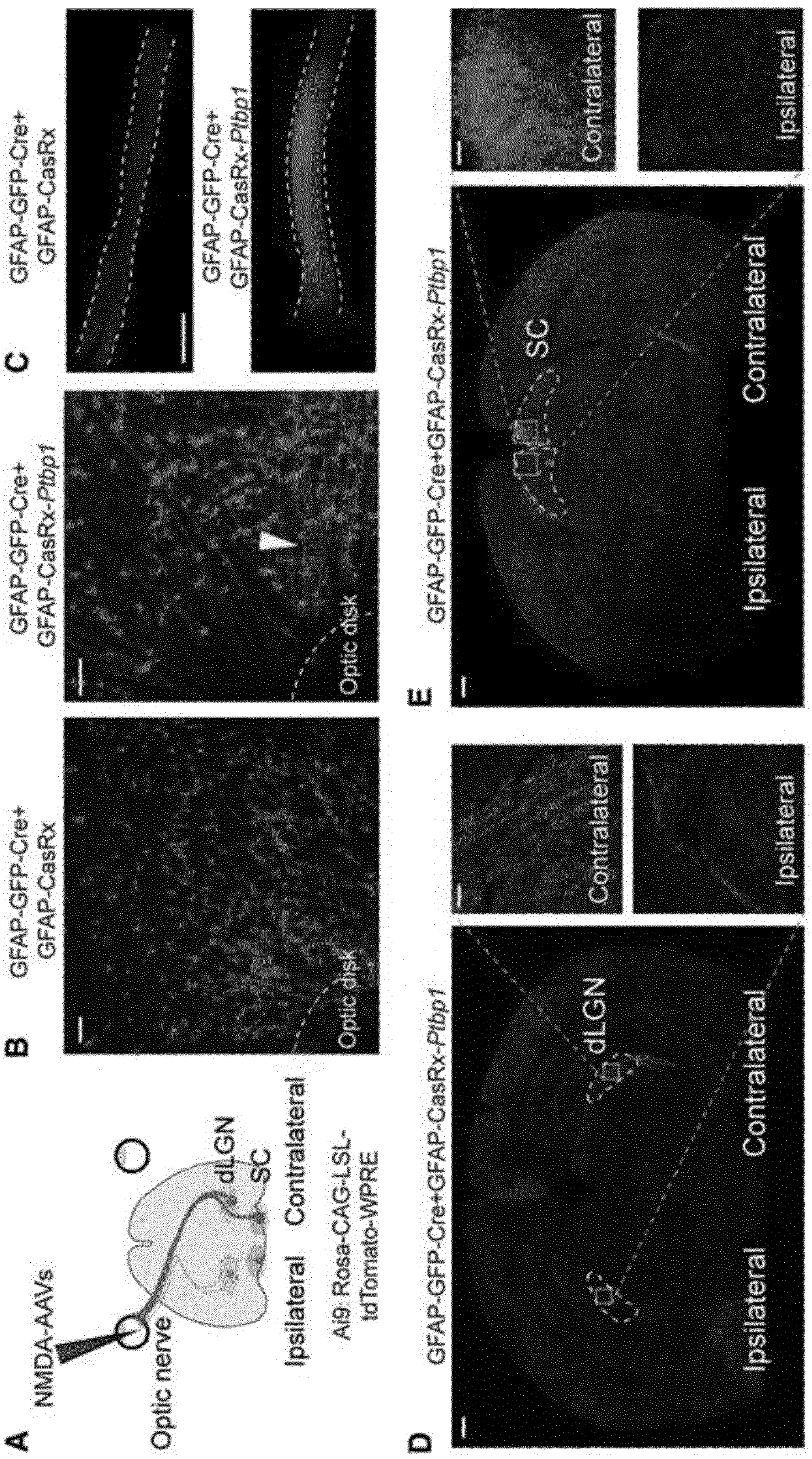
A
NMDA-AAVs
Optic nerve
dLGN
SC
Ipsilateral
Contralateral
Ai9: Rosa-CAG-LSL-tdTomato-WPRE
B
GFAP-GFP-Cre+
GFAP-CasRx
Optic disk
GFAP-GFP-Cre+
GFAP-CasRx-Ptbp1
Optic disk
C
GFAP-GFP-Cre+
GFAP-CasRx
GFAP-GFP-Cre+
GFAP-CasRx-Ptbp1
D
GFAP-GFP-Cre+GFAP-CasRx-Ptbp1
dLGN
Ipsilateral
Contralateral
Contralateral
Ipsilateral
E
GFAP-GFP-Cre+GFAP-CasRx-Ptbp1
SC
Ipsilateral
Contralateral
Contralateral
Ipsilateral

Figure 8

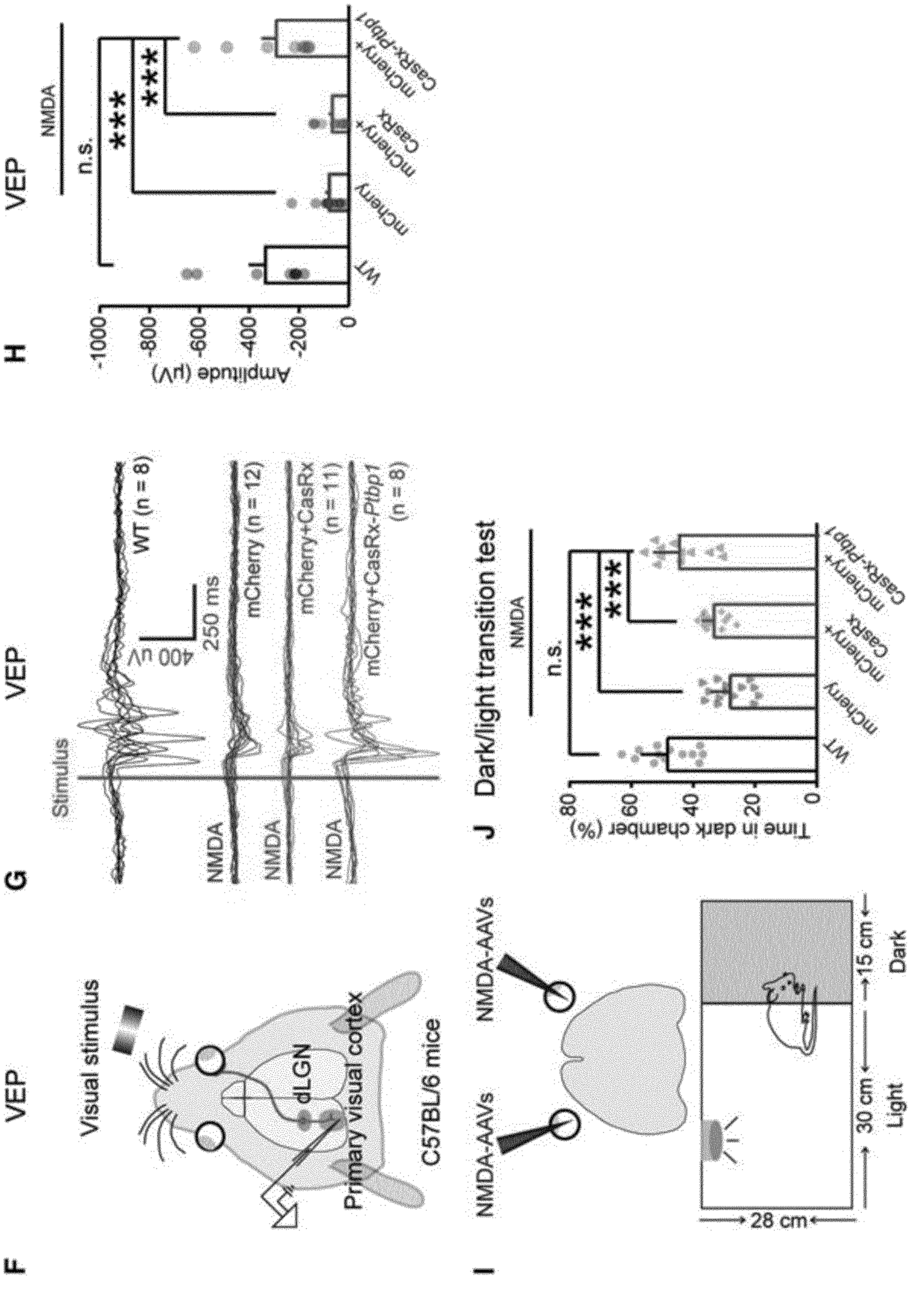


Figure 8 (continued)

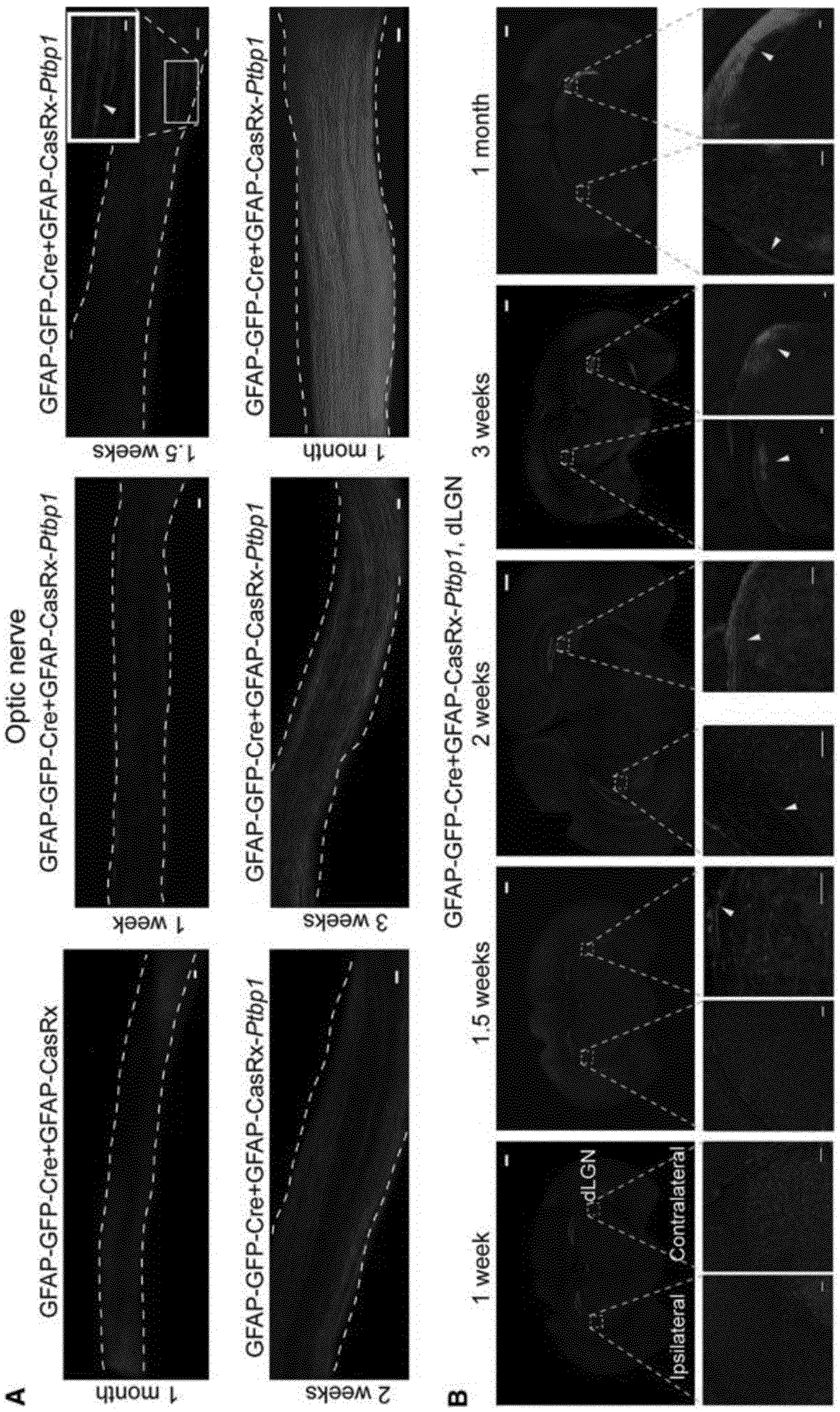
A
Optic nerve
GFAP-GFP-Cre+GFAP-CasRx
GFAP-GFP-Cre+GFAP-CasRx-Ptbp1
1 month
1 week
1.5 weeks
2 weeks
3 weeks
1 month
B
GFAP-GFP-Cre+GFAP-CasRx-Ptbp1, dLGN
1 week
1.5 weeks
2 weeks
3 weeks
1 month
dLGN
Ipsilateral
Contralateral

Figure 9

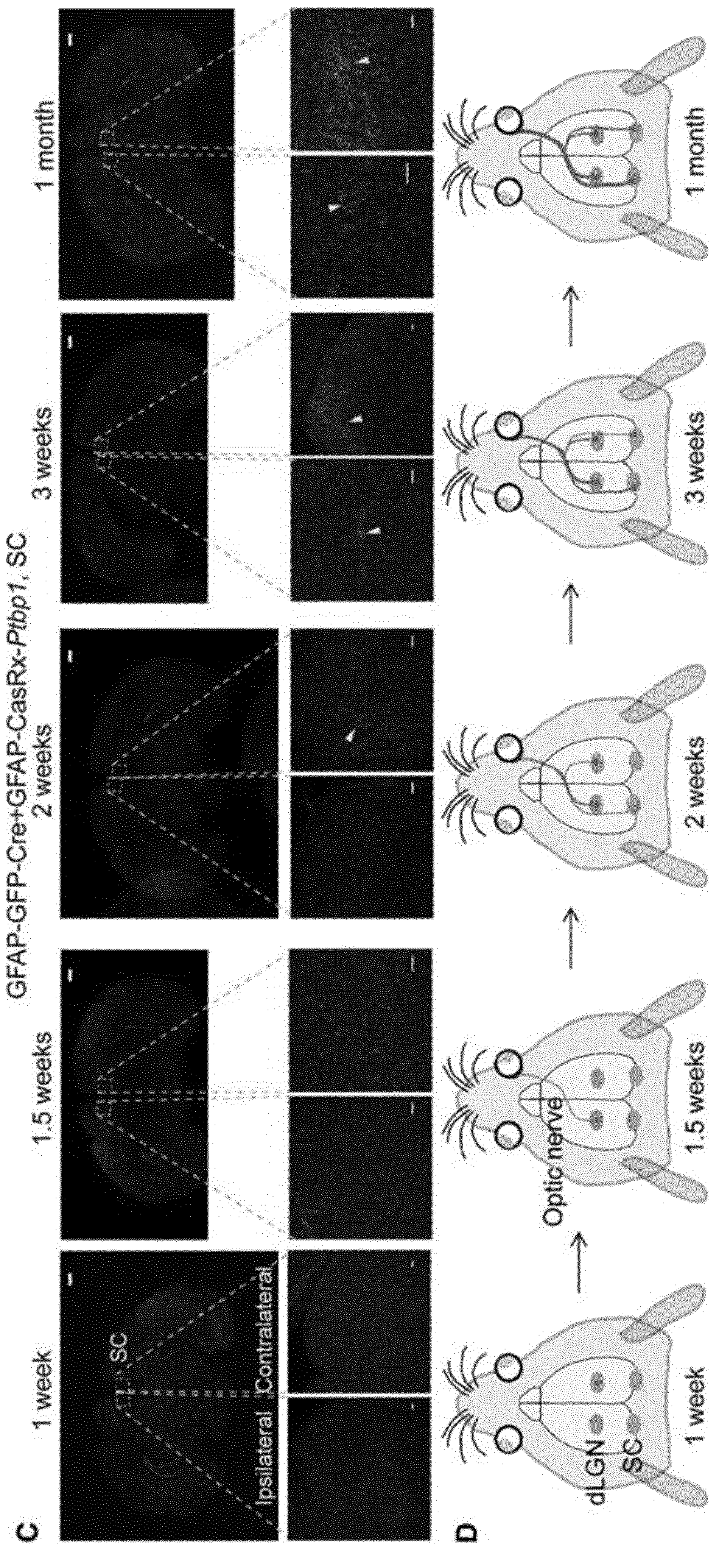


Figure 9 (continued)

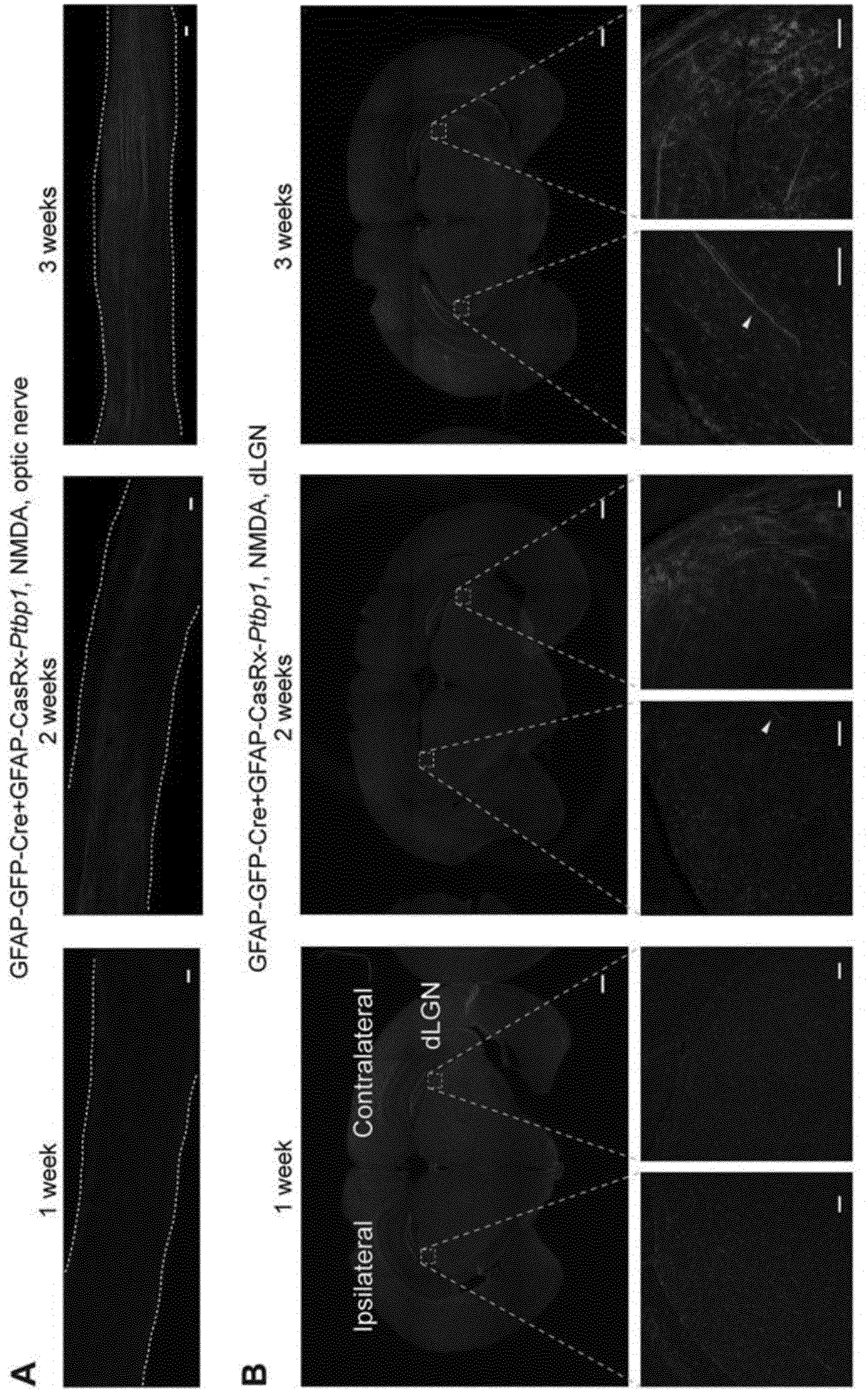
A
GFAP-GFP-Cre+GFAP-CasRx-*Ptbp1*, NMDA, optic nerve
1 week
2 weeks
3 weeks
B
GFAP-GFP-Cre+GFAP-CasRx-*Ptbp1*, NMDA, dLGN
1 week
2 weeks
3 weeks
Ipsilateral
Contralateral
dLGN

Figure 10

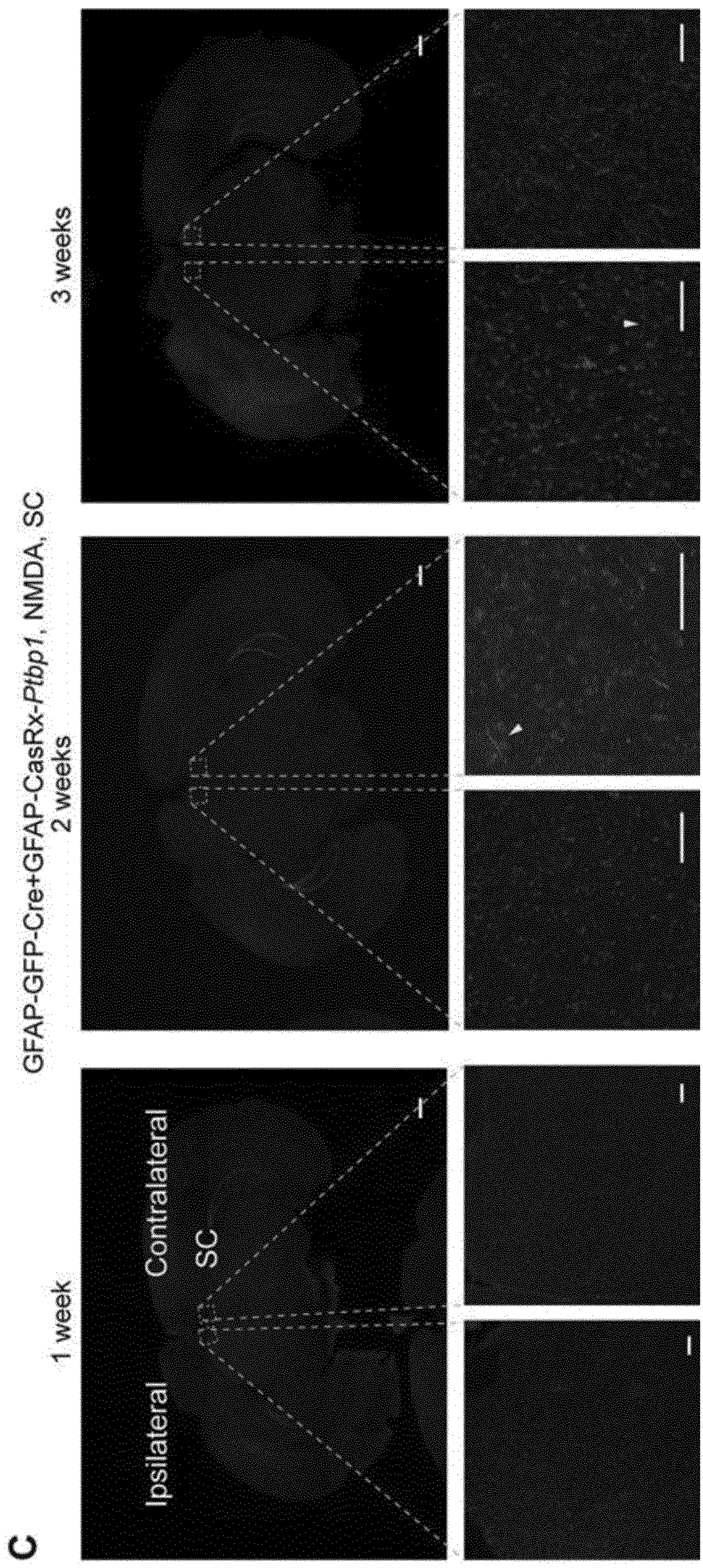


Figure 10 (continued)

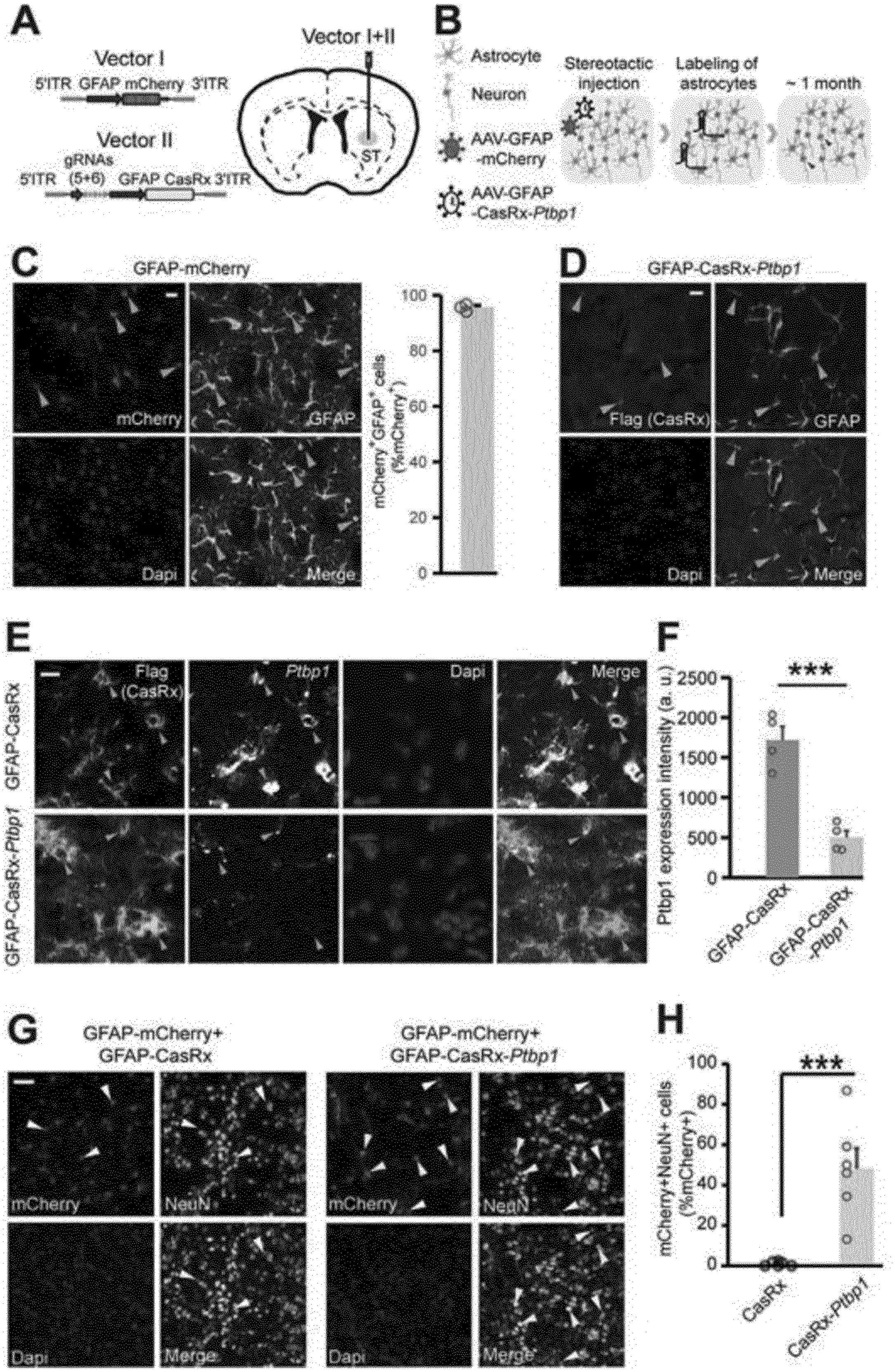
A
Vector I
5'ITR GFAP mCherry 3'ITR
Vector II
gRNAs
5'ITR (5+6) GFAP CasRx 3'ITR
Vector I+II
ST
B
Astrocyte
Neuron
AAV-GFAP -mCherry
AAV-GFAP -CasRx-Ptbp1
Stereotactic injection
Labeling of astrocytes
~ 1 month
C
GFAP-mCherry
mCherry
GFAP
Dapi
Merge
mCherry+GFAP+ cells (%mCherry+)
D
GFAP-CasRx-Ptbp1
Flag (CasRx)
GFAP
Dapi
Merge
E
GFAP-CasRx
GFAP-CasRx-Ptbp1
Flag (CasRx)
Ptbp1
Dapi
Merge
F
Ptbp1 expression intensity (a. u.)
***
GFAP-CasRx
GFAP-CasRx -Ptbp1
G
GFAP-mCherry+ GFAP-CasRx
GFAP-mCherry+ GFAP-CasRx-Ptbp1
mCherry
NeuN
Dapi
Merge
H
mCherry+NeuN+ cells (%mCherry+)
***
CasRx
CasRx-Ptbp1

Figure 11

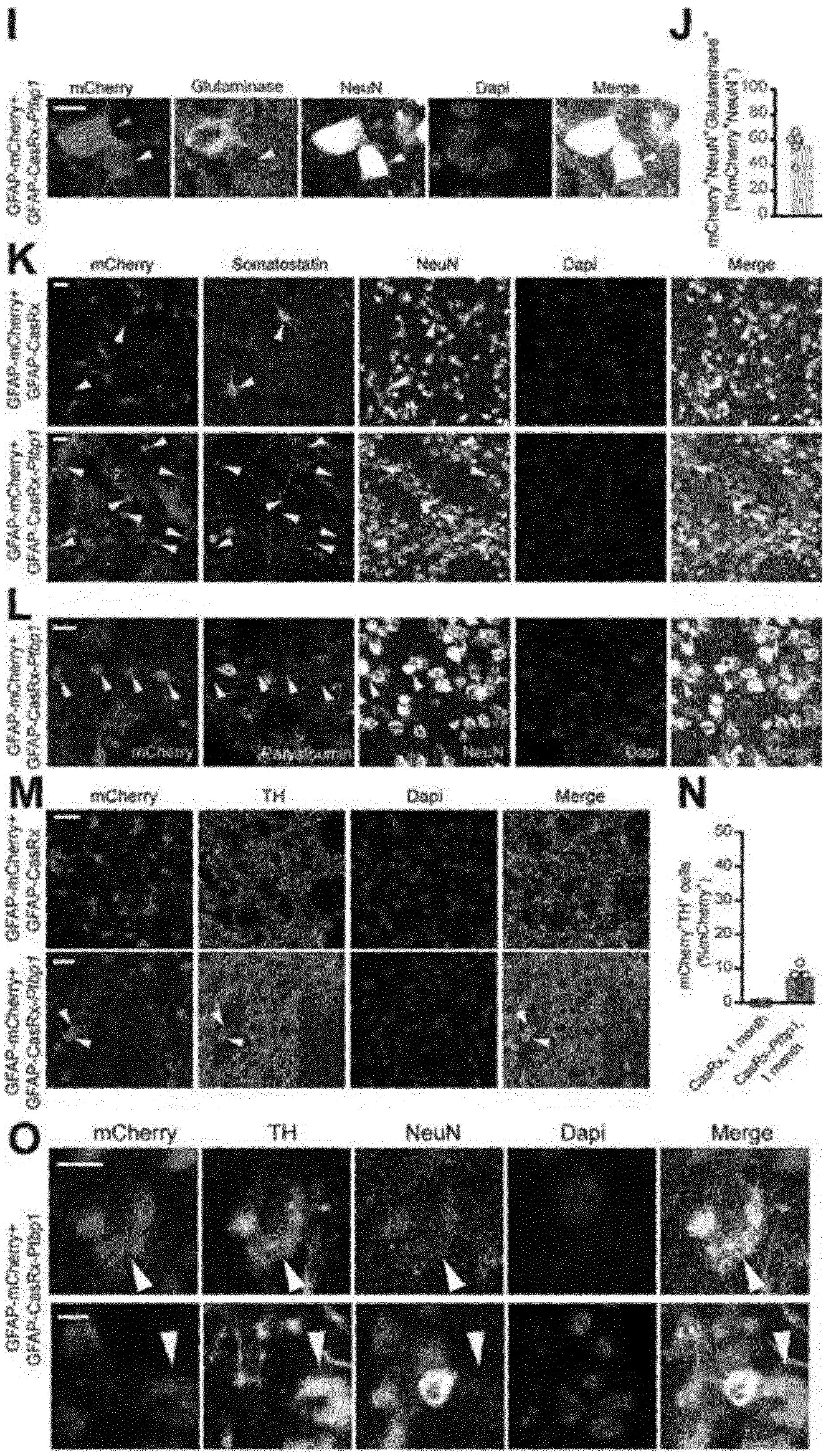


Figure 11 (continued)

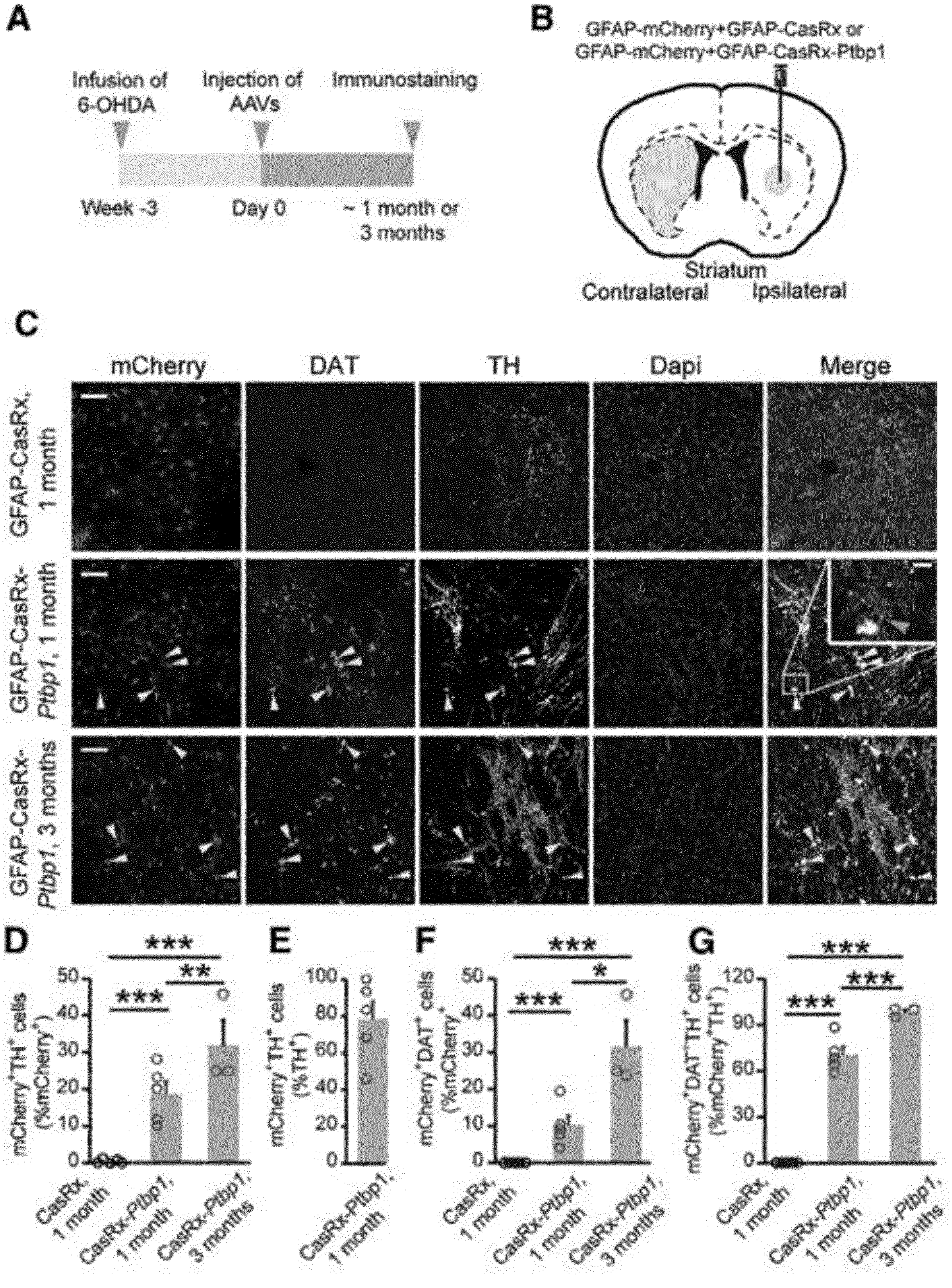
A
Infusion of 6-OHDA
Injection of AAVs
Immunostaining
Week -3
Day 0
~ 1 month or 3 months
B
GFAP-mCherry+GFAP-CasRx or
GFAP-mCherry+GFAP-CasRx-Ptbp1
Striatum
Contralateral
Ipsilateral
C
mCherry
DAT
TH
Dapi
Merge
GFAP-CasRx, 1 month
GFAP-CasRx-Ptbp1, 1 month
GFAP-CasRx-Ptbp1, 3 months
D
mCherry+TH+ cells (%mCherry+)
E
mCherry+TH+ cells (%TH+)
F
mCherry+DAT+ cells (%mCherry+)
G
mCherry+DAT+TH+ cells (%mCherry+TH+)
CasRx, 1 month
CasRx-Ptbp1, 1 month
CasRx-Ptbp1, 3 months

Figure 12

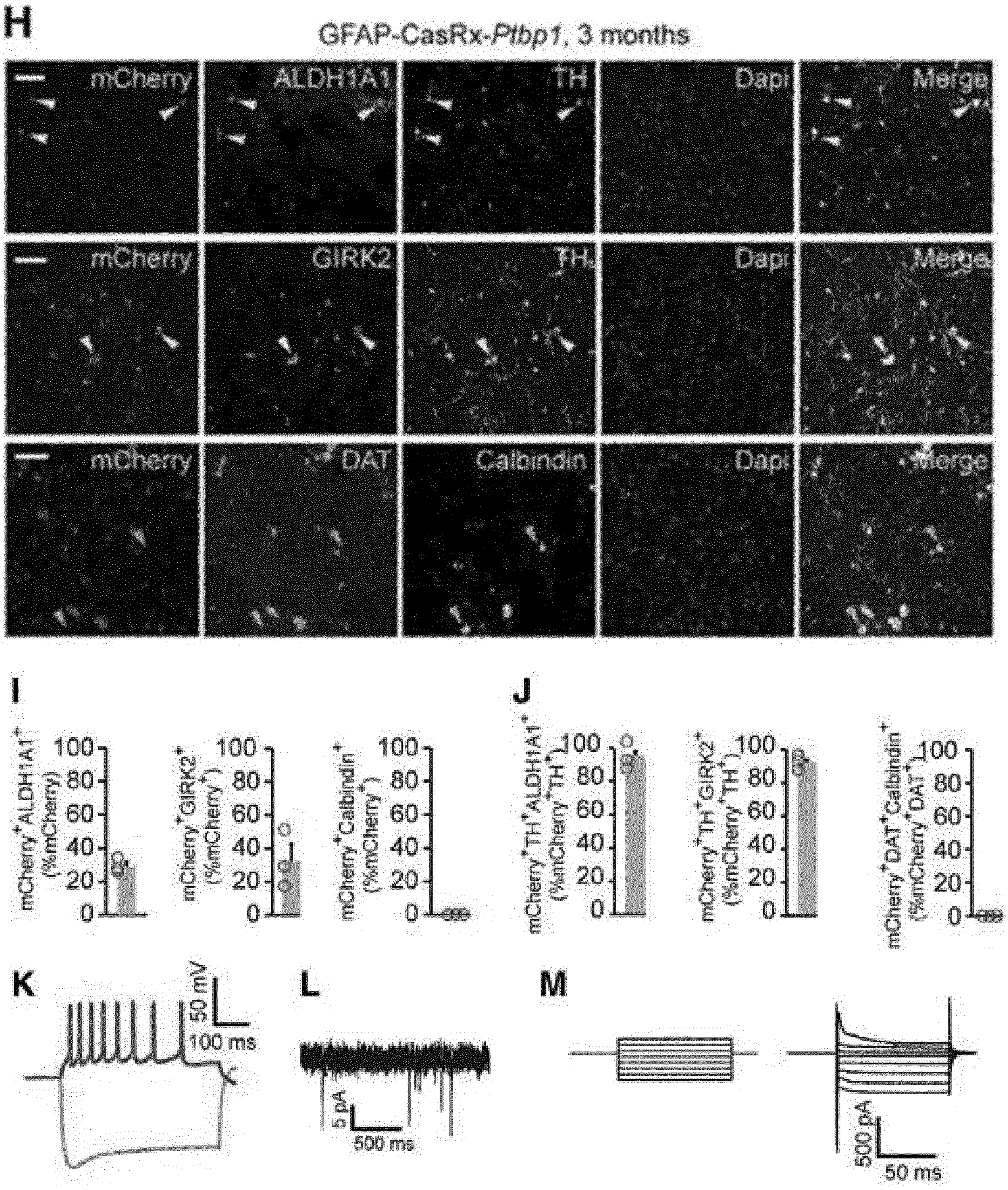


Figure 12 (continued)

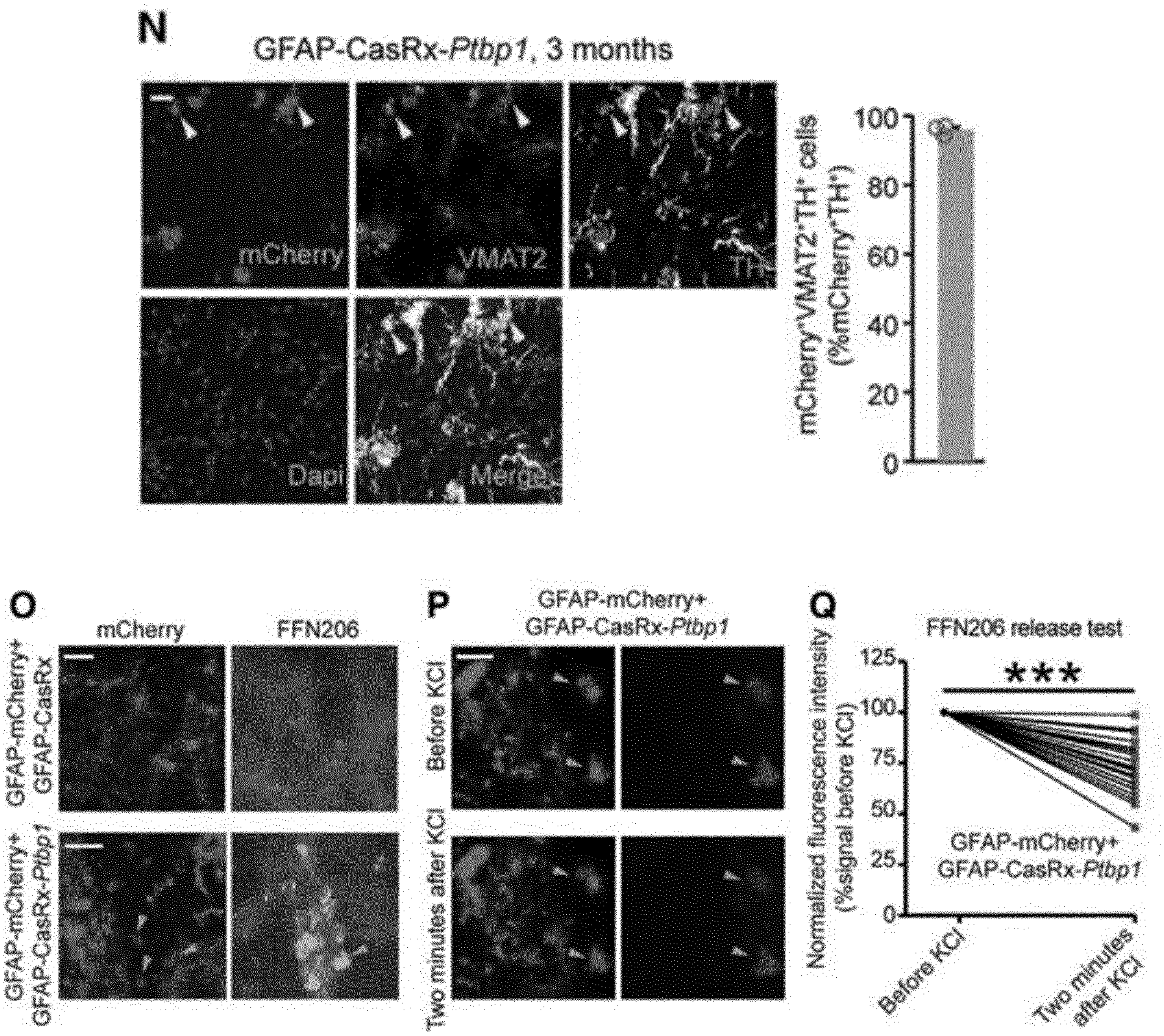


Figure 12 (continued)

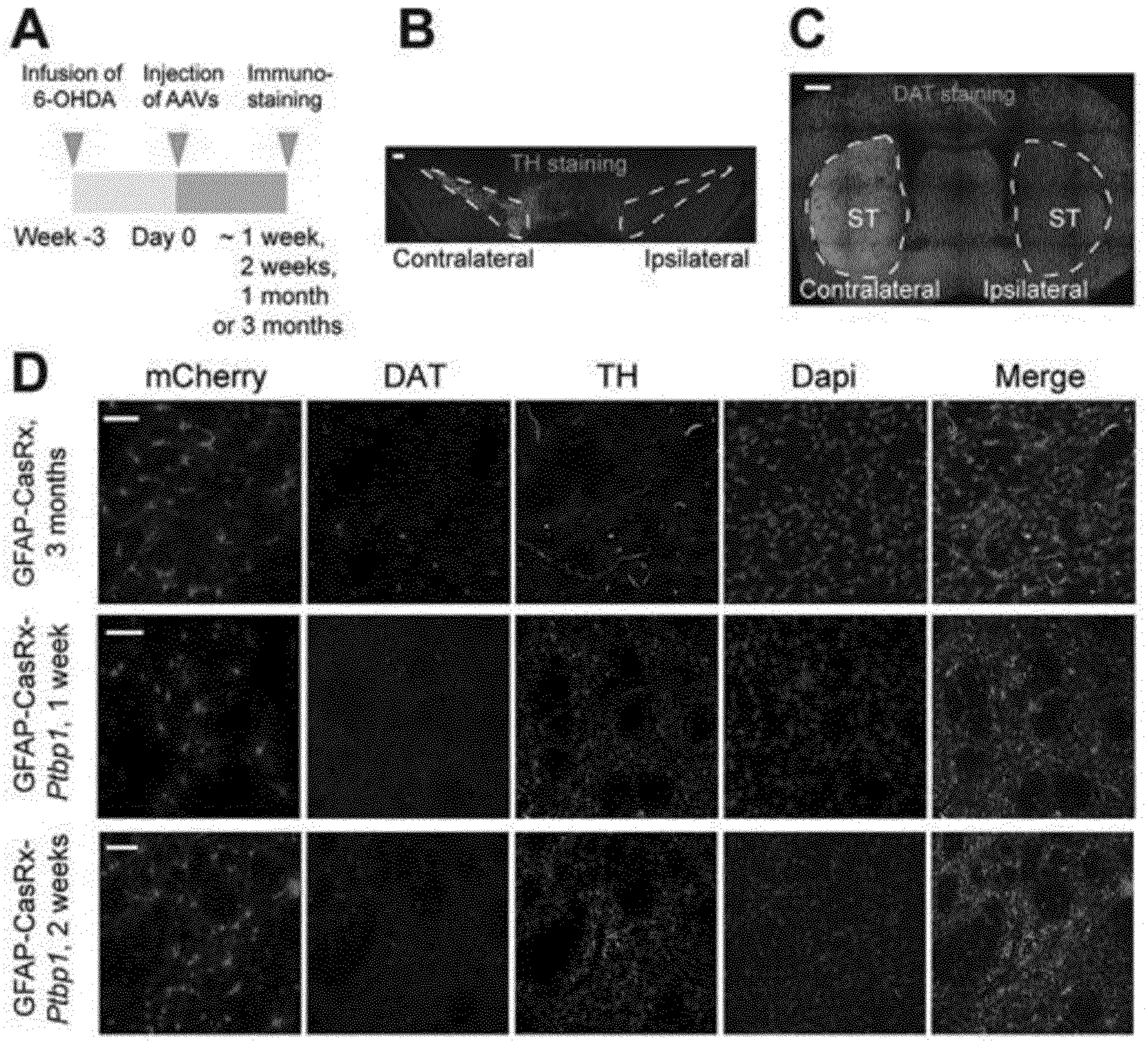
A
Infusion of 6-OHDA
Injection of AAVs
Immuno-staining
Week -3
Day 0
~ 1 week, 2 weeks, 1 month or 3 months
B
TH staining
Contralateral
Ipsilateral
C
DAT staining
ST
ST
Contralateral
Ipsilateral
D
mCherry
DAT
TH
Dapi
Merge
GFAP-CasRx, 3 months
GFAP-CasRx-Ptbp1, 1 week
GFAP-CasRx-Ptbp1, 2 weeks

Figure 13

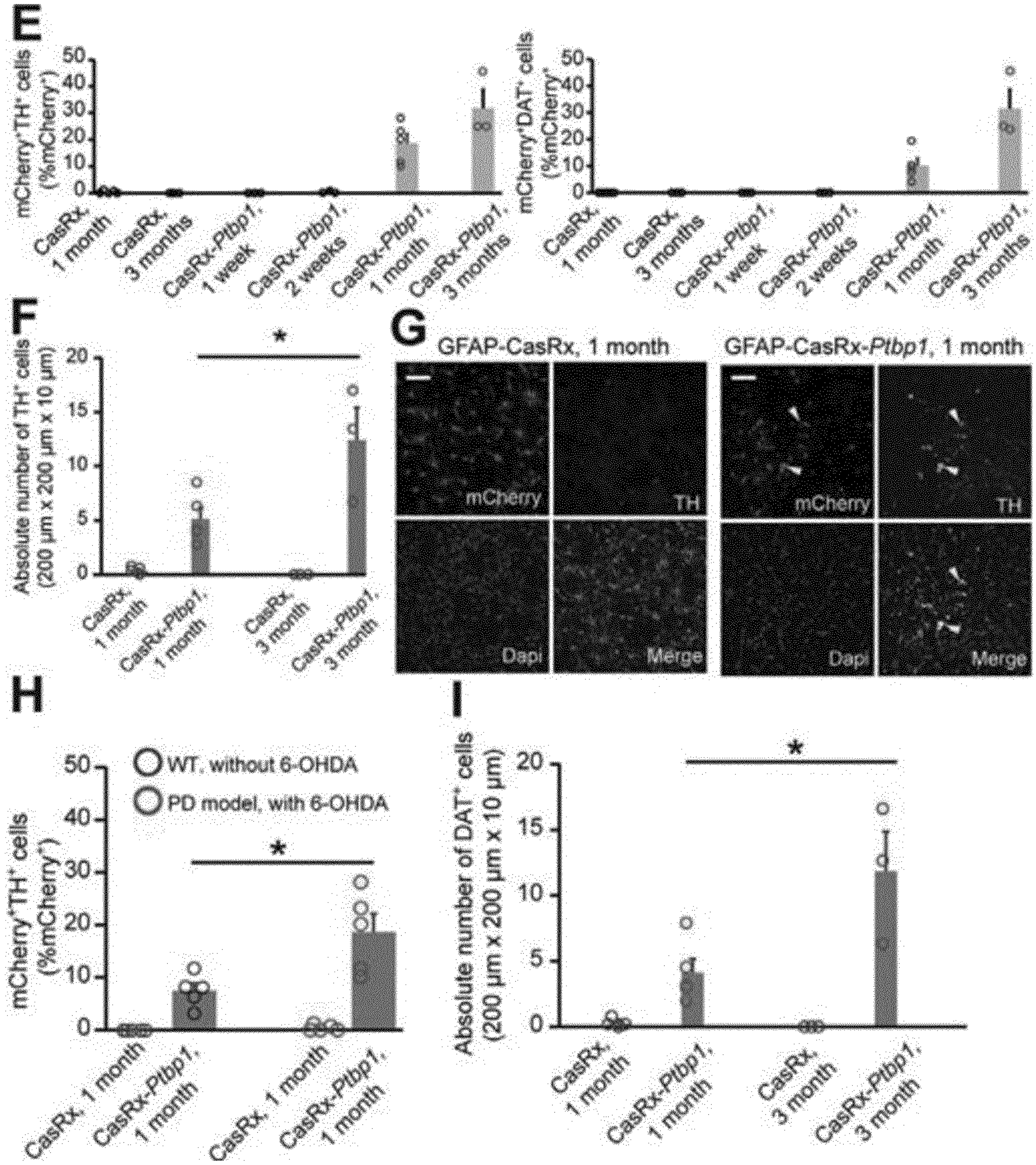


Figure 13 (continued)

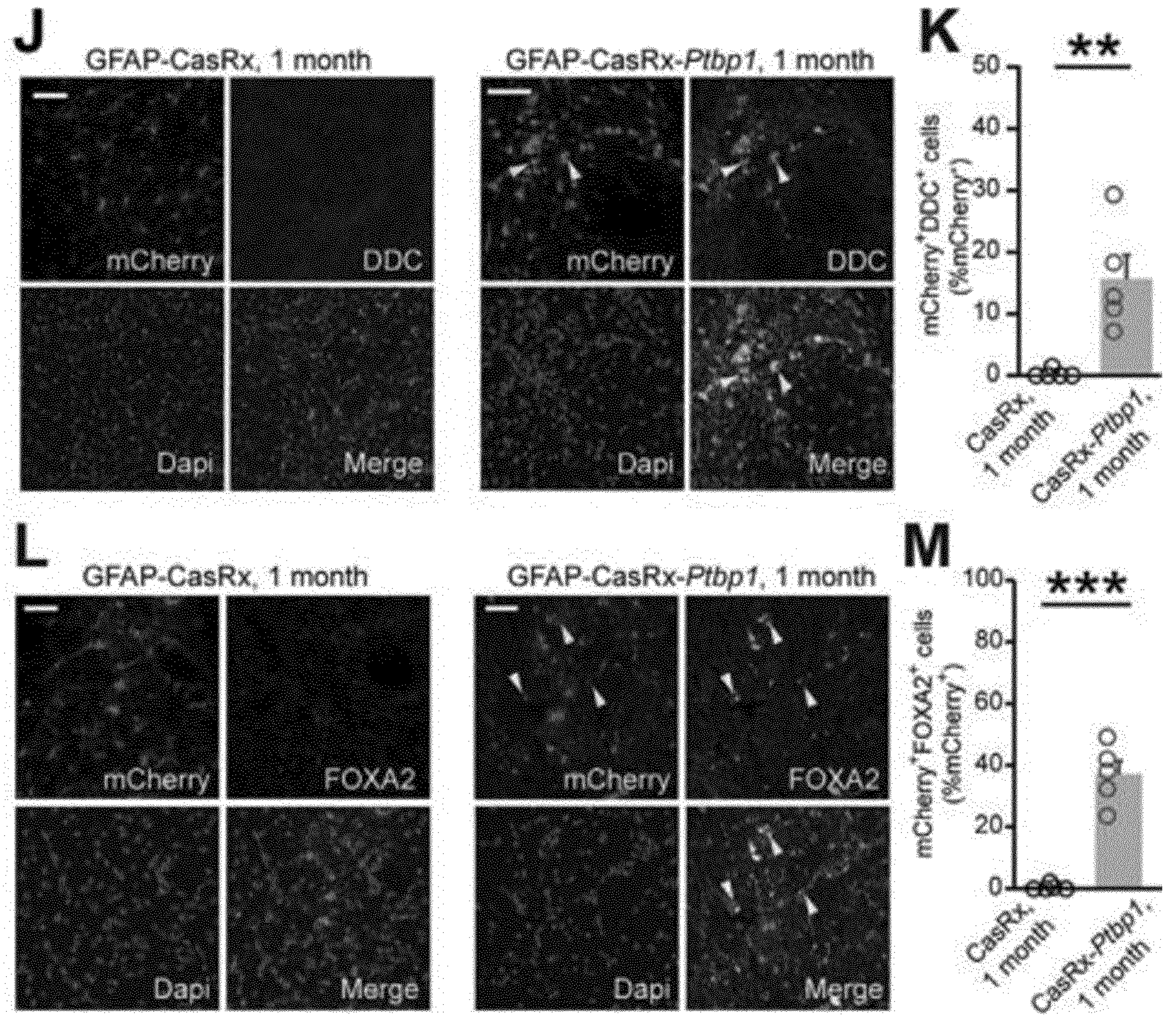


Figure 13 (continued)

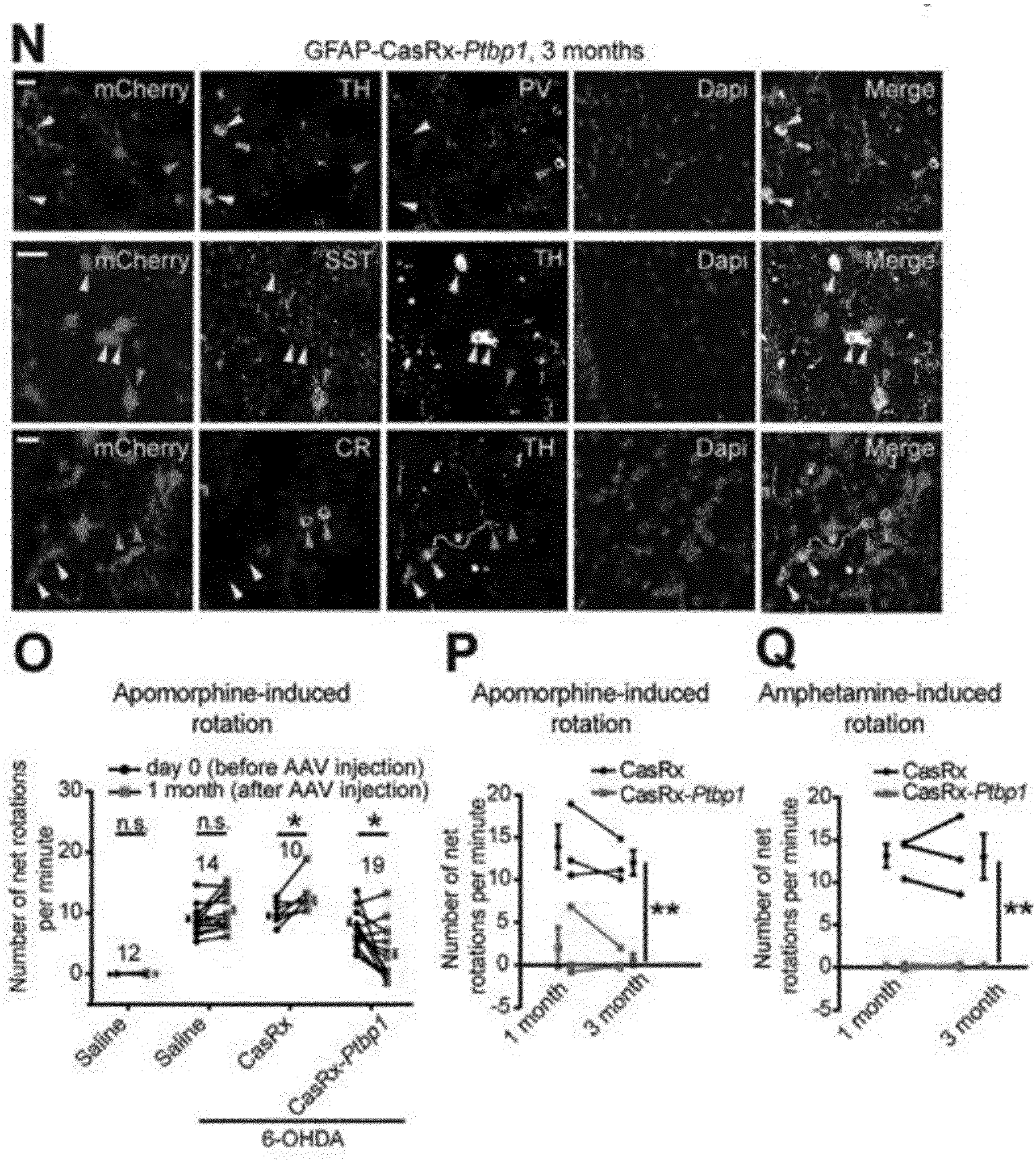


Figure 13 (continued)

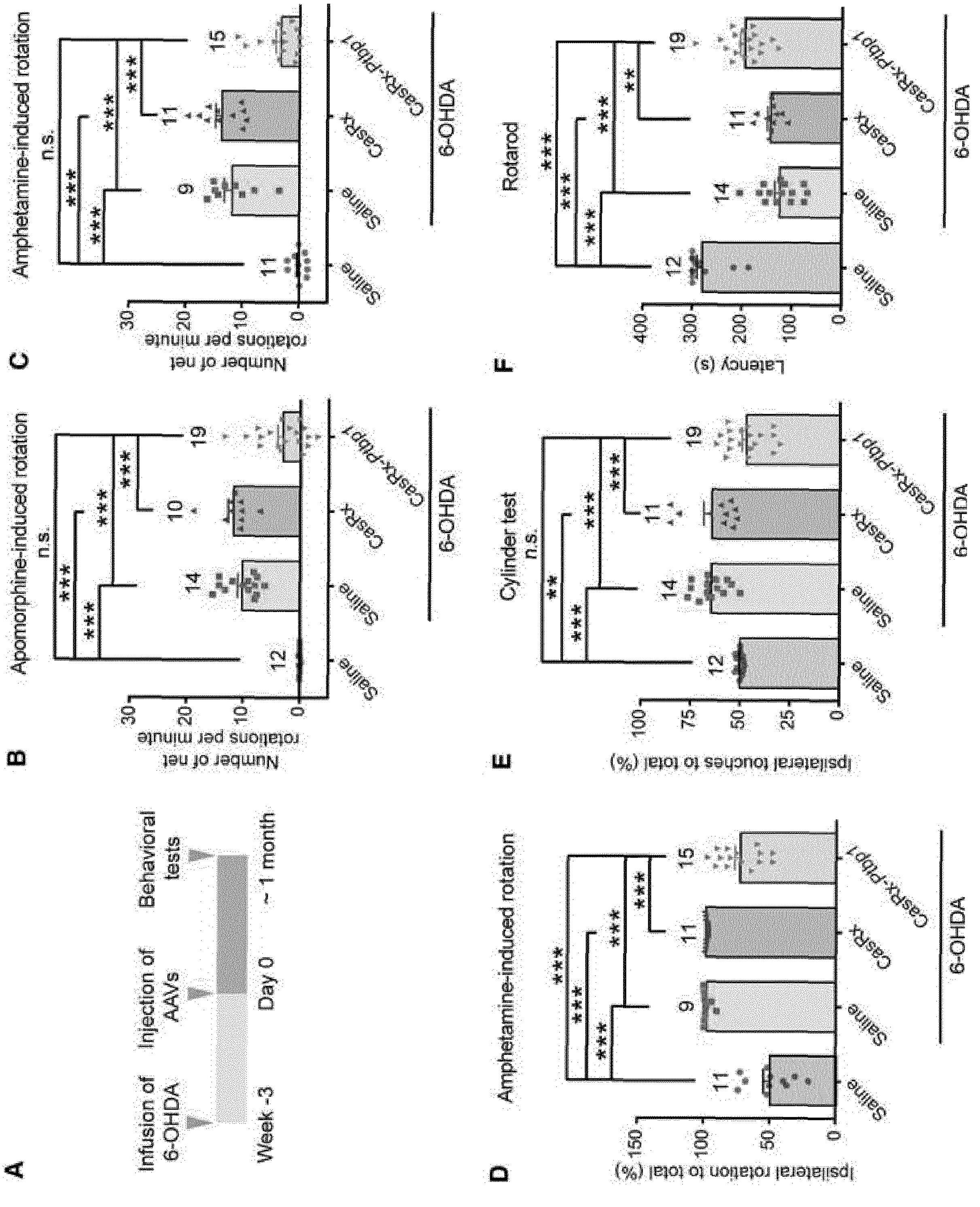
A
Infusion of 6-OHDA
Injection of AAVs
Behavioral tests
Week -3
Day 0
~ 1 month
B
Apomorphine-induced rotation
n.s.
Number of net rotations per minute
C
Amphetamine-induced rotation
n.s.
Number of net rotations per minute
D
Amphetamine-induced rotation
Ipsilateral rotation to total (%)
E
Cylinder test
n.s.
Ipsilateral touches to total (%)
F
Rotarod
Latency (s)
Saline
CasRx
CasRx-Ptbp1
6-OHDA

Figure 14

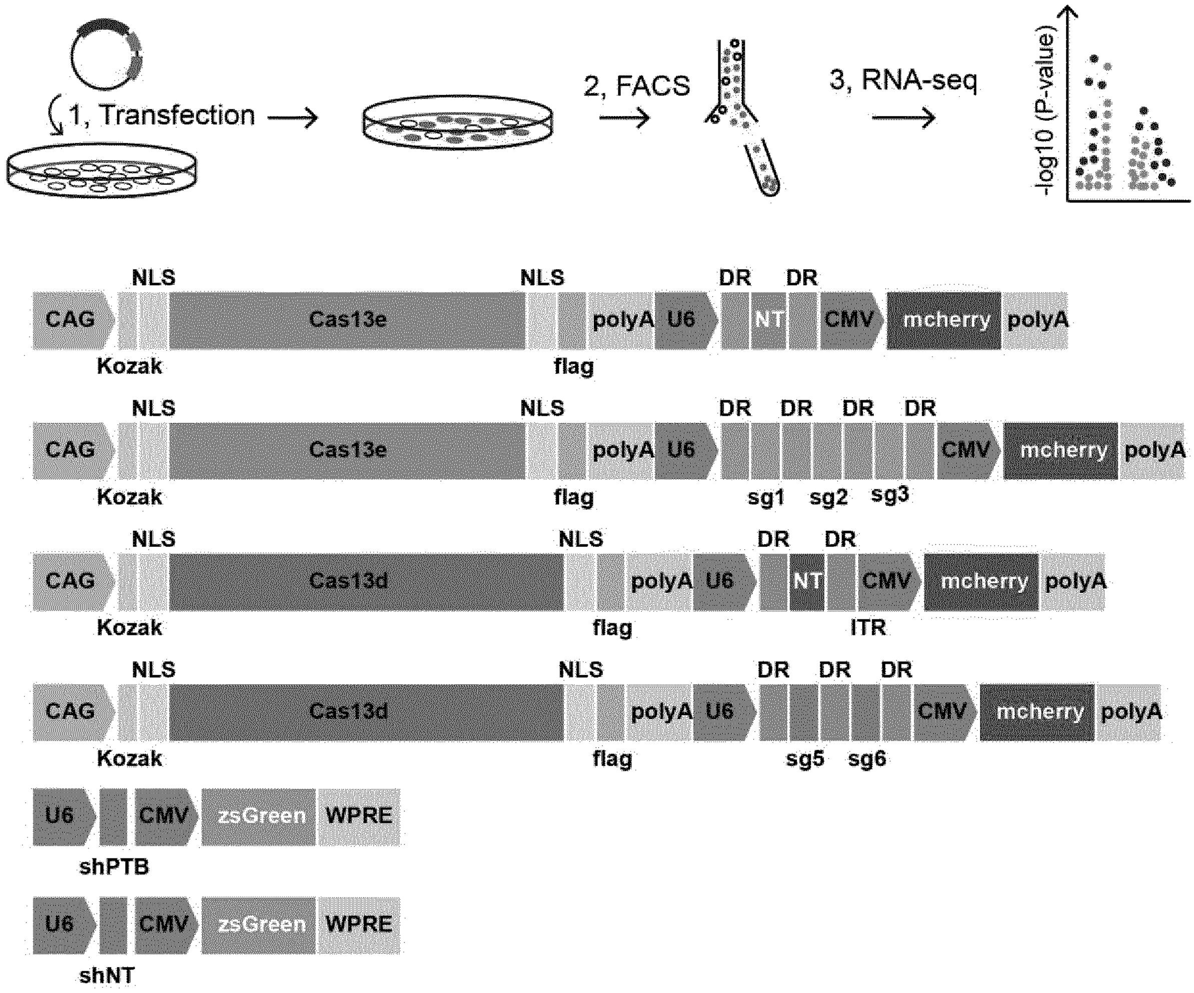
1, Transfection
2, FACS
3, RNA-seq
-log10 (P-value)
NLS
NLS
DR
DR
CAG
Cas13e
polyA
U6
NT
CMV
mcherry
polyA
Kozak
flag
NLS
NLS
DR
DR
DR
DR
CAG
Cas13e
polyA
U6
CMV
mcherry
polyA
Kozak
flag
sg1
sg2
sg3
NLS
NLS
DR
DR
CAG
Cas13d
polyA
U6
NT
CMV
mcherry
polyA
Kozak
flag
ITR
NLS
NLS
DR
DR
DR
CAG
Cas13d
polyA
U6
CMV
mcherry
polyA
Kozak
flag
sg5
sg6
U6
CMV
zsGreen
WPRE
shPTB
U6
CMV
zsGreen
WPRE
shNT

Figure 15

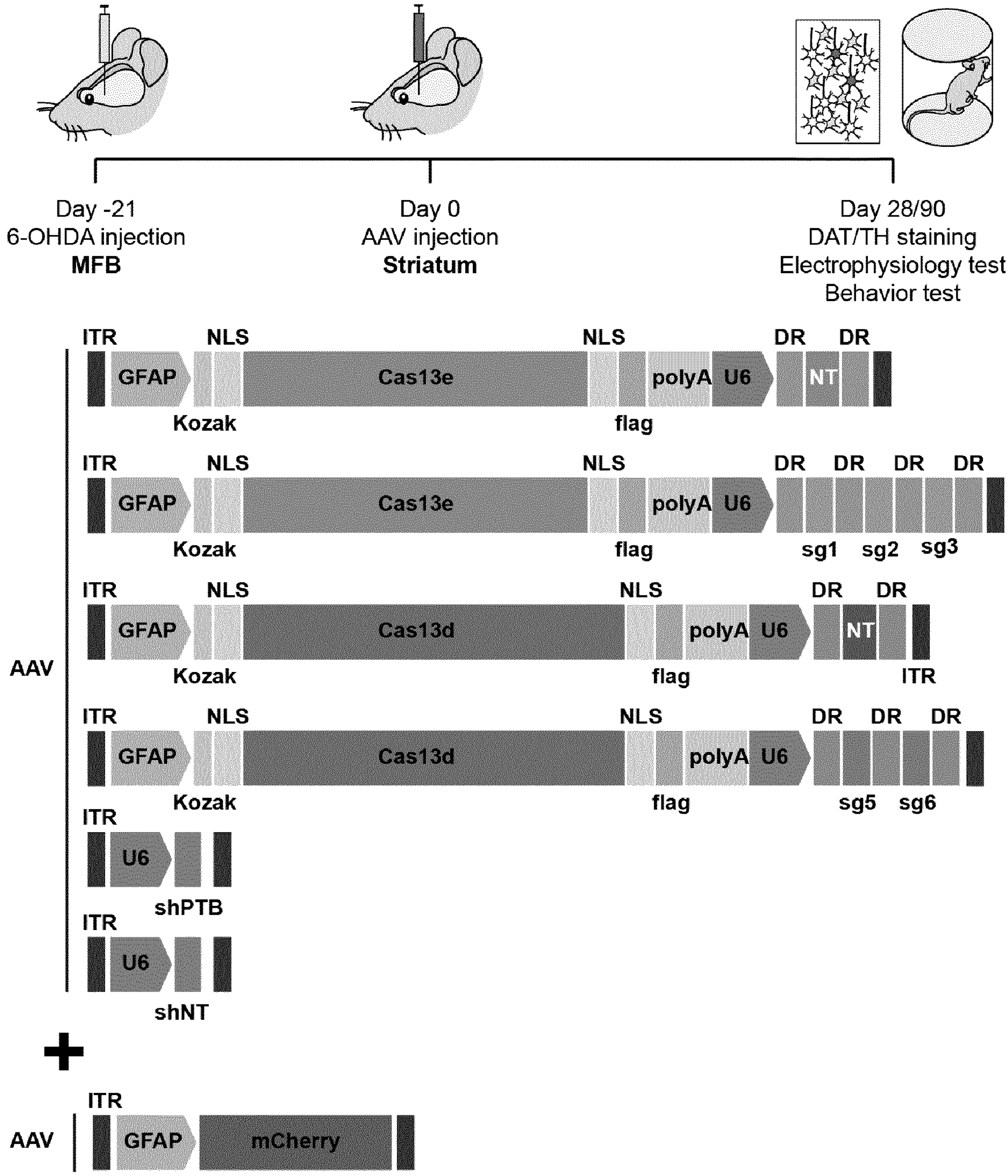
Day -21
6-OHDA injection
MFB
Day 0
AAV injection
Striatum
Day 28/90
DAT/TH staining
Electrophysiology test
Behavior test
AAV
ITR
GFAP
NLS
Kozak
Cas13e
NLS
flag
polyA
U6
DR
NT
DR
sg1
sg2
sg3
Cas13d
ITR
sg5
sg6
shPTB
shNT
mCherry

Figure 16

## INTERNATIONAL SEARCH REPORT

International application No.
**PCT/CN2020/109653**

**A. CLASSIFICATION OF SUBJECT MATTER**

C12N 5/079(2010.01)i; C12N 15/864(2006.01)i; A61K 48/00(2006.01)i; A61P 25/00(2006.01)i; C07K 14/47(2006.01)i

According to International Patent Classification (IPC) or to both national classification and IPC

**B. FIELDS SEARCHED**

Minimum documentation searched (classification system followed by classification symbols)

C12N; A61K; A61P; C07K

Documentation searched other than minimum documentation to the extent that such documents are included in the fields searched

Electronic data base consulted during the international search (name of data base and, where practicable, search terms used)

VEN; DWPI; EPODOC; CNABS; PATENTICS; CNKI; WANFANG; ISI WEB OF SCIENCE: 中国科学院脑科学与智能技术卓越创新中心, PTBP1, PTB, 抑制剂, 多巴胺神经元, 胶质细胞, 星型胶质细胞, 穆勒细胞, 视神经节细胞, 帕金森, 神经退行性疾病, 基因编辑, 筛选, polypyrimidine trac-binding protein 1, inhibit, dopaminergic neuron, glial, astrocyte, Muller cell, RGC, Parkinson's , neurodegenerative disease, CRISPR, screen

**C. DOCUMENTS CONSIDERED TO BE RELEVANT**

| Category* | Citation of document, with indication, where appropriate, of the relevant passages | Relevant to claim No. |
|---|---|---|
| PX | ZHOU, Hua et al. "Glia-to-Neuron Conversion by CRISPR-CasRx Alleviates Symptoms of Neurological Disease in Mice" *CELL*, Vol. 181, 30 April 2020 (2020-04-30), pp. 1-14 | 1-11 |
| PX | WO 2019200129 A1 (THE REGENTS OF THE UNIVERSITY OF CALIFORNIA) 17 October 2019 (2019-10-17) claims 1-121, description, embodiment 11 | 1-11 |
| PX | WO 2020113338 A1 (ADAERATA, LIMITED PARTNERSHIP) 11 June 2020 (2020-06-11) claims 1-22, description, embodiment 18 | 1-11 |
| X | WO 2014071157 A1 (THE REGENTS OF THE UNIVERSITY OF CALIFORNIA) 08 May 2014 (2014-05-08) claims 1, 5, 15-16, description, page 30, paragraphs 2-33 from the bottom | 1-9, 11 |
| Y | WO 2014071157 A1 (THE REGENTS OF THE UNIVERSITY OF CALIFORNIA) 08 May 2014 (2014-05-08) claims 1, 5, 15-16, description, page 30, paragraphs 2-33 from the bottom | 2, 4, 10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 November 2020** | **25 November 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT

International application No.
**PCT/CN2020/109653**

**C. DOCUMENTS CONSIDERED TO BE RELEVANT**

| Category* | Citation of document, with indication, where appropriate, of the relevant passages | Relevant to claim No. |
|---|---|---|
| X | WO 2017003466 A1 (UNIVERSITY OF SOUTH FLORIDA) 05 January 2017 (2017-01-05) claims 1-13 | 1-9, 11 |
| Y | WO 2017003466 A1 (UNIVERSITY OF SOUTH FLORIDA) 05 January 2017 (2017-01-05) claims 1-13 | 2, 4, 10 |
| X | WO 2018048877 A1 (THE UNIVERSITY OF NORTH CAROLINA AT CHAPEL HILL) 15 March 2018 (2018-03-15) claims 1-36 | 1-9, 11 |
| Y | WO 2018048877 A1 (THE UNIVERSITY OF NORTH CAROLINA AT CHAPEL HILL) 15 March 2018 (2018-03-15) claims 1-36 | 2-4, 10 |
| Y | US 2010144838 A1 (BECK, W. T. et al.) 10 June 2010 (2010-06-10) claims 1, 24-25, 28, description, table 1, embodiment 5 | 10 |
| X | XUE, Y.C. et al. "Direct Conversion of Fibroblasts to Neurons by Reprogramming PTB-Regulated microRNA Circuits" *CELL*, Vol. 152, 17 January 2013 (2013-01-17), pp. 82-96 | 1-9, 11 |
| Y | XUE, Y.C. et al. "Direct Conversion of Fibroblasts to Neurons by Reprogramming PTB-Regulated microRNA Circuits" *CELL*, Vol. 152, 17 January 2013 (2013-01-17), pp. 82-96 | 2-4, 10 |
| Y | WOHL, S.G. et al. "miR-124-9-9 potentiates Ascl1-induced reprogramming of cultured Müller glia" *GLIA*, Vol. 64, No. 5, 31 May 2016 (2016-05-31), pp. 743-762 | 2-4 |
| A | LINARES, A.J. et al. "The splicing regulator PTBP1 controls the activity of the transcription factor Pbx1 during neuronal differentiation" *eLife*, Vol. 4, 24 December 2015 (2015-12-24), document no. e09268, full text, pages 1-25 | 1-11 |

Form PCT/ISA/210 (second sheet) (January 2015)

**INTERNATIONAL SEARCH REPORT**
**Information on patent family members**

International application No.
**PCT/CN2020/109653**

| Patent document cited in search report | | | Publication date (day/month/year) | Patent family member(s) | | | Publication date (day/month/year) |
|---|---|---|---|---|---|---|---|
| WO | 2019200129 | A1 | 17 October 2019 | None | | | |
| WO | 2020113338 | A1 | 11 June 2020 | None | | | |
| WO | 2014071157 | A1 | 08 May 2014 | US | 2019119673 | A1 | 25 April 2019 |
| | | | | US | 2015299698 | A1 | 22 October 2015 |
| WO | 2017003466 | A1 | 05 January 2017 | US | 2017002317 | A1 | 05 January 2017 |
| | | | | US | 9481864 | B1 | 01 November 2016 |
| WO | 2018048877 | A1 | 15 March 2018 | US | 2019224256 | A1 | 25 July 2019 |
| US | 2010144838 | A1 | 10 June 2010 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 6566118 B
- US 6989264 B
- US 6995006 B
- US 2005027091 W
- WO 9118088 A
- WO 9309239 A
- US 4797368 A
- US 6596535 B
- US 5139941 A
- EP 0488528 A

### Non-patent literature cited in the description

- molecular cloning: conditions. **SAMBROOK.** laboratory manual. Cold Spring Harbor Laboratory Press, 1989
- **MCCARTHY, K.D. ; DEVELLIS, J.** Preparation Of Separate Astroglial And Oligodendroglial Cell-Cultures From Rat Cerebral Tissue. *Journal Of Cell Biology,* 1980, vol. 85, 890-902
- **ZHOU, H. et al.** Cerebellar modules operate at different frequencies. *Elife,* 2014, vol. 3, e02536
- **XU, H.T. et al.** Distinct lineage-dependent structural and functional organization of the hippocampus. *Cell,* 2014, vol. 157, 1552-1564
- **SU, J. et al.** Reduction of HIP2 expression causes motor function impairment and increased vulnerability to dopaminergic degeneration in Parkinson's disease models. *Cell Death Dis,* 2018, vol. 9, 1020
- **CHAVEZ, A. et al.** Comparison of Cas9 activators in multiple species. *Nat Methods,* 2016, vol. 13, 563-567
- **QIAN, HAO et al.** Reversing a model of Parkinson's disease with in situ converted nigral neurons. *Nature,* 2020, vol. 582, 550-556
- **ZHOU, HAIBO et al.** Glia-to-Neuron Conversion by CRISPR-CasRx Alleviates Symptoms of Neurological Disease in Mice. *Cell,* 2020, vol. 181, 590-603.e16

sequence listing

<110> CENTER FOR EXCELLENCE IN BRAIN SCIENCE AND INTELLIGENCE TECHNOLOGY, CHINESE ACADEMY OF SCIENCES

<120> APPLICATION OF PTBP1 INHIBITOR IN PREVENTING AND/OR TREATING NERVOUS SYSTEM DISEASE RELATED TO FUNCTIONAL NEURONAL DEATH

<130> P2020-0568

<150> CN 201910760367.6
<151> 2019-08-16

<150> CN 201911046435.9
<151> 2019-10-30

<150> PCT/CN2020/081489
<151> 2020-03-26

<150> CN 202010740568.2
<151> 2020-07-28

<160> 17

<170> PatentIn version 3.5

<210> 1
<211> 29
<212> DNA
<213> artificial sequence

<220>
<223> guide 1

<400> 1
tttgtaccga ctgctatgtc tgggacgat 29

<210> 2
<211> 30
<212> DNA
<213> artificial sequence

<220>
<223> guide 2

<400> 2
ggctggctgt ctccagaggg caggtcaggt 30

<210> 3
<211> 30
<212> DNA
<213> artificial sequence

<220>
<223> guide 3

<400> 3
gtatagtagt taaccatagt gttggcagcc 30

<210> 4
<211> 30
<212> DNA
<213> artificial sequence

<220>
<223> guide 4

<400> 4
gctgtcggtc ttgagctctt tgtggttgga 30

<210> 5
<211> 30
<212> DNA
<213> artificial sequence

<220>
<223> guide 5

<400> 5
tgtagatggg ctgtccacga agcactggcg 30

<210> 6
<211> 30
<212> DNA
<213> artificial sequence

<220>
<223> guide 6

<400> 6
gcttggagaa gtcgatgcgc agcgtgcagc 30

<210> 7
<211> 20
<212> DNA
<213> artificial sequence

<220>
<223> forward primer

<400> 7
agaggaggct gccaacacta 20

<210> 8
<211> 20
<212> DNA
<213> artificial sequence

<220>
<223> reverse primer

<400> 8
gtccagggtc actgggtaga 20

<210> 9
<211> 19
<212> DNA

<213> artificial sequence

<220>
<223> forward primer

<400> 9
ccctggtgtc cggctctaa 19

<210> 10
<211> 20
<212> DNA
<213> artificial sequence

<220>
<223> reverse primer

<400> 10
ggactcgccg aagtacctct 20

<210> 11
<211> 555
<212> PRT
<213> Homo sapiens

<400> 11

```
Met Asp Gly Ile Val Pro Asp Ile Ala Val Gly Thr Lys Arg Gly Ser
1               5                   10                  15

Asp Glu Leu Phe Ser Thr Cys Val Ser Asn Gly Pro Phe Ile Met Ser
            20                  25                  30

Ser Ser Ala Ser Ala Ala Asn Gly Asn Asp Ser Lys Lys Phe Lys Gly
        35                  40                  45

Asp Asn Arg Ser Ala Gly Val Pro Ser Arg Val Ile His Val Arg Lys
    50                  55                  60

Leu Pro Ser Asp Val Thr Glu Gly Glu Val Ile Ser Leu Gly Leu Pro
65                  70                  75                  80

Phe Gly Lys Val Thr Asn Leu Leu Met Leu Lys Gly Lys Asn Gln Ala
                85                  90                  95

Phe Ile Glu Met Asn Thr Glu Glu Ala Ala Asn Thr Met Val Asn Tyr
            100                 105                 110

Tyr Thr Ser Val Ala Pro Val Leu Arg Gly Gln Pro Ile Tyr Ile Gln
        115                 120                 125

Phe Ser Asn His Lys Glu Leu Lys Thr Asp Ser Ser Pro Asn Gln Ala
    130                 135                 140
```

Arg Ala Gln Ala Ala Leu Gln Ala Val Asn Ser Val Gln Ser Gly Asn
145 150 155 160

Leu Ala Leu Ala Ala Ser Ala Ala Ala Val Asp Ala Gly Met Ala Met
165 170 175

Ala Gly Gln Ser Pro Val Leu Arg Ile Ile Val Glu Asn Leu Phe Tyr
180 185 190

Pro Val Thr Leu Asp Val Leu His Gln Ile Phe Ser Lys Phe Gly Thr
195 200 205

Val Leu Lys Ile Ile Thr Phe Thr Lys Asn Asn Gln Phe Gln Ala Leu
210 215 220

Leu Gln Tyr Ala Asp Pro Val Ser Ala Gln His Ala Lys Leu Ser Leu
225 230 235 240

Asp Gly Gln Asn Ile Tyr Asn Ala Cys Cys Thr Leu Arg Ile Asp Phe
245 250 255

Ser Lys Leu Thr Ser Leu Asn Val Lys Tyr Asn Asn Asp Lys Ser Arg
260 265 270

Asp Tyr Thr Arg Pro Asp Leu Pro Ser Gly Asp Ser Gln Pro Ser Leu
275 280 285

Asp Gln Thr Met Ala Ala Ala Phe Gly Ala Pro Gly Ile Met Ser Ala
290 295 300

Ser Pro Tyr Ala Gly Ala Gly Phe Pro Pro Thr Phe Ala Ile Pro Gln
305 310 315 320

Ala Ala Gly Leu Ser Val Pro Asn Val His Gly Ala Leu Ala Pro Leu
325 330 335

Ala Ile Pro Ser Ala Ala Ala Ala Ala Ala Ala Ser Arg Ile Ala Ile
340 345 350

Pro Gly Leu Ala Gly Ala Gly Asn Ser Val Leu Leu Val Ser Asn Leu
355 360 365

Asn Pro Glu Arg Val Thr Pro Gln Ser Leu Phe Ile Leu Phe Gly Val
370 375 380

Tyr Gly Asp Val Gln Arg Val Lys Ile Leu Phe Asn Lys Lys Glu Asn
385 390 395 400

```
Ala Leu Val Gln Met Ala Asp Gly Ser Gln Ala Gln Leu Ala Met Ser
                405                 410                 415

His Leu Asn Gly His Lys Leu His Gly Lys Ser Val Arg Ile Thr Leu
            420                 425                 430

Ser Lys His Gln Ser Val Gln Leu Pro Arg Glu Gly Gln Glu Asp Gln
        435                 440                 445

Gly Leu Thr Lys Asp Tyr Gly Ser Ser Pro Leu His Arg Phe Lys Lys
    450                 455                 460

Pro Gly Ser Lys Asn Phe Gln Asn Ile Phe Pro Pro Ser Ala Thr Leu
465                 470                 475                 480

His Leu Ser Asn Ile Pro Pro Ser Val Ser Glu Asp Asp Leu Lys Ser
                485                 490                 495

Leu Phe Ser Ser Asn Gly Gly Val Val Lys Gly Phe Lys Phe Phe Gln
            500                 505                 510

Lys Asp Arg Lys Met Ala Leu Ile Gln Met Gly Ser Val Glu Glu Ala
        515                 520                 525

Val Gln Ala Leu Ile Glu Leu His Asn His Asp Leu Gly Glu Asn His
    530                 535                 540

His Leu Arg Val Ser Phe Ser Lys Ser Thr Ile
545                 550                 555


<210>  12
<211>  1668
<212>  DNA
<213>  Homo sapiens

<400>  12
atggacggca tcgtcccaga catagcagtc ggtacaaagc ggggatccga cgagctcttc        60

tccacgtgtg tcagcaacgg ccccttcatc atgagcagct ctgcctcagc agccaatgga       120

aacgatagca agaagttcaa aggtgacaac aggagcgcag gagtcccttc cagagtcatc       180

catgtcagaa agctgcccag cgatgtcact gagggcgagg tcatctccct agggctgccc       240

tttggaaagg ttaccaacct tctcatgctg aaggggaaga accaggcctt cattgagatg       300

aacacagagg aggctgccaa cactatggtt aactactata catcggtggc gccagtgctt       360

cgtggacagc ccatctacat ccagttctcc aaccacaaag agctcaagac cgacagctcg       420

cccaaccagg cacgtgccca ggcagccctg caggctgtaa actccgtcca gtctggaaac       480
```

ctggccttgg cagcgtccgc tgctgccgtg gatgcaggaa tggcaatggc agggcagagc 540

ccagtgctca ggatcattgt ggaaaacctt ttctacccag tgaccctgga cgtgctgcac 600

cagatcttct ctaagtttgg caccgtcctg aagatcatca cgttcaccaa gaacaaccag 660

ttccaggcgc tgctgcagta tgctgaccct gtgagcgccc agcatgccaa gctgtccctg 720

gatggccaga acatctacaa cgcctgctgc acgctgcgca tcgacttctc caagctcacc 780

agtctcaatg tcaagtacaa caatgataag agcagagact acactcgacc tgacctgccc 840

tctggagaca gccagccttc actagaccag accatggcag cagcctttgg tgcgcccggc 900

ataatgtcag cctctccgta tgcaggagcc gggttccctc ccacctttgc catccctcag 960

gccgcaggcc tctctgtccc taatgtccat ggagccttgg ccccccctggc catcccgtct 1020

gctgctgctg ctgctgcggc cagccgcatt gccatcccag ggttggcagg tgctgggaat 1080

tctgtccttt tggtcagcaa tctgaaccct gagagagtca caccccaaag cctctttatt 1140

ctcttcggcg tctacggtga tgtgcagcgg gtgaagatcc tgttcaataa gaaggagaac 1200

gcacttgtgc agatggcaga cggcagccag gcccagctgg ccatgagcca cctgaacggg 1260

cacaagctgc acgggaagtc agtgcgcatt acactgtcca agcatcagag tgtgcagctg 1320

cctcgggagg gtcaggagga ccagggcctg accaaggact atggcagctc cccgctgcac 1380

cgcttcaaga aaccaggctc caagaacttc cagaacatct ttccaccctc agctaccctg 1440

cacctctcca acatcccgcc ctctgtgtca gaggacgacc tcaagagcct cttctccagc 1500

aacggtggtg tggtcaaagg cttcaagttc ttccagaagg accgcaagat ggcactgatc 1560

cagatgggct ctgtggagga ggctgtgcag gcgctgattg aactgcacaa ccatgacctg 1620

ggcgagaacc accacctgcg agtgtccttt tccaagtcca ccatctag 1668

<210> 13
<211> 20
<212> DNA
<213> artificial sequence

<220>
<223> forward primer

<400> 13
agaggaggct gccaacacta 20

<210> 14
<211> 20
<212> DNA
<213> artificial sequence

<220>
<223> reverse primer

<400> 14

```
gtccagggtc actgggtaga                                                 20


<210>  15
<211>  30
<212>  DNA
<213>  artificial sequence

<220>
<223> sgRNA1

<400>  15
tgtggttgga gaactggatg tagatgggct                                      30


<210>  16
<211>  30
<212>  DNA
<213>  artificial sequence

<220>
<223> sgRNA2

<400>  16
gagcccatct ggatcagtgc catcttgcgg                                      30


<210>  17
<211>  30
<212>  DNA
<213>  artificial sequence

<220>
<223> sgRNA3

<400>  17
agtcgatgcg cagcgtgcag caggcgttgt                                      30
```